# EUROPEAN PATENT APPLICATION

(11) **EP 2 223 913 A1**
(43) Date of publication of application: **01.09.2010**
(21) Application number: 10154393.2
(22) Date of filing: 23.02.2010
(51) Int. Cl.: C07D 231/54, C07D 405/12, C07D 491/04, A61K 31/4155, A61K 31/416, A61K 9/107

(54) **Tricyclic pyrazole derivatives and microemulsions thereof as CB1- and/or CB2-inhibitors**

(30) Priority: 25.02.2009 IT MI20090262
(71) Applicant: Neuroscienze Pharmaness S.C. A R.L., 09010 Pula (CA) (IT)
(72) Inventor: Lazzari, Paolo, 09010, PULA (CAGLIARI) (IT); Loriga, Giovanni, 07100, SASSARI (IT); Manca, Ilaria, 07100, SASSARI (IT); Pinna, Gerard Aime, 07100, SASSARI (IT)
(74) Representative: Sama, Daniele

(57) **Abstract**

Microemulsions of pharmaceutical compositions comprising the following components (% by weight), the sum of the components being 100%:
S) from 0.01 to 95% of one or more compounds selected from surfactants, polymers forming organized structures as: aggregates, micelles, liquid crystals, vesicles, in the liquid in which they are solublized,
O) from 0.01 to 95% of one or more oils selected from esters of C₄-C₃₂ acids or C₄-C₃₂ acids,
PA) from 0.001 to 90% of compounds having affinity for the CB1 and/or CB2 cannabinoidergic receptors of formula A': 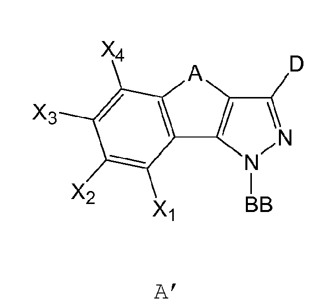 AD) from 0 to 60% by weight of one or more compounds selected from modifiers of the water and/or oil polarity, modifiers of the film curvature of component S), co-surfactants,

water or a saline aqueous solution the difference to 100%, wherein the ratio by weight S)/PA) is lower than that of microemulsions wherein component O) is absent.

## Description

The present invention relates to pharmaceutical compositions in the form of microemulsions comprising condensed tricyclic compounds having affinity for CB1 and/or CB2 cannabinoidergic receptors, the corresponding solvates and pharmaceutically acceptable salts.

Specifically the present invention relates to pharmaceutical compositions in the form of microemulsion, comprising compounds having a condensed structure containing one phenyl and one pyrazole ring linked with each other by a central ring having from five to eight atoms.

More specifically the invention relates to pharmaceutical compositions in the form of microemulsion comprising the mentioned tricyclic compounds and an oil phase, the ratio by weight surfactant/tricyclic compound being lower than that of microemulsions not comprising an oil phase.

Cannabinoids of natural origin are compounds derived from *Cannabis sativa,* commonly known as marijuana. Among the at least 66 cannabinoid compounds characterizing marijuana, tetrahydrocannabinols (THC) and Δ⁹-tetrahydrocannabinol (Δ⁹-THC) in particular, are considered to be those most active. The properties which have brought to the use of marijuana as therapeutic agent of natural origin both in mammals and in human beings have indeed been correlated to said compounds. Said properties are the following: the analgesic effect, the antiinflammatory activity, the reduction of the blood and intraocular pressure, the antiemetic activity. To tetrahydrocannabinols the negative effects which are associated to the marijuana use have furthermore been correlated, with particular reference to the perception psychological distortion, to the motory coordination loss, to the euphoria, to the sedative effect. The pharmacological action of cannabinoids appears directly correlated to their affinity towards two different classes of specific receptors belonging to the family of the "G protein-coupled" receptors: the CB1 receptors, located in the central nervous system besides that in peripheral tissues, and the CB2 receptors, found in the cerebellum (Q.J.Lu et al.; Visual Neurosci.; 2000, 17,9 1-95) but which are mostly found in peripheral tissues (M.Glass; Progr. Neuro-Psychopharmacol. & Biol. Psychiat.; 2001, 25, 743-765). In the brain the CB1 receptors are abundantly expressed in the hippocampus, in the cortical regions, in the cerebellum and inside the basal ganglia. Among the peripheral tissues wherein the CB1 receptors have been found, the testicles, the small intestine, the vesica, the deferent duct can be mentioned. The CB1 receptors have been furthermore identified both in the rat eye and in the human eye, both in the retina and in the iris and in the ciliary body (A. Porcella et al.; Molecular Brain Research; 1998, 58, 240-245; A. Porcella et al.; European Journal of Neuroscience; 2000, 12, 1123-1127). The CB2 receptors are on the contrary prevailingly located in the marginal zones of the spleen, in tonsils, besides in several cells of the immune system, as macrophages, monocytes, the cells of the bone marrow, of thymus and of pancreas. Other cells of the immune system wherein the CB2 receptors are significantly present are T4 and T8 cells, the polymor-phonucleated leucocytes, in particular the cells called "natural killers" and lymphocytes B.

The compounds able to interact, as agonists or antagonists, with the CB2 receptors can therefore be used in the treatment of diseases wherein immune system cells or immune disorders are involved. The activation (modulation) of the CB2 receptors is also important in the treatment of other diseases, such as in the treatment of osteoporosis, renal ischaemia, pain, neuropathic pain, inflammatory conditions, lateral amyotrophic sclerosis.

The compounds with affinity for the CB1 receptors can be used in the treatment of eye diseases such as glaucoma, pulmonary diseases, as asthma and chronic bronchitis, inflammations as arthritis, allergies and allergic reactions, such as allergic rhinitis, contact dermatitis, allergic conjunctivitis. Said compounds can also be used in the treatment of pain, in conditions of anxiety, behaviour disorders, delirium conditions, psychotic problems in general, furthermore for the treatment of schizophrenia, depression and in the treatment of drug and/or alcohol abuse and dependence, (for example alcoholism and tabagism). The same compounds can also be used to combat vomit, nausea, vertigoes, especially in the case of patients subjected to chemotherapy, in the treatment of neuropathies, hemicrania, stress, psychosomatic origin diseases, epilepsy, Tourette syndrome, Parkinson disease, Huntington disease, Alzheimer disease, senile dementia and in the case of recognition disorders and memory loss. Further applications of the compounds having affinity towards the CB1 receptors are the treatment of pathologies related to appetite disorders (obesity, bulimia), pathologies of the gastrointestinal tract and of the bladder, cardiovascular diseases, urinary, erectile and fertility disorders, neuroinflammatory pathologies, such as multiple sclerosis, Guillain-Barré syndrome, viral encefalitis. For example some CB1 agonist active principles are successfully used in the treatment of nausea and vomit associated to chemotherapy and in the appetite whetting in AIDS patients. Compounds having antagonist activity towards the CB1 receptors can be used for example in the treatment of psychosis, anxiety, depression, schizophrenia, obesity, neurological diseases (for example: dementia, Parkinson disease, Alzheimer disease, epilepsy, Tourette syndrome), in conditions of memory loss, of central nervous system diseases involving the neurotransmission of cannabinoids, in the treatment of gastrointestinal and/or cardiovascular disorders.

The compounds which are effective in activating cannabinoid receptors show immunosuppressive activity and are used in the treatment of eye inflammatory conditions and autoimmune diseases, for instance uveitis and uveoretinitis (H. Xu et al., J. Leukocyte Biology, 82, 2007, 532-541). They are used also for treating retina neurodegeneration (G. Pryce et al., Brain 126 2003 2191-2202).

With reference to the wide pharmacological applications of cannabinoids, in the latest years several studies have been carried out for finding endocannabinoids and for the synthesis of new compounds capable of selectively interacting with the two subclasses of CB1 and CB2 cannabinoidergic receptors. Researches have led on the one hand to the identification of anandamide endocannabinoids (arachidonyl ethanolamide) and 2-arachidonyl glycerol, on the other hand to the preparation of different classes of synthesis compounds, agonist or antagonist towards the CB1 or CB2 receptors.

The class of the compounds having agonist activity towards the CB1 receptors (cannabimimetic activity) comprises both synthesis compounds with a basic structure directly derived from that of Δ⁹-THC, as (-)-11-OH-Δ⁸THC-dimethylheptyl (HU210) and nabilone, and compounds structurally different from Δ⁹-THC, as aminoalkylindols of the WIN 55,212-2 series (M. Pacheco et al.; J. Pharmacol. Exp. Ther.; 1991, 257, 1701-183) or as bicyclic cannabinols (non classic cannabinoids) which refer to the compound CP 55,940 (M. Glass; Progr. Neuro-Psychopharmacol. & Biol. Psychiat.; 2001, 25, 743-765). The compounds having cannabimimetic activity show in vivo the following effects: hypoactivity, hypothermia, analgesia and catalepsy (B.R. Martin et al.; Pharmacol. Biochem. Behav.; 1991, 40, 471-478; P.B. Smith et al.; J. Pharmacol. Exp. Ther.; 1994, 270, 219-227).

Clinical data have shown that the CB1 antagonist pyrazole compound Rimonabant is effective in reducing both weight and metabolic and/or cardiovascular risk factors in patients with metabolic syndrome and/or dyslipidemia (J. P. Després et al., the New England Journal of Medicine, 2005, 353, 2121-2134, D. Tonstad, Nutrition, Metabolism and Cadiovascular Diseases 2006, 16, 156-162. The effectiveness of Rimonabant in reducing metabolic and/or cardiovascular risk factors has been shown also in patients with type 2 diabetes (A. J.Scheen et Al., Lancet 2006, 368 1660-1672).

A known compound for the selectivity towards the CB2 receptors, having agonist activity towards this subclass of receptors, is the compound 1-propyl-2-methyl-3-naphthoyl-indole, called JWH-015 (M. Glass; Progr. Neuro-Psychopharmacol. & Biol. Psychiat.; 2001, 25, 743-765).

Among the synthesis compounds having high affinity for the CB1 and/or CB2 cannabinoidergic receptors, condensed tricyclic derivatives containing one pyrazole ring are described.

Antagonist compounds having high affinity for the cannabinoidergic receptors and, especially, high selectivity for the CB1 receptors are described for example in EP 1,230,244. In particolar, said CB1 antagonist compounds have the following structure: 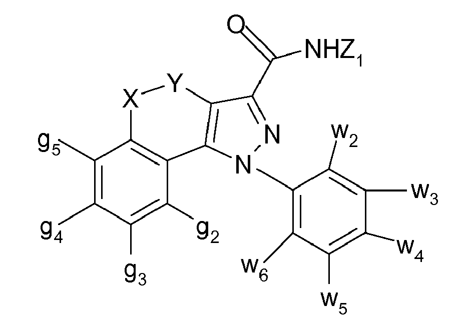 wherein Z₁, W₂, W₃, W₄, W₅, W₆, g₂, g₃, g₄, g₅ have different meanings; X-Y- represent a group selected from: -(CH₂)_{d}-CH₂-, -CH₂-S(O)_{g}-, -S(O)_{g}-CH₂-, with d equal to 1 or 2, g equal to zero, 1 or 2.

In the document no mention is made that the described compounds could be formulated under the form of a microemulsion.

Antagonist compounds having a high affinity for the cannabinoidergic receptors and, above all, high selectivity for the CB2 receptors, are described for example in EP 1,230,222. In particular said CB2 antagonist compounds have the following strucure: 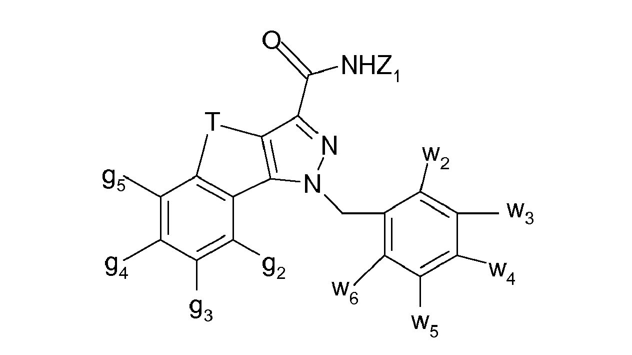 wherein: -T- represents a -(CH₂)ₘ- group, with m equal to 1 or 2; Z₁, W₂, W₃, W₄, W₅, W₆, g₂, g₃, g₄, g₅ have different meanings.

In this document no mention is made that the compounds described can be formulated under the microemulsion form.

A class of benzopyranopyrazolyl derivatives is described in USP 5,547,975 and shows the following general formula: 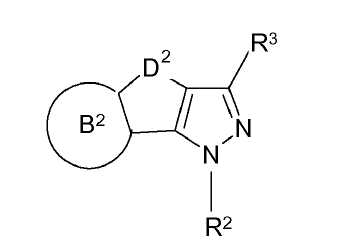 wherein B² and D² have different meanings, R² is a group selected between aryl and heteroaryl, optionally substituted with different substituents, R³ is a group selected from hydrogen, halogen, haloalkyl, cyano, nitro, formyl, alkoxycarbonyl, carboxyl, carboxyalkyl, alkoxycarbonylalkyl, amidino, cyanoamidino, aminocarbonyl, alkoxy, alkoxyalkyl, aminocarbonylalkyl, N-alkylaminocarbonyl, N-arylamino-carbonyl, N,N-dialkylaminocarbonyl, N-alkyl-N-arylamino-carbonyl, alkylcarbonyl, alkylcarbonylalkyl, hydroxyalkyl, alkylthio, alkylsulphinyl, alkylsulphonyl, alkylthioalkyl, alkylsulphinylkyl, alkylsulphonylalkyl, N-alkylsulphamyl, N-arylsulphamyl, arylsulphonyl, N,N-dialkylsulphamyl, N-alkyl-N-arylsulphamyl, heterocycle.

These compounds are described for the treatment of the inflammation or disorders related to inflammation. No mention is made that these compounds have affinity for the CB1 and/or CB2 cannabinoid receptors. Furthermore no mention is made that the formulations of said compounds could also be in the form of a microemulsion.

A further class of condensed tricyclic compounds containing one pyrazole ring is described in WO 03/070,706. The described compounds have the following general formula: 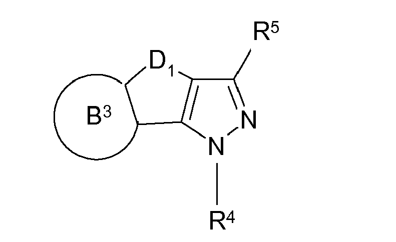 wherein D₁ has various meanings, B³ is heteroaryl, R⁴ is aryl or heteroaryl with a 5 or 6 atom ring, R⁵ is a group selected from amidine, alkylamino, aminoalkyl, NH₂, CONHR¹⁶, NHCOR⁶, CH₂-NH-COR⁶, being:
R¹⁶ a group selected from hydrogen, aryl, arylalkyl, alkyl, haloalkyl, alkenyl, alkynyl, hydroxyalkyl, aminoalkyl, alkoxy, alkoxyalkyl,
R⁶ is a group selected from hydrogen, aryl, heteroaryl, alkyl, haloalkyl, alkenyl, alkynyl, hydroxyalkyl, aminoalkyl, alkylammonialkyl, alkoxy, alkoxyalkyl, heterocycloalkyl, heterocycle.

These compounds are described for the use in the treatment of cancer, inflammation and disorders related thereto. No indication is reported as to the affinity of the compounds for the CB1 and/or CB2 cannabinoid receptors. No mention is made that the compounds could also be formulated in the form of a microemulsion.

The compounds of the above mentioned patents and patent applications are obtained as oils, waxes or powders and have lipophilic characteristics. Therefore said compounds are insoluble in water or in aqueous solutions that are compatible with the various administration routes, for instance intravenous or intraperitoneal. Therefore the use as therapeutic agents of these compounds or of their corresponding salts is limited. In fact, as such, their effectiveness in the treatment of the above mentioned pathologies and disorders is very reduced.

In the known pharmaceutical formulations surfactants are generally used. It is well known that surfactants, especially if present in significant amounts, can give side effects such as anaphylactic shocks.

The need was felt to have available pharmaceutical compositions, preferably liquid, having an improved shelf life, comprising condensed tricyclic compounds, having affinity for the CB1 and/or CB2 cannabinoidergic receptors, or the corresponding solvates or pharmaceutically acceptable salts, showing the following combination of properties:
- dilutable with water or with aqueous solutions,
- solubility of the tricyclic compounds in the liquid pharmaceutical composition at concentrations at least equal to those effective for a therapeutic treatment in human beings and in mammals,
- restoration of the homogeneity of the composition when separated phases are formed in the formulation during storage,
- reduced ratio by weight surfactant/active compound to avoid the drawbacks of the prior art,
- for the case of agonist compounds having affinity for the CB1 cannabinoidergic receptors activity at a peripheral level, at least in the reduction of the intraocular pressure.

Pharmaceutical compositions have been surprisingly and unexpectedly found by the Applicant which solve the above described technical problem.

It is an object of the present invention microemulsions of pharmaceutical compositions comprising the following components (% by weight), the sum of the components being 100%:
S) from 0.01 to 95% of one or more pharmaceutically acceptable compounds, selected from the following classes:
   - surfactants selected from non-ionic, anionic, cationic and amphotheric surfactants, optionally containing fluorine atoms,
   - polymers forming organized structures as aggregates, micelles, liquid crystals, vesicles, in the liquid in which they are solubilized,
O) from 0.01 to 95% of one or more oils selected from the following classes of pharmaceutically acceptable compounds:
   - esters of C₄-C₃₂ acids, linear or branched, the acid optionally containing one or more unsaturations, preferably of ethylene type,
   - linear or branched C₄-C₃₂ acids, optionally containing one or more unsaturations, preferably of ethylene type, that can be included when the composition has a pH such that the acid is not converted into the corresponding salt,
PA) from 0.001 to 90% of condensed tricyclic compounds having affinity for the CB1 and/or CB2 cannabinoidergic receptors of formula A': 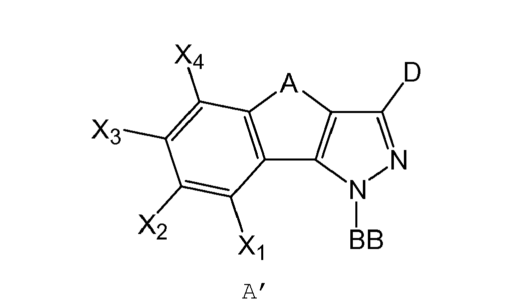 wherein:
   A is a group selected from:
      A1 is -(CH₂)ₜ-,
      A2 is -(CH₂)ᵣ-O-(CH₂)ₛ-
      A3 is -(CH₂)ᵣ-S(O)ₚ-(CH₂)ₛ-
   wherein
      t is an integer equal to 1, 2 or 3,
      p is an integer equal to 0, 1 or 2,
      r and s, equal to or different from each other, are integers equal to 0, 1 or 2 with the proviso that r+s is equal to 0, 1, 2 or 3,
   BB is a substituent selected from phenyl, arylalkyl, arylalkenyl, heteroaryl, heteroarylalkyl, or a bivalent C₁-C₁₀ aliphatic chain, linear or branched when possible, wherein the main chain end not linked to the nitrogen atom is linked to W, W being selected from hydrogen, halogen, isothiocyanate, CN, OH, OCH₃, NH₂, SO₂NH₂ or -CH=CH₂,
   X₁, X₂, X₃ and X₄, equal to or different from each other, are groups selected from hydrogen, halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
   D is a group selected from the following:
      D1: -(CH₂)-O-(CH₂)_{z}-(Z')ᵥ-R"
      wherein z is an integer equal to 1 or 2, v is an integer equal to 0 or 1, Z' is a bivalent C₁-C₈ aliphatic chain, linear or branched when possible, R" is selected from C₃-C₁₅ cycloalkyl, saturated or unsaturated heterocycle, aryl, or heteroaryl,
      D2: -C(O)-(Z')ᵥ-R"
      wherein v, Z' and R" are as defined above,
      D3: -CH(OH)-(Z')ᵥ-R"
      wherein v, Z' and R" are as defined above,
      D4: -C(O)-NH-(Z')ᵥ-T' wherein v and Z' are as defined above and T' is a group selected from:
         - C₁-C₈ alkyl, C₁-C₇ haloalkyl with the proviso that in both cases in D4 v = 0,
         - C₃-C₁₅ cycloalkyl,
         - monocyclic aryl or heteroaryl,
         - NR₁R₂, wherein R₁ and R₂, equal to or different from each other, have the following meanings: hydrogen, C₁-C₇ alkyl, C₁-C₇ haloalkyl, heteroaryl, heteroarylalkyl, aryl, arylalkyl or arylalkenyl,
            or R₁ and R₂ with the nitrogen atom form a saturated or unsaturated heterocycle from 5 to 10 atoms,
         - C₃-C₁₅ heterocycloalkyl, containing one or more heteroatoms, equal to or different from each other selected from N, O, S, with the proviso that Z' is linked to one carbon atom of the heterocycloalkyl ring,
AD) from 0 to 60% by weight of one or more compounds selected from the following classes:
   modifiers of the water and/or oil polarity,
   modifiers of the film curvature of component S),
   co-surfactants,
WA) from 0.01 to 99.9% of water or of a saline aqueous solution the sum of the components of the microemulsion being 100%,
   wherein the ratio by weight S)/PA) is lower than at least 10%, preferably at least lower than 30%, with respect to the ratio by weight S)/PA) of a prepared microemulsion starting from the same component amounts but without component O).

It has been found by the Applicant that the compositions wherein the oil phase (component O)) is absent could also not lead to the formation of microemulsions. Therefore in this case the prior art compositions are not even comparable with the compositions of the invention since they do not lead even to the formation of a microemulsion, that is the necessary condition for making the comparison for the ratio S/PA.

The microemulsions of the invention are limpid and transparent, preferably liquid. When the viscosity is very high, the microemulsions are under the form of a gel, optionally constituted by liquid crystals. The gels by dilution allow to obtain liquid pharmaceutical compositions.

In component S) the surfactants containing fluorine atoms can have (per)fluorinated chains, for example (per)fluoropolyether chains.

The liquids wherein the polymers of component S) are solubilized for forming the organized structures, are water and/or oil. The types of usable oils are mentioned hereinafter and can be of both natural and synthetic origin.

When BB in component PA) has the meaning of phenyl, arylalkyl, arylalkenyl, heteroaryl, heteroarylalkyl, BB can be substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When X₁, X₂, X₃ or X₄ have the meaning of phenyl, cycloalkyl, saturated or unsaturated heterocycle, heteroaryl, said phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocianate, phenyl, cycloalkyl, saturated or unsaturated heterocycle, heteroaryl, amino optionaly mono- or bisubstituted with a C₁-C₇ alkyl chain.

R" can be substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio or C₁-C₇ alkoxy.

When T' has the meaning of C₃-C₁₅ cycloalkyl, monocyclic aryl, monocyclic heteroaryl, C₃-C₁₅ heterocycloalkyl, T' can be substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bistubstituted with a C₁-C₇ alkyl chain, the substituents phenyl and benzyl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When R₁ and R₂ are heteroaryl, heteroarylalkyl, aryl, arylalkyl or arylalkenyl, or R₁ and R₂ with the nitrogen atom form an heterocycle, the aromatic rings or the heterocycle can be substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, the substituents phenyl and benzyl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

The preferred compounds PA) are those wherein:
A is a group selected between A1 and A2,
BB is a substituent selected from phenyl, benzyl, monocyclic heteroaryl, monocyclic heterocycloalkyl, bivalent C₄-C₁₀ aliphatic chain, linear or branched, wherein one end of the main chain not linked to the nitrogen atom is linked to W selected from hydrogen, halogen, isothiocyanate, CN, OH, OCH₃, NH₂, SO₂NH₂ or -CH=CH₂, said phenyl, benzyl, monocyclic heteroaryl and monocyclic heterocycloalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.
X₁, X₂, X₃ and X₄ are as defined above,
D is a group selected from D2, D3 or D4.

The most preferred compounds PA) are those wherein:
A is selected between A1 and A2,
BB is a substituent selected from phenyl, benzyl, thiophene, bivalent C₄-C₁₀ aliphatic chain, linear or branched, wherein the end of the main chain not linked to the nitrogen atom is linked to W, W being selected from hydrogen, halogen, OH, OCH₃, NH₂, SO₂NH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂ isothiocyanate, phenyl, cycloalkyl, saturated or unsaturated heterocycle, heteroaryl, amino optionally mono- or bisubstituted with a C₁-C₇ alkyl chain,
X₁, X₂, X₃ and X₄, equal to or different from each other, are groups selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, the substituents phenyl, cycloalkyl, saturated or unsaturated heterocycle and heteroaryl are optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, cycloalkyl, saturated or unsaturated heterocycle, heteroaryl, amino optionally mono- or bisubstituted with a C₁-C₃ alkyl chain,
D is a group selected from the following:
   a group D2 but with Z' selected from -CH₂- or -CH(CH₃)-and R" selected from C₃-C₁₅ cycloalkyl, saturated or unsaturated heterocycle, aryl, or heteroaryl, said C₃-C₁₅ cycloalkyl, saturated or unsaturated heterocycle, aryl, or heteroaryl being optionally substituted with one or more groups, equal to or different from each other, selected from SO₂NH₂, halogen, C₁-C₃ alkyl, C₁-C₃ haloalkyl, C₁-C₃ haloalkoxy, C₁-C₃ alkylthio or C₁-C₃ alkoxy,
   or a group D4 but with Z' selected from -CH₂- or -CH(CH₃)- and T' a group selected from the following: - C₃-C₁₅ cycloalkyl, - monocyclic aryl or monocyclic heteroaryl, - a group NR₁R₂, wherein R₁ and R₂, equal to or different from each other, form with the nitrogen atom a saturated or unsaturated heterocycle having from 5 to 10 atoms, - C₃-C₁₅ heterocycloalkyl, wherein Z' is linked to one carbon atom of the heterocycloalkyl,
T' can be substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, the substituents phenyl and benzyl are optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,

By microemulsion a system is meant, formed of two or more immiscible phases among each other, transparent, isotropic, comprising at least an aqueous phase and at least an oil phase, wherein the phases are stabilized by component S), optionally in the presence of one or more compounds AD). See for example R.K. Mitra, Physicochemical investigations of microemulsification of eucalyptus oil and water using mixed surfactants (AOT+ Brij-35) and butanol, J. Colloid and Interface Science, 283 (2005) 565-577.

The preferred microemulsions of the invention are the following (% by weight):
- Component S) from 0.01 to 90%,
- Component O) from 0.01 to 90%,
- Component PA) from 0.001 to 50%,
- Component AD) from 0 to 30%,
- Component WA) from 0.1 to 99.9%,
the sum of the components being 100%.

More preferred microemulsions have the following composition:
- Component S) from 0.01 to 80%,
- One or more oils of component O) from 0.01 to 70%,
- Component PA) from 0.05 to 40%,
- Component AD) from 0 to 20%,
- Component WA) from 10 to 99.9%,
the sum of the components being 100%.

Still more preferred microemulsions have the following composition:
- Component S) from 0.01 to 70%,
- Component O) from 0.01 to 50%,
- Compounds component PA) from 0.05 to 30%,
- Component AD) from 0 to 15%,
- Component WA) from 20 to 99.9%,
the sum of the components being 100%.

The preferred surfactants cmponent S) are the non-ionic and anionic ones. Among the non-ionic surfactants, the most preferred are those containing polyoxyalkylene chains, preferably polyoxyethylene chains. The following ones can for example be mentioned:
polyoxyl 35 castor oil, known for example by the trademark Cremophor^{®} EL (BASF), prepared by ethoxylation of castor oil,
polyoxyl 40 hydrogenated castor oil, known for example by the trademark Cremophor^{®} RH40 (BASF), prepared by ethoxylation of hydrogenated castor oil,
polyethylenglycol 15 hydroxystearate, known for example by the trademark Solutol^{®} HS15 (BASF), prepared by reaction of 15 moles of ethylene oxide with 1 mole of 12-hydroxystearic acid,
polyoxyethylene polysorbate, such as Tween^{®} 80, Tween^{®} 20, Tween^{®} 60, Tween^{®} 85,
sorbitan esters of fatty acids, as sorbitan monolaurate and sorbitan monostearate, commercialized for example with the trademark Span^{®} 20 and Span^{®} 60, respectively,
vitamin E/TPGS: tocopheryl propylenglycol 1000 succinate, polyoxyethylen ethers of fatty acids, as those of the series Brij^{®}, as Brij^{®} 35, Brij^{®} 76, Brij^{®} 98,
PEG-12-acyloxy-stearates, see for example C.E. McNamee et al. in "Physicochemical Characterization of PEG 1500-12-acyloxy-stearate micelles and liquid cristalline phases", Langmuir, 2005, 21, 8146-8154, among these the following can for example be mentioned:
   - PEG 1500 mono-12-capryloyloxy stearate (PEG 1500-C₁₈C₈)
   - PEG 1500 mono-12-caproyloxy stearate (PEG 1500-C₁₈C₁₀)
   - PEG 1500 mono-12-lauroyloxy stearate (PEG 1500-C₁₈C₁₂)
   - PEG 1500 mono-12-myristoyloxy stearate (PEG 1500-C₁₈C₁₄)
   - PEG 1500 mono-12-palmitoyloxy stearate (PEG 1500-C₁₈C₁₆).

Among the anionic surfactants the following can for example be mentioned: soya lecithin, for example known by the trademark Epikuron^{®} 200, bis-2-ethylhexylsulphosuccinate (AOT), sodium taurocholate.

Among cationic surfactants, hexadecyltrimethylammonium bromide (CTAB) and didodecylammonium bromide (DDAB) can for example be mentioned.

The polymers which can be used as component S) must be soluble in the aqueous phase and/or in the oily phase. By soluble it is meant that the polymers must reach in the phase in which they are soluble concentrations at least equal to those allowing the formation of organized structures as aggregates, micelles, liquid crystals, vesicles. The presence of said organized structures may be detected by specific techniques of the physical chemistry of the dispersed systems. Laser Light Scattering (LLS), Neutron Scattering, microscopy can for example be mentioned.

As said, the polymers component S) can be used also in combination with the above mentioned surfactants. Also in this case the concentration of the solubilized polymer in the liquid phase used must be such as to lead to the formation of the above mentioned organized structures.

The polymers component S) are for example polyvinylpyrrolidone and vinylpyrrolidone/vinyl acetate copolymers, commercialized for example with the trademark Kollidon^{®}, as Kollidon^{®} 12PF and Kollidon^{®} 17PF (BASF), and the block copolymers containing polyoxyalkylene chains, more preferably containing polyoxyethylene chains (PEO), as for example the block copolymers PEO with polyoxypropylene chains (PPO) characterized by PEO-PPO-PEO structures, commercially available for example with the trademark Pluronic® or Poloxamer® or Lutrol^{®}, as Lutrol^{®} F68 and Lutrol^{®} F127 commercialized by Basf.

In component O) the organic acid esters are preferably obtained by esterification of the corresponding acid, preferably aliphatic carboxylic acid, with an alcohol having an aliphatic chain, preferably C₁-C₅, or having a polyoxyethylene chain, or with glycerine. In this case mono-, di- or triglycerides are obtained.

The following can for example be mentioned:
oleoyl macrogol 6 glyceride (unsaturated polyglycosylated glyceride), commercialized for example with the trademark Labrafil^{®} 1944 CS, (Gattefossé),
propylenglycol caprylate caprate, known for example by the trademark Labrafac^{®} PG (Gattefossé),
propylenglycol monoester of the caprylic acid, commercialized for example with the trademark Capmul^{®} PG-8 (Abitec), glycerol oleate (for example Peceol^{®} (Gattefossé)),
medium chain mono- and diglycerides, for example capric and caprylic acid glycerides (for example Capmul^{®} MCM (Abitec), Imwitor^{®} 308 (Sasol)),
polyglycerol oleate (for example Pluro^{®} oleic (Gattefossé)), capric/caprylic acid triglycerides (for example Miglyol^{®} 812 and Miglyol^{®} 810 (Sasol), Labrafac^{®} CC CS (Gattefossé)), ethyl butyrate, ethyl caprylate, ethyl oleate,
tripalmitine, commercialized for example with the trademark DYNASAN^{®} 116 by Sasol.
Vegetable oils for pharmaceutical use, containing one or more of the above mentioned esters can also be used. Soya oil can for example be mentioned.

The acids component O) are preferably aliphatic carboxylic acids. Among the acids component O), the stearic acid, the omega-3 and omega-6 acids can be mentioned.

In component AD) as modifiers of the water and/or oil polarity can be cited for example polyethylenglycols. Lutrol^{®}E300 and Lutrol^{®}E400 (BASF) can be mentioned. Aliphatic alcohols, for example ethanol, can also be used.

In component AD) the modifiers of the film curvature of component S) are for example aliphatic alcohols, preferably C₂-C₅.

In component AD) the co-surfactants can be for example surfactant compounds as defined above, or aliphatic alcohols, preferably having a chain with at least 6 carbon atoms. There can be mentioned for example:
propylen glycol monolaurate, known for example with the trademark Capmul® PG12 (Gattefossé) or Lauroglycol® 90 (Gattefossé),
caprylocaproyl macrogol 8 glyceride (saturated ethyldiglycosylated glyceride) commercialized for example with the trademarks Labrasol^{®}, Gelucire 44-14 (Gattefossé),
diethylenglycol monoethyl ether, known for example by the trademark Transcutol^{®} (Gattefossé).

The compositions according to the present invention in the form of microemulsions are stable in a wide range of temperature, generally from 0°C to 80°C, preferably from 4°C to 45°C.

The microemulsions of the present invention can be prepared with a a process comprising the following steps:
(IP) solubilization of the compound component PA) in component O), obtaining an oily solution of component PA)
(IIP) addition of component S) to oily solution obtained in (IP), obtaining an oily phase comprising component S) and PA)
(IIIP) optionally, addition of component AD) to the oily phase obtained in (IIP), obtaining an oily phase comprising components AD), S) and PA)
(IVP) addition of water or of a saline aqueous solution to the oily phase obtained in (IIP) or optionally in (IIIP), obtaining a limpid phase, that is the microemulsion.

In step (IVP) the water or the saline solution are added to the oily phase preferably obtained under stirring.

The steps of the process can be carried out at temperatures in the range 0°C - 80°C.

It is possible to obtain the microemulsions of the present invention also by varying the order of performance of the above mentioned steps, or, for example, by proceeding as follows:
(IP') solubilization of the compound component PA) in component O), obtaining an oily solution or oily phase of component PA)
(IIP') addition of component S) to water or to a saline aqueous solution, obtaining an aqueous phase comprising component S)
(IIIP') optionally, addition of component AD) to the aqueous phase obtained in (IIP'), obtaining an aqueous phase comprising components S) and AD)
(IVP') mixing of the oily phase of step (IP') with the aqueous phase of step (IIP') or (IIIP'), obtaining a limpid phase that is the microemulsion.

In step (IVP') mixing is preferably carried out under stirring.

It has surprisingly and unexpectedly been found by the Applicant that the microemulsions of the invention have a surfactant content, calculated as ratio by weight surfactant/ active principle, lower than that of microemulsions having the same component amount but without component O). This is extremely advantageous since it reduces the potential undesired effects of surfactants, especially if they are present in significant amounts with respect to PA).

The Applicant has surprisingly and unexpectedly found that it is possibile to prepare pharmaceutical formulations in the form of microemulsions, containing the condensed tricyclic compounds of formula A' with affinity for the CB1 and/or CB2 cannabinoidergic receptors. The present invention makes available pharmaceutical formulations of formula (A') with improved stability. The pharmaceutical formulations of the invention show also the following combination of properties:
- restoration of the homogeneity of the composition also when separated phases are formed in the formulation during the storage,
- dilutable with water or with aqueous solutions,
- solubilization of the active principles in the liquid pharmaceutical composition at concentrations at least equal to those effective for a therapeutic treatment in human beings and in mammals,
- improved shelf life,
- reduced ratio by weight surfactant/active principle to avoid the drawbacks of the prior art.

The Applicant has furthermore surprisingly and unexpectedly found that it is possibile to obtain active principle (component PA)) concentrated microemulsions, for example having concentrations higher than 50% by weight.

It is also possibile to dilute the concentrated microemulsion with water, obtaining diluted compositions. This is a further remarkable advantage, as it is possible to obtain, by starting from a concentrated microemulsion, for example by dilution with water or saline isotonic solution, a ready to use pharmaceutical composition containing an effective dose of the active principle.

The compounds A' can be present in the microemulsions in the form of geometrical isomers (ex. cis and trans), and/or stereoisomers, when one or more chiral centres are present in the compounds.

The present invention refers also to a specific class of condensed tricyclic derivatives having high affinity and selectivity for one or more CB1 or CB2 cannabinoidergic receptors, so to act both on a peripheral level and on the central nervous system or substantially only on one receptor of this receptorial class, or simultaneously on more cannabinoid receptors.

A further object of the present invention is therefore constituted by condensed tricyclic compounds having a structure with condensed rings containing one phenyl and one pyrazole ring linked with each other by a central ring comprising from five up to eight atoms, having affinity for the CB1 and/or CB2 receptors, with central and/or peripheral activity, having formula (I): 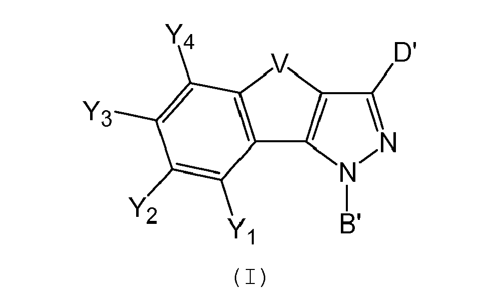 wherein:
B is a substituent selected from phenyl, arylalkyl, arylalkenyl, heteroaryl, heteroarylalkyl, or a bivalent C₁-C₁₀ aliphatic chain, linear or branched when possible, wherein the end of the main chain not linked to the nitrogen atom is linked to W^{I} selected from hydrogen, halogen, isothiocyanate, CN, OH, OCH₃, NH₂, SO₂NH₂ or -CH=CH₂,
Y₁, Y₂, Y₃ and Y₄, equal to or different from each other, have the same meaning of X₁, X₂, X₃ and X₄ as defined in formula A',
V has the same meanings of A as defined in formula A',
when B' is a bivalent C₁-C₁₀ aliphatic chain, linear or branched when possible, wherein the end of the main chain not linked to the nitrogen atom is linked to W^{I} as defined above,
D' has the same meanings of D as defined in formula A',
when B' is a substituent selected from phenyl, arylalkyl, arylalkenyl, heteroaryl, heteroarylalkyl, D' is selected from the following groups:

   D'2: -C(O)-Z'-R"
wherein Z' and R" are as defined in formula A',

   D'3: -CH(OH)-Z'-R"
wherein Z' and R" are as defined in formula A',

   D'4: -C(O)-NH-Z'-T'
Z' and T' being as defined in formula A', excluding for T' the meanings of C₁-C₈ alkyl, C₁-C₇ haloalkyl and, when in D'4 Z'= -CH₂-, T' is not: 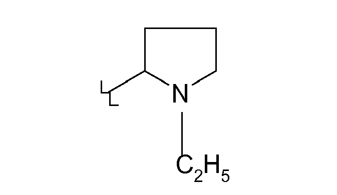
   D"2: -C(O)-R", with the proviso that V=A2,
   D"3: -CH(OH)-R", with the proviso that V=A2,
   D"4: -C(O)-NH- T', with the proviso that V has one of the following meanings: -O-, -CH₂-O-.

The compounds of formula (I) comprise the isomeric forms, both geometrical and stereoisomers and mixtures thereof. Besides, the atoms of the compounds of formula (I) can be in different isotopic forms, so to allow the radiolabelling of said compounds.

In formula (I) when B' is selected from phenyl, or arylalkyl, arylalkenyl, heteroaryl, heteroarylalkyl, B' can optionally be substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When Y₁, Y₂, Y₃ or Y₄ are selected from phenyl, cycloalkyl, saturated or unsaturated heterocycle, heteroaryl, said phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

R" can be substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio or C₁-C₇ alkoxy.

T' can be substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, the phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When R₁ and R₂ of T' are aromatic rings selected from heteroaryl, heteroarylalkyl, aryl, arylalkyl or arylalkenyl, or R₁ and R₂ with the nitrogen atom form an heterocycle, the armatic rings or the heterocycle can be substituted with one or more groups equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothocyanate, phenyl, benzyl, amino optionally mono- or bisubstituted with a C₁-C₇ alkyl chain, said phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

Where not otherwise specified, the following meanings are meant in the present invention:
by alkyl or alkyl chain it is meant a saturated C₁-C₂₀ hydrocarbon chain, linear or branched when possible,
by alkenyl or alkenyl chain it is meant a mono- or polyunsaturated C₂-C₂₀ hydrocarbon chain, preferably mono-unsaturated, linear or branched when possible,
by cycloalkyl, wherein the ring or the rings do not contain unsaturations, it is meant an aliphatc monocyclic ring, having from 3 to 10, preferably from 4 to 9 carbon atoms, or a polycyclic structure from 7 to 19 carbon atoms,
by heterocycloalkyl and saturated heterocycle it is meant a cycloalkyl as defined above wherein one or more carbon atoms are substituted by heteroatoms, equal to or different from each other, selected from S, O, N; when the ring is monocyclic, preferably the heteroatoms are no more than 2,
by unsaturated heterocycle it is meant a cyclalkyl as defined above with one or more double bonds, with the proviso that the ring structure is not aromatic, and wherein at least one carbon atom is substituted by one heteroatom selected from S, O, N,
by halogen it is meant one atom selected from fluorine, chlorine, bromine, iodine,
by haloalkyl or haloalkyl chain it is meant an alkyl as defined above, wherein one or more hydrogen atoms are substituted with halogen atoms. Examples of haloalkyl are trifluoromethyl, 1-bromo-n-butyl, pentachlorethyl, etc.
by aryl it is meant an aromatic monocyclic radical, or a condensed aromatic polycyclic radical having from 6 to 20 carbon atoms,
by heteroaryl it is meant an aryl as defined above, except that the monocyclic radical is C₅-C₆ wherein at least one or more carbon atoms are substituted with one or more heteroatoms, equal to or different from each other, selected from S, O, N, when the radical is monocyclic preferably the heteroatoms are no more than 2,
by arylalkyl it is meant an alkyl as defined above, preferably C₁-C₇, linked to an aryl as defined above, for example benzyl,
by arylalkenyl it is meant an alkenyl as defined above linked to an aryl as defined above,
by heteroarylalkyl it is meant an alkyl as defined abve, preferably C₁-C₇, linked to an heteroaryl as defined above,
by bivalent aliphatic chain it is meant a C₁-C₂₀ aliphatic chain, preferably C₁-C₈, saturated or unsaturated, linear or branched when possible, having at each end a free valence, one or more hydrogen atoms can optionally be substituted with halogen atoms,
by compound having affinity towards the receptors it is meant a compound having in vitro and/or in vivo and/or in ex-vivo agonist, or antagonist, or partial agonist, or partial antagonist, or inverse agonist, or inverse antagonist, or inverse partial agonist activity towards the receptors. The meaning of said terms is well known to the skilled in the field.

The preferred compounds of formula (I) are those wherein:
B' is a substituent selected from phenyl, benzyl, monocyclic heteroaryl, monocyclic heteroarylalkyl, or a bivalent C₁-C₁₀ aliphatic chain, linear or branched when possibile, wherein the end of the main chain not linked to the nitrogen atom is linked to W^{I} selected from hydrogen, halogen, isothiocyanate, CN, OH, OCH₃, NH₂, SO₂NH₂ or -CH=CH₂, said phenyl, benzyl, monocyclic heteroaryl and monocyclic heteroarylalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
Y₁, Y₂, Y₃ and Y₄, equal to or different from each other, have the same meanings of X₁, X₂, X₃ and X₄ as defined in formula A',
V is a group selected from A1 or A2 of compound A',
when B' has the meaning of bivalent C₁-C₁₀ aliphatic chain, linear or branched when possible, wherein the end of the main chain not linked to the nitrogen atom is linked to W^{I} as defined above, D' has the same meanings of D as defined in formula A',
when B' is a substituent selected from phenyl, benzyl, monocyclic heteroaryl or monocyclic heteroarylalkyl, D' has the meanings of D'2, D'4, D"2 or D"4.

The most preferred compounds of formula (I) are those wherein:
B' is a substituent selected from phenyl, benzyl, thiophene or a bivalent C₄-C₁₀ aliphatic chain, linear or branched, wherein the end of the main chain not linked to the nitrogen atom is linked to W¹ selected from hydrogen, halogen, isothiocyanate, CN, OH, OCH₃, NH₂, SO₂NH₂ or -CH=CH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, have the same meanings as X₁, X₂, X₃ and X₄ as defined above in formula A',
V is a group selected from A1 or A2 of compound A',
when B' is a bivalent C₄-C₁₀ aliphatic chain, linear or branched, wherein the end of the main chain not linked to the nitrogen atom is linked to W^{I} as defined above, D' has the same meanings of D as defined in formula A',
when B' is a substituent selected from phenyl, benzyl or thiophene, D' is selected from D'2, D'4, D"2 or D"4.

The still more preferred compounds of formula (I) are those wherein:
B' is a substituent selected from phenyl, benzyl, thiophene, a bivalent C₄-C₁₀ aliphatic chain, linear or branched, wherein the end of the main chain not linked to the nitrogen atom is linked to W^{I} selected from hydrogen, halogen, OH, OCH₃, NH₂, SO₂NH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
V is a group selected from A1 or A2 of compound A',
when B' has the meaning of bivalent C₄-C₁₀ aliphatic chain, linear or branched, wherein the end of the main chain not linked to the nitrogen atom is linked to W^{I} as defined above, D' has the same meanings of D as defined in formula A',
when B' is selected from phenyl, benzyl or thiophene, D' has the meanings of D'2, D'4, D"2 or D"4, with the proviso that: Z' is -CH₂- or -CH(CH₃)-,
R" is selected from C₃-C₁₅ cycloalkyl, saturated or unsaturated heterocycle, aryl, or heteroaryl, said C₃-C₁₅ cycloalkyl, saturated or unsaturated heterocycle, aryl, or heteroaryl being optionally substituted with one or more groups, equal to or different from each other, selected from SO₂NH₂, halogen, C₁-C₃ alkyl, C₁-C₃ haloalkyl, C₁-C₃ haloalkoxy, C₁-C₃ alkylthio, C₁-C₃ alkoxy,
T' is selected from:
   - C₃-C₁₅ cycloalkyl,
   - monocyclic aryl when one of the following alternative conditions is satisfied:
      V different from A1, or
      B' different from phenyl, benzyl or thiophene independently from V,
   - NR₁R₂, wherein R₁ and R₂, equal to or different from each other, with the nitrogen atom form a saturated or unsaturated heterocycle from 5 to 10 atoms,
   - C₃-C₁₅ heterocycloalkyl, wherein Z' is linked to one carbon atom of the heterocycloalkyl.

T' can be substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, said phenyl and benzyl substituents being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain.

Examples of compounds of the invention of formula (I) are the compounds having formula (X') to (XXVII') reported hereinafter: 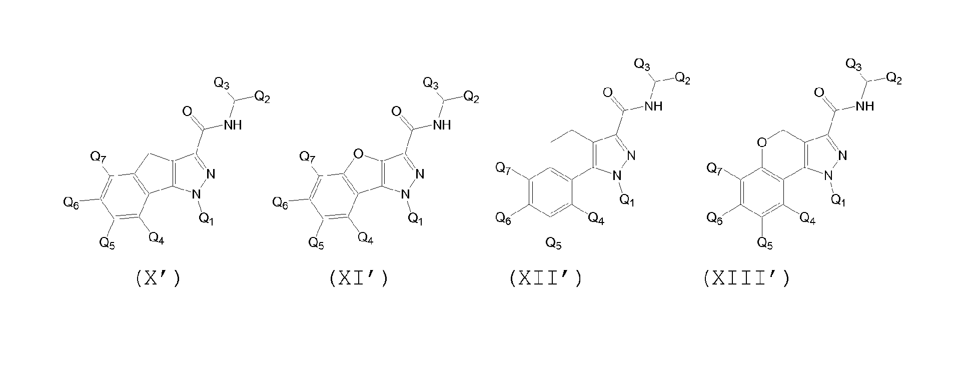 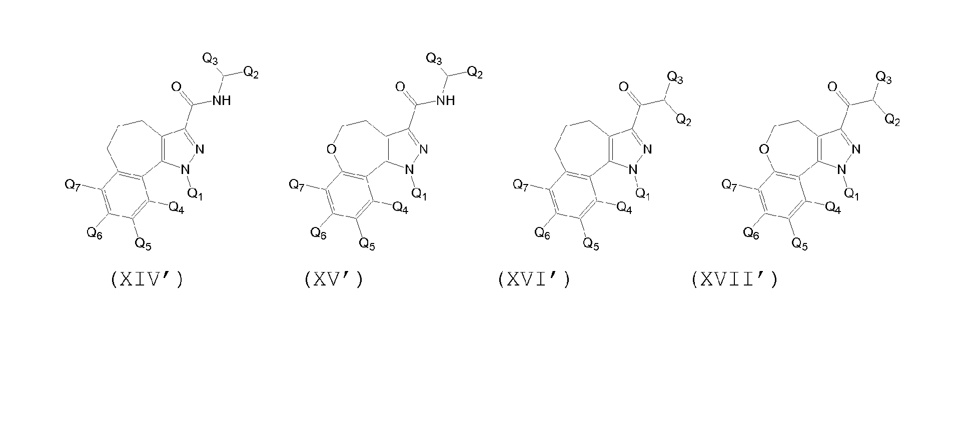 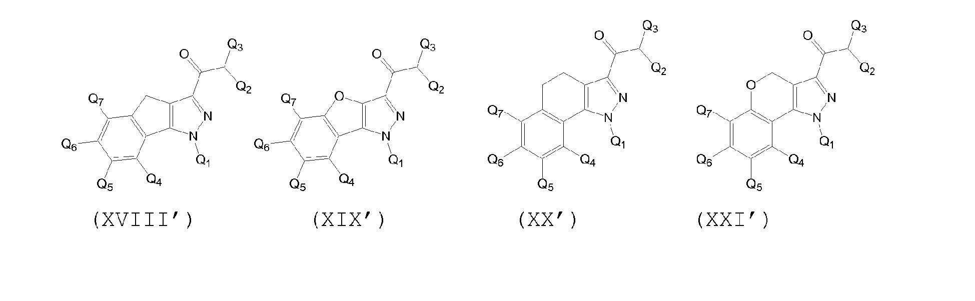 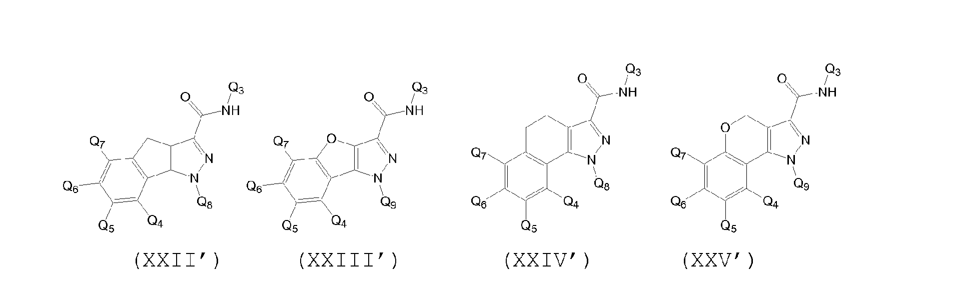 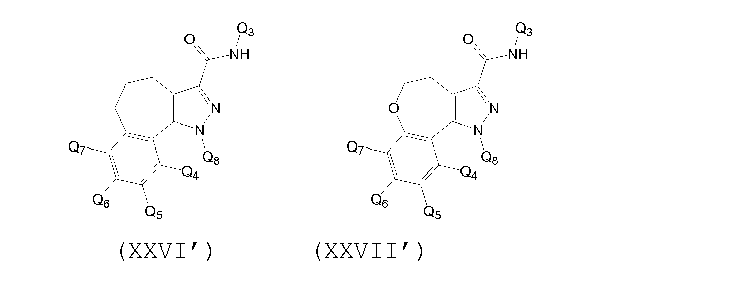 wherein:
Q₁ has the following meanings:
   Q₁A: bivalent C₄-C₁₀ aliphatic chain, linear or branched when possible, wherein the end of the main chain not linked to the nitrogen atom is linked to W^{IV} selected from hydrogen, halogen, OH, OCH₃, NH₂ or SO₂NH₂,
   Q₁B, selected from phenyl and benzyl, optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, or amino optionally mono- or bisubstituted with a C₁-C₃ alkyl chain,
   Q₈ has the meaning of Q₁A as defined above,
   Q₉ has the meaning of Q₁ as defined above,
   Q₂ has the meaning of hydrogen or methyl,
   Q₄, Q₅, Q₆, Q₇, equal to or different from each other, are groups selected frm hydrogen, halogen, C₁-C₃ alkyl, C₁-C₃ alkoxy, C₁-C₃ haloalkyl, cyano, SO₂NH₂, isothiocyanate, phenyl, cycloalkyl, saturated or unsaturated heterocycle, thiophene, amino optionally mono- or bisubstituted with a C₁-C₃ alkyl chain, said phenyl, cyclalkyl, saturated or unsaturated heterocycle and thiophene being optionally substituted with one or more groups, equal to or different from each other, selected from halogen, C₁-C₃ alkyl, C₁-C₃ alkoxy, C₁-C₃ haloalkyl, cyano, SO₂NH₂, isothiocyanate, amino optionally mono- or bisubstituted with a C₁-C₃ alkyl chain,
   Q₃ is a group selected from the following structures:

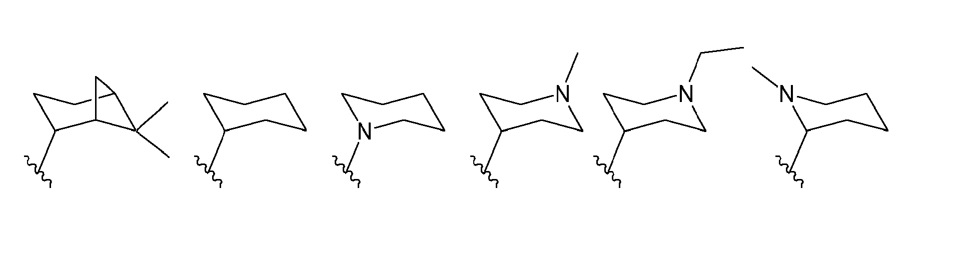

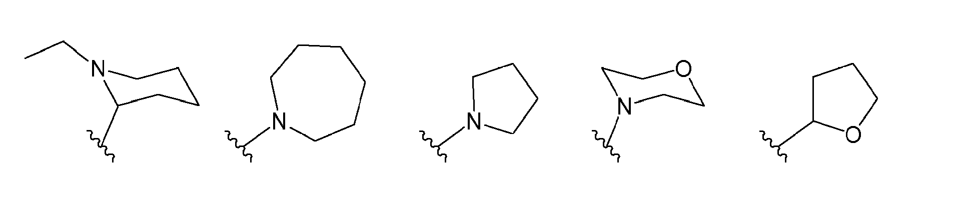

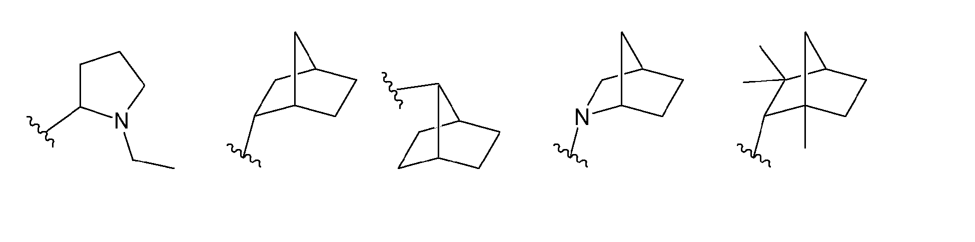

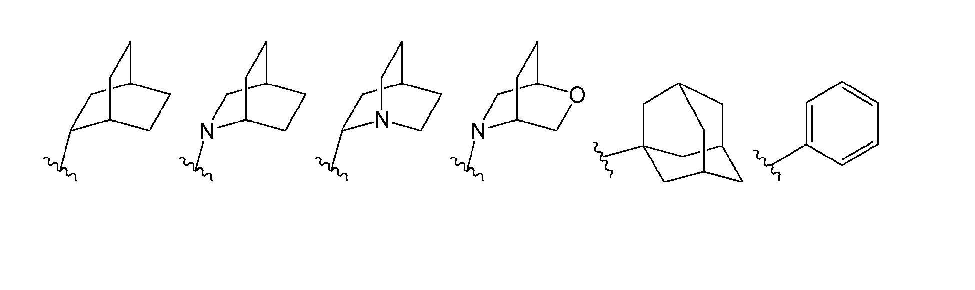

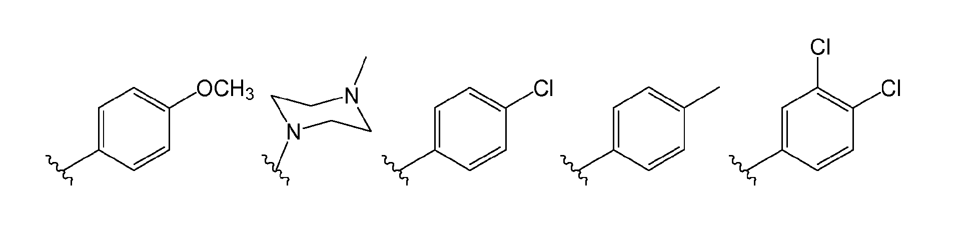

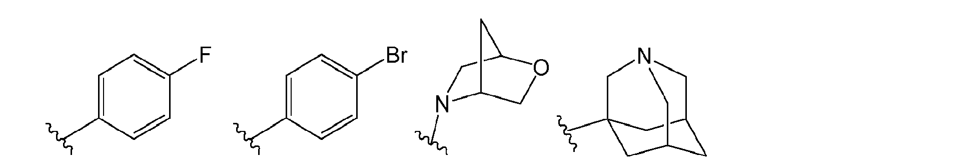

Examples of the specific compounds of formula (I) are the compounds having formula (X") to (XCIII") reported hereinafter:

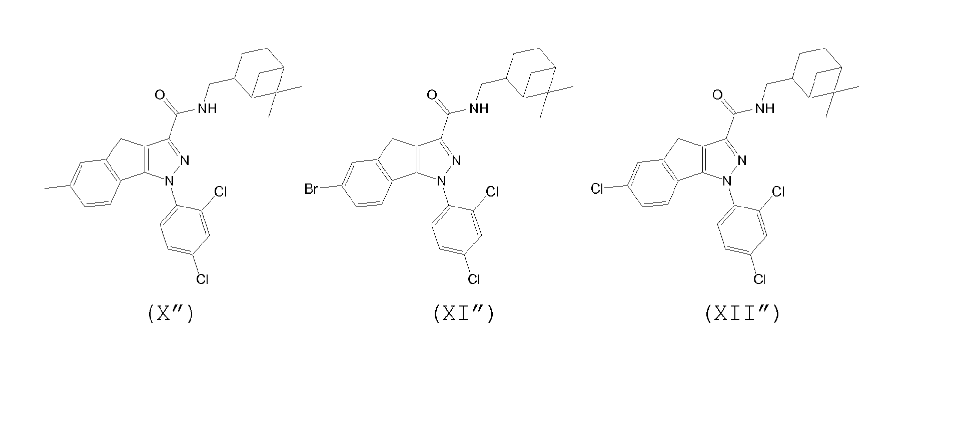

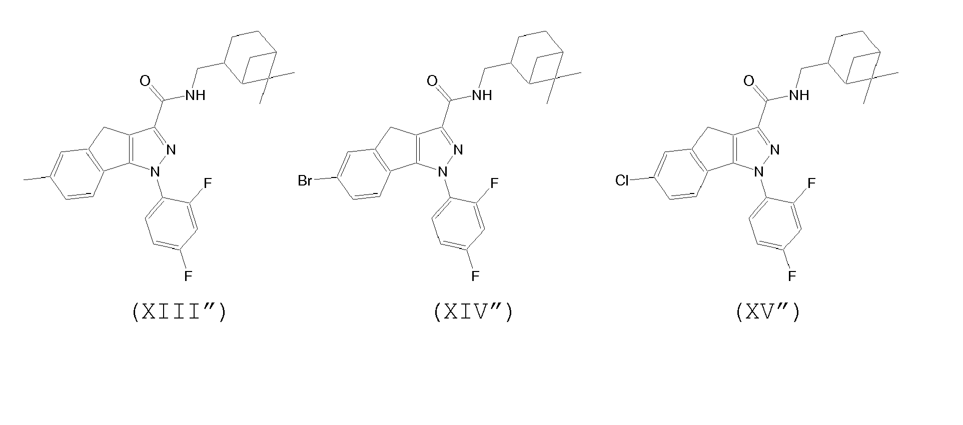

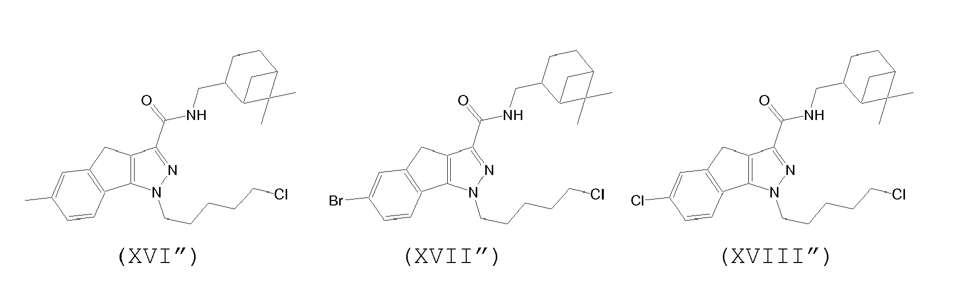

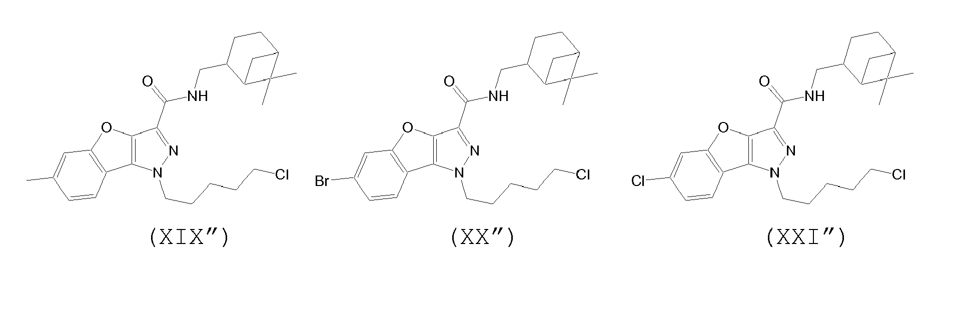

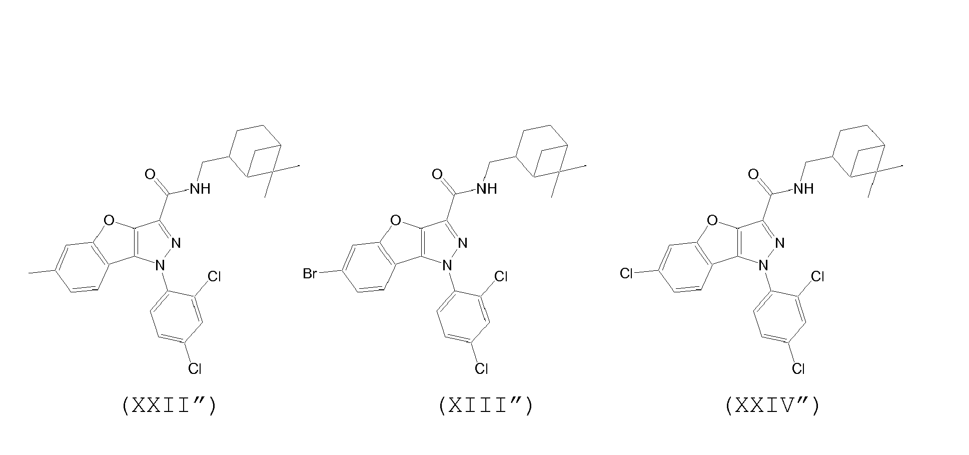

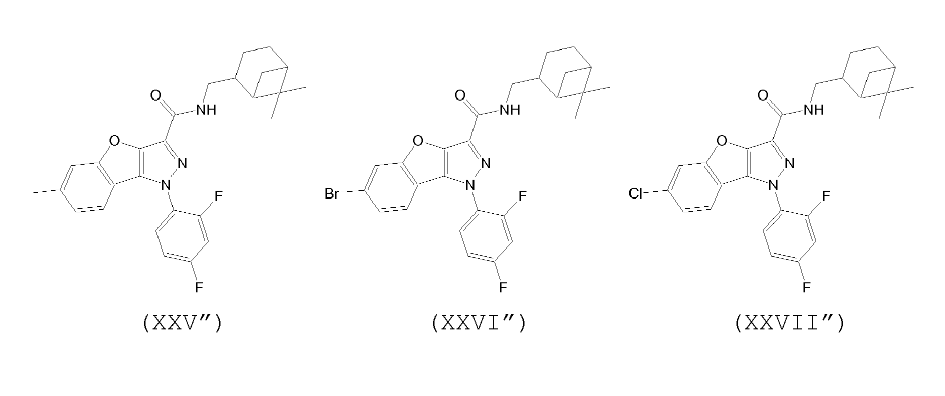

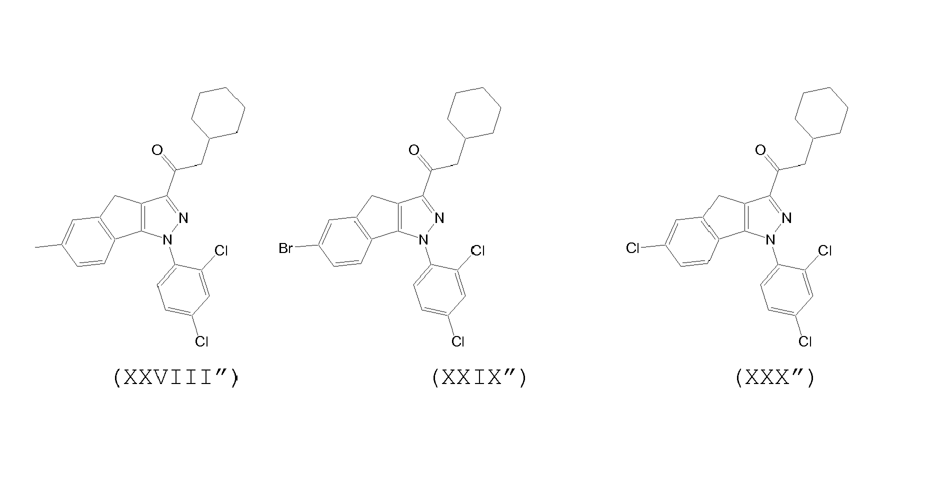

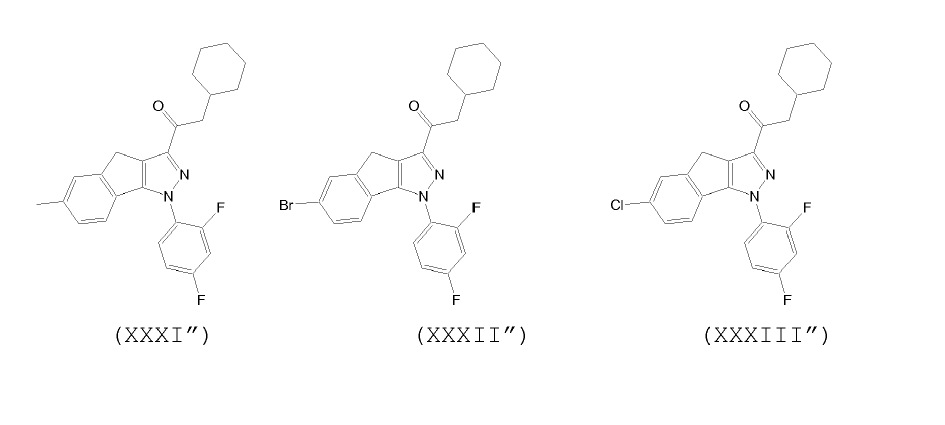

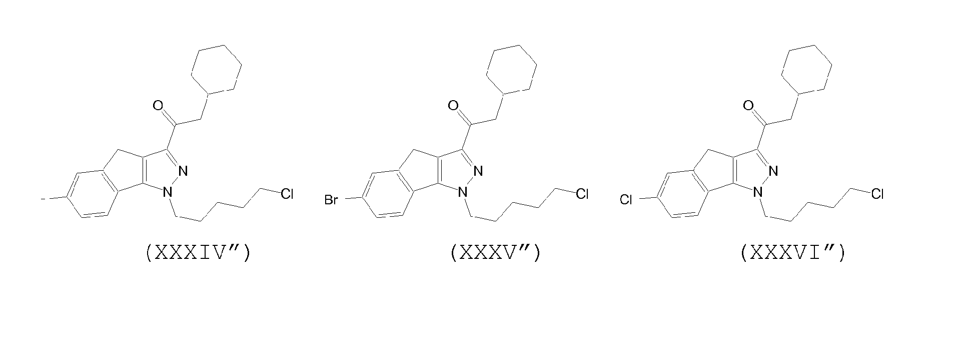

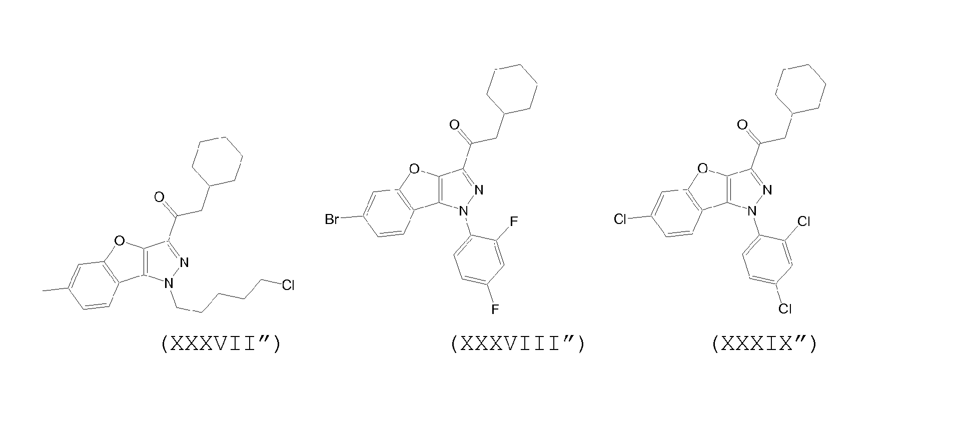

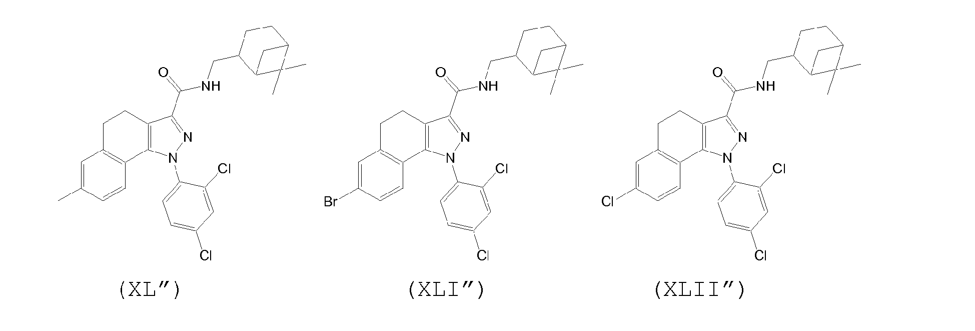

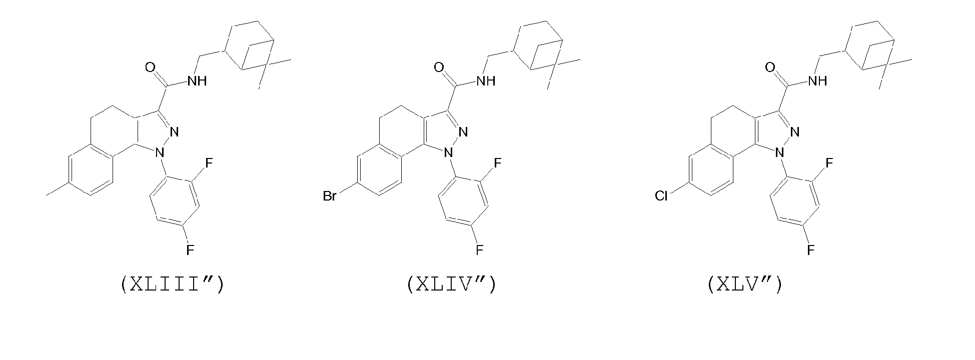

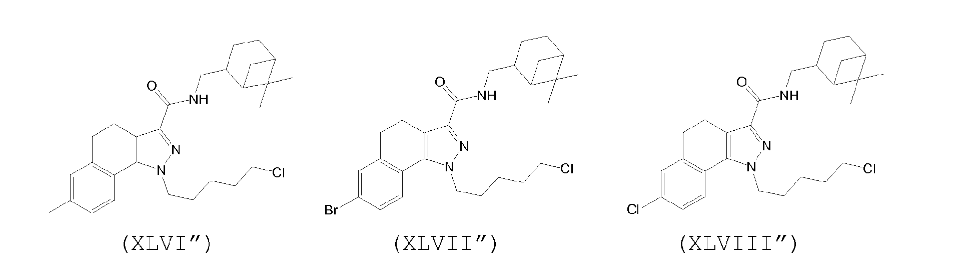

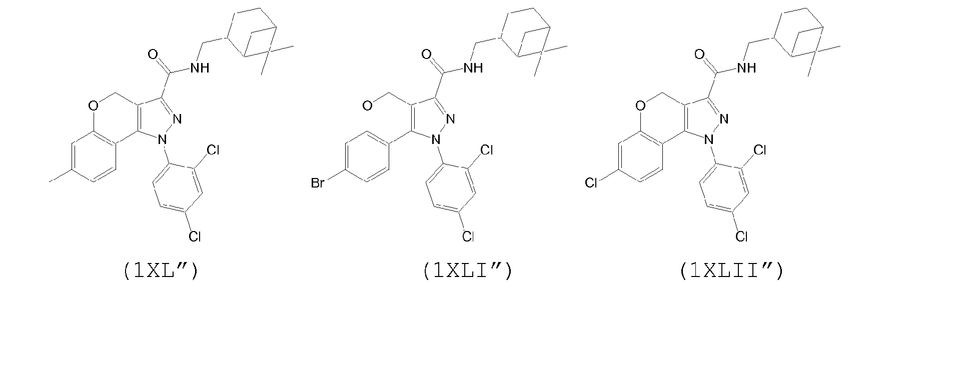

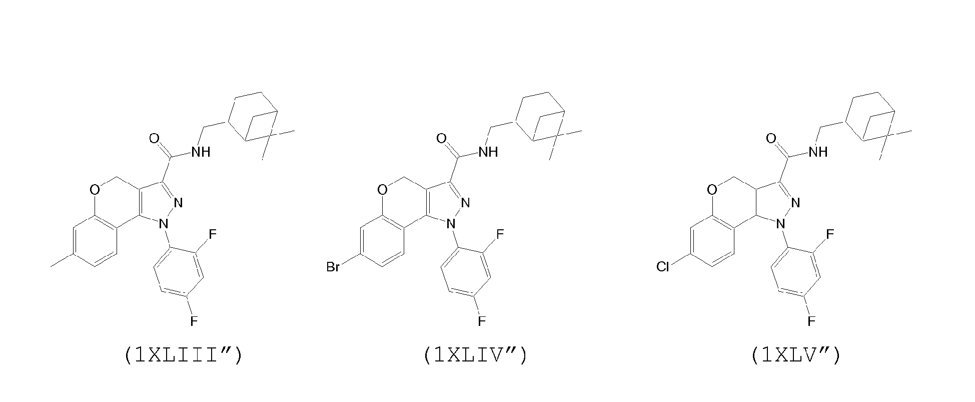

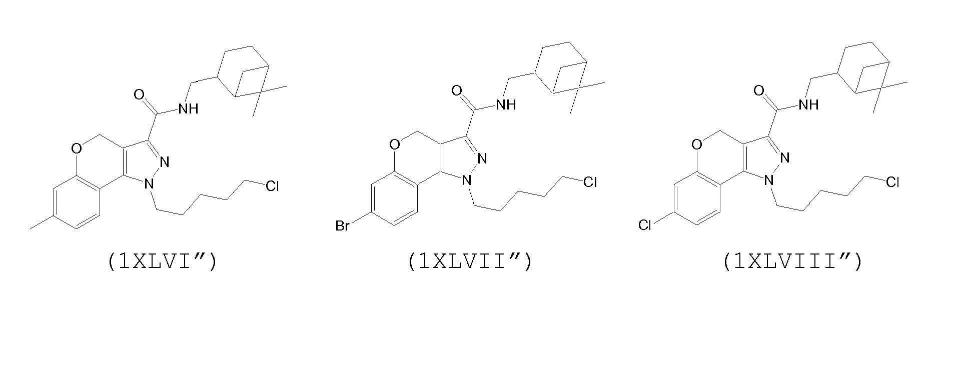

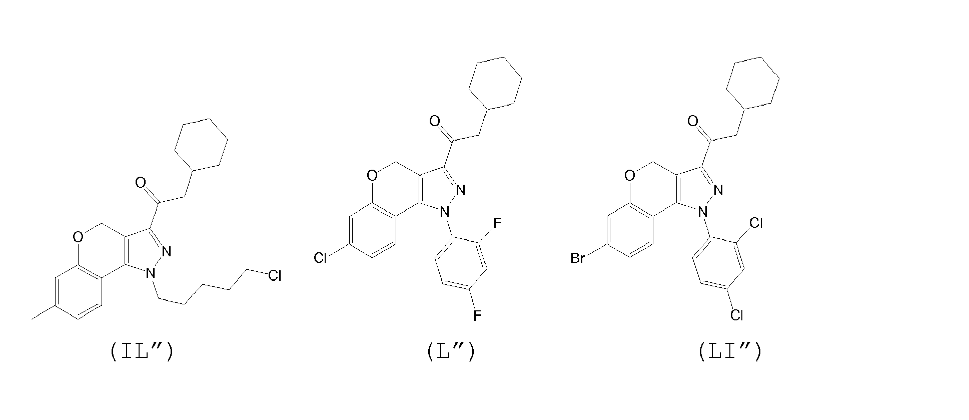

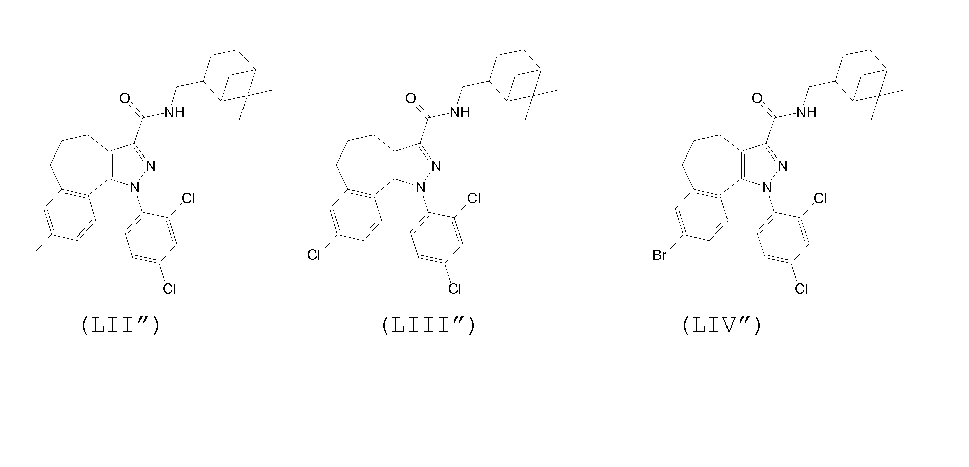

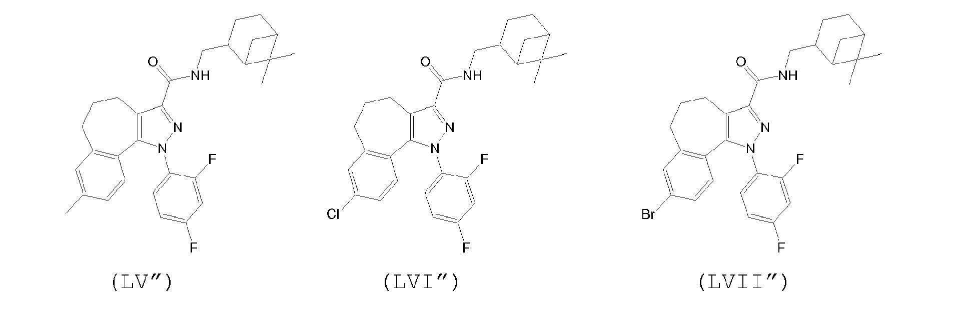

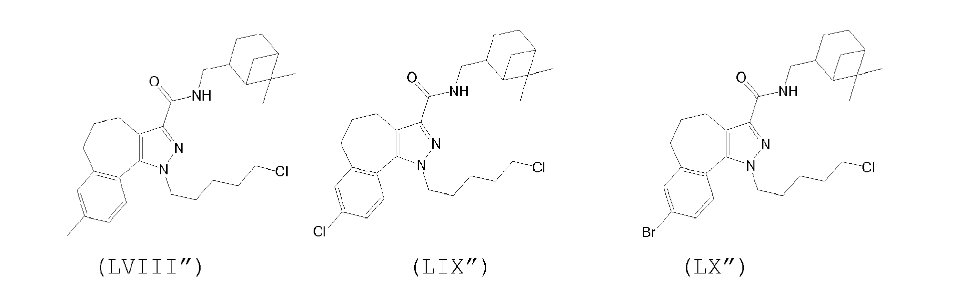

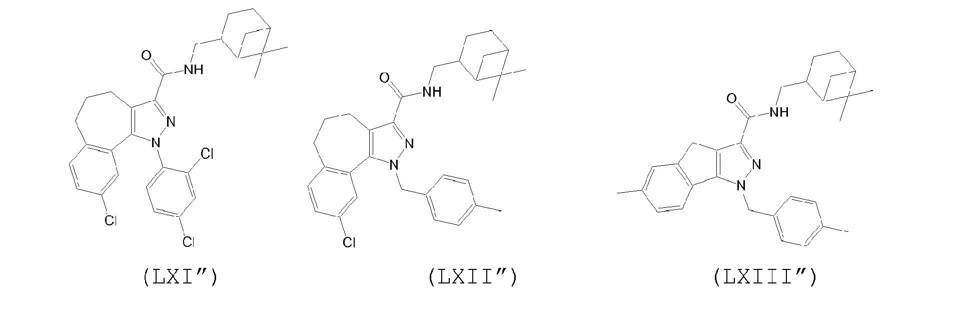

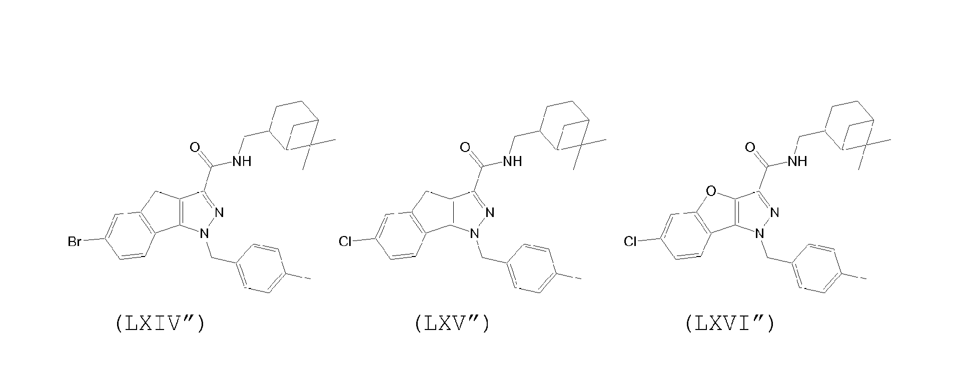

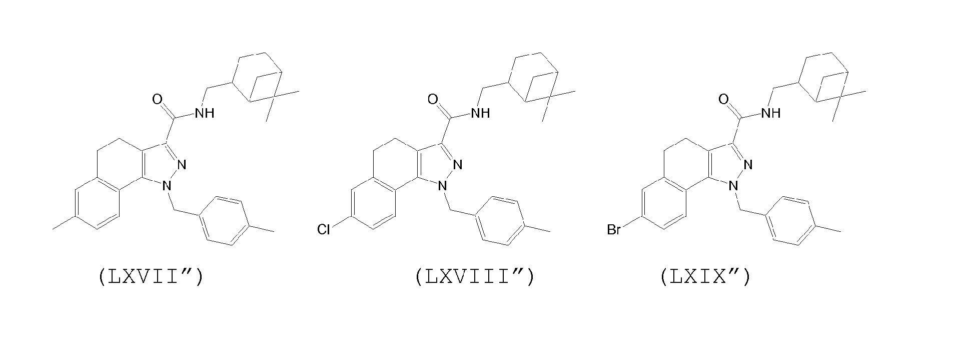

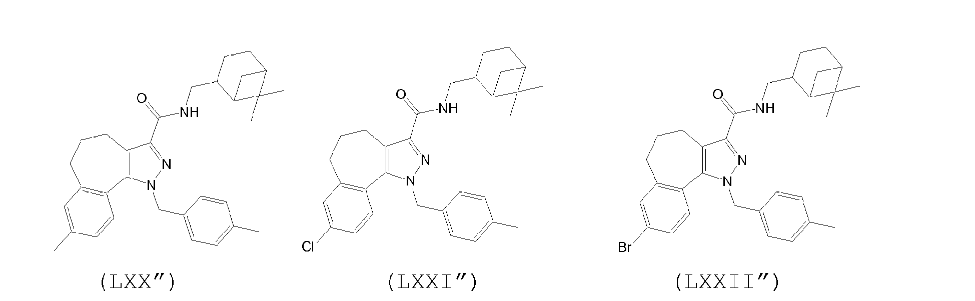

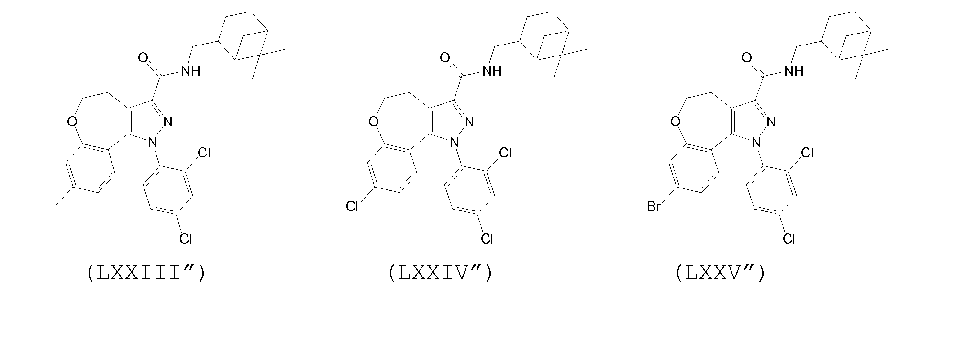

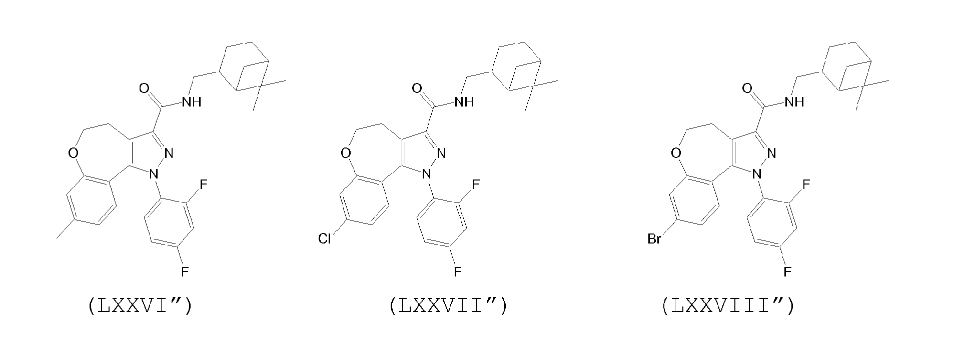

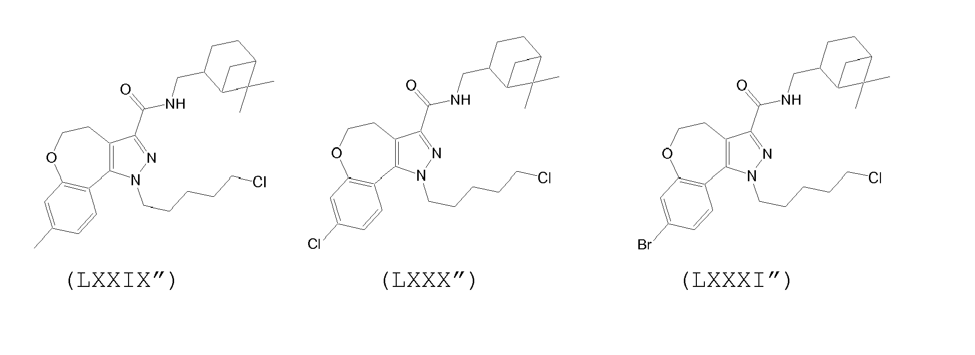

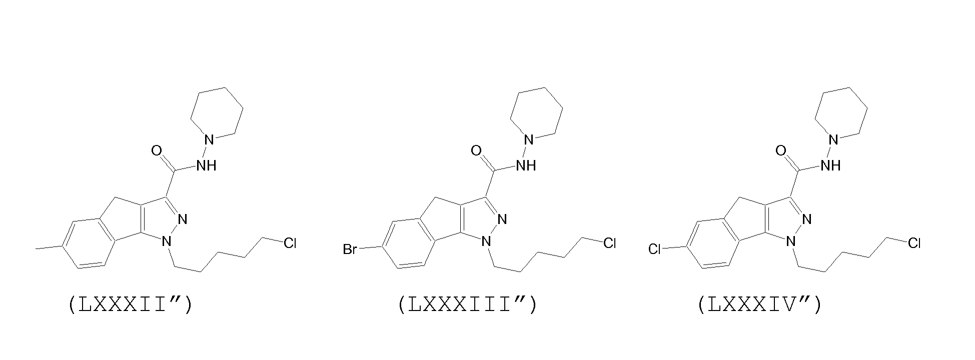

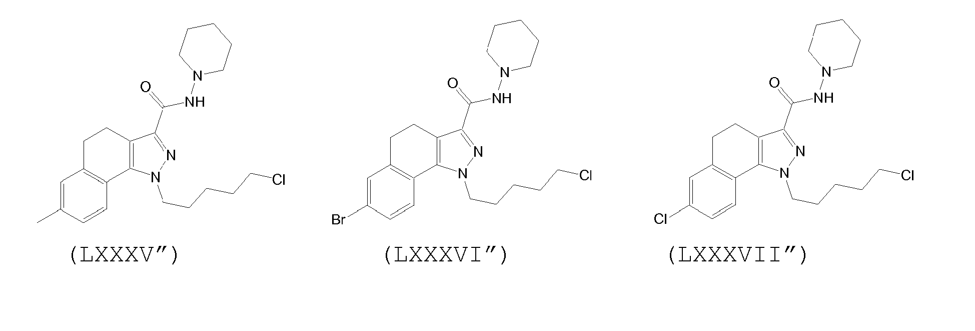

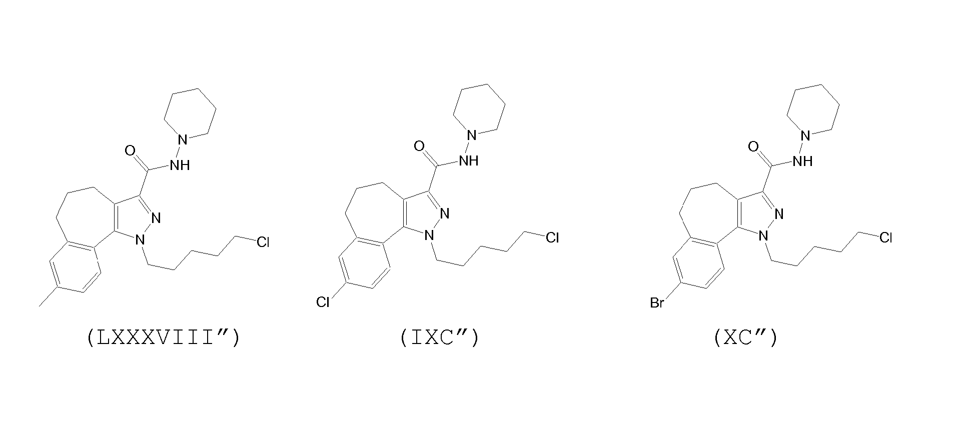

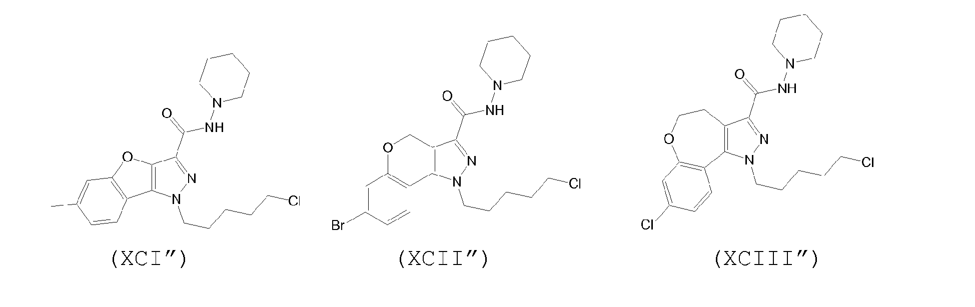

Particularly preferred compounds of the present invention are the compounds having formulas (XVI"), (XVII") (XVIII"), (XIX"), (XX"), (XXI"), (XXXIV"), (XXXV") (XXXVI"), (XXXVII"), (XLVI"), (XLVII"), (XLVI"), (XLVII"), (XLVIII"), (1XLVI"), (1XLVII"), (1XLVIII"), (IL"), (LVIII"), (LVIX"), (LX"), (LXXIX"), (LXXX"), (LXXXI"), (LXXXII"), (LXXXIII"), (LXXXIV"), (LXXXV"), (LXXXVI"), (LXXXVII"), (LXXXVIII"), (IXC"), (XC"), (XCI"), (XCII"), (XCII").

As said, hydrates, solvates and pharmaceutically acceptable salts of the compounds of formula (I), comprising the different optical and geometrical isomers (for example cis and trans isomers, optical isomers when in the compounds one or more chiral centres are present) and the corresponding mixtures of the compounds of formula (I), are a further object of the present invention. The meaning of the terms hydrate and solvate is well known to the skilled in the field. In particular by hydrate it is meant a compound containing one or more molecules of hydration water, generally from 1 to 10 molecules of water. By solvate it is meant that the compound contains one or more molecules of a solvent different from water.

By pharmaceutically acceptable salts, those salts are meant that are obtained by treating the compounds of formula (I) with organic or inorganic acids acceptable from a pharmaceutical point of view. For example hydrochlorides, sulphates, fumarates, oxalates, citrates, hydrogensulphates, succinates, paratoluensulphonates can be mentioned. See the volume: "Remington, The Science and Practice of Pharmacy", vol. II, 1995, page 1457.

The metabolites derived from the administration in human beings and in animals of the compounds of formula (I) are a further object of the present invention.

Surprisingly and unexpectedly it has been found by the Applicant that the compounds of formula (I) of the invention have in vitro and/or in vivo one or more of the following activities towards the CB1 and/or CB2 cannabinoid receptors: agonist, or antagonist, or partial agonist, or partial antagonist, or inverse agonist, or inverse antagonist, or inverse partial agonist, or inverse partial antagonist.

A further object of the present invention is a process for preparing the compounds of general formula (I) carried out as follows:
i) synthesis of the acid of the following general formula (II), or optionally of a reactive derivative thereof, selected from acyl halides, anhydrides, mixed anhydrides, imidazolides, ester-amide adducts, linear or branched C₁-C₄ alkyl esters: 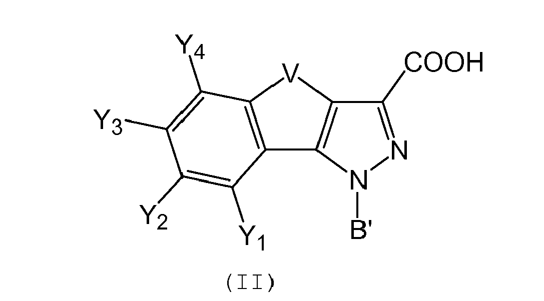 said synthesis comprising the following steps:
   - preparation of α-hydroxy-γ-ketoesters of formula (IV), wherein V, Y₁, Y₂, Y₃ and Y₄ are as previously defined, by reacting a compound of formula (III) with sodium alkoxide (RONa) and diethyloxalate in a C₁-C₃ alcoholic solvent at reflux (Claisen condensation): 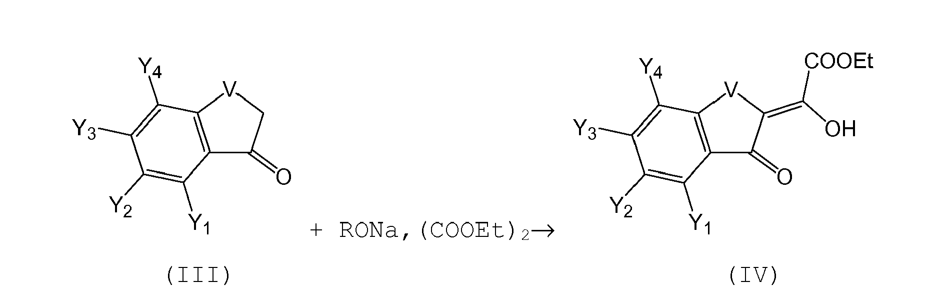
   - reaction of the compounds of formula (IV) with an hydrazine of formula (VI) wherein B' is as previously defined, said compound (VI) being optionally in the form of an hydrochloride salt thereof, in an alcoholic solvent or in acetic acid, at reflux, to yield the tricyclic compound of formula (VII):

      (IV) + NH₂-NH-B' → (VI) 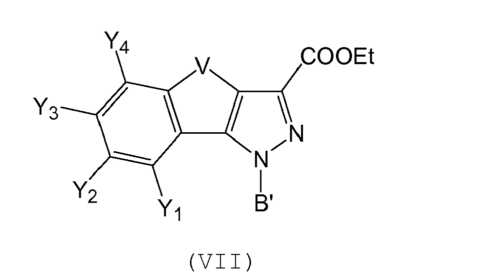
   - alkaline hydrolysis with alkaline hydroxides in hydroalcoholic solution of the compound of formula (VII) at reflux to obtain the acid of general formula (II);
   - optionally, formation of a reactive derivative of the acid of general formula (II), said reactive derivative being as defined above,
ii) when in general formula (I) D' is an ethereal group of formula D1, the compounds of formula (I) can be prepared starting from the acid of formula (II) or from an ester thereof, preferably the ethyl ester, which is reduced in a first step, by operating at room temperature, to a primary alcohol in an inert solvent under the reaction conditions (for example tetrahydrofuran), for example using an organic metal hydride, such as di-isobutyl aluminum hydride (DIBAL-H), or lithium and aluminum hydride LiAlH₄; then the thus prepared primary alcohol is reacted at room temperature with an alkyl halide of formula R"-(Z')ᵥ -(CH₂)_{z}-Hal, wherein Hal is halogen and Z', v and z are as defined above, in the presence of an alkali hydride, as sodium hydride, obtaining the above mentioned compounds of formula (I) wherein D'=D1,
iii) when in general formula (I) D'=D2, the compounds of formula (I) can be prepared according to one of the following processes:
   first process, comprising:
      - reaction of an ester of the acid of general formula (II), with trialkylaluminum and with the hydrochloride salt of an amine in a solvent, inert under the reaction conditions, until disappearance of the ester, and subsequent addition to the reaction mixture of R"-(Z')ᵥ-MgBr, wherein Z', v and R" are as defined above, by reacting at room temperature until obtaining the compound of formula (I) wherein D'= D2,
   second process, comprising:
      - reaction of the acid of formula (II), or a reactive derivative thereof, with a metallorganic salt of formula (R"-(Z')ᵥ)⁻ Me⁺, wherein Me⁺ is an alkaline metal cation, in an inert solvent under the reaction conditions, obtaining the compound of formula (I) wherein D'=D2,
iiii) when in general formula (I) D'=D3 the synthesis is carried out in two steps:
   - formation of the compound of formula (I) wherein D'=D2, by using one of the two processes described above under iii),
   - reduction of the compound obtained in the previous step at room temperature, obtaining the final compound of formula (I) wherein D'=D3,
iiiii) when in general formula (I) D'=D4, the compounds of the invention are prepared by reaction of the acid of formula (II), in the form of a suitable reactive derivative thereof as defined above, with a compound of formula:

   H₂N-(Z')ᵥ-T' (VIIA)
wherein Z', v and T' have the previously defined meanings. The reaction is carried out in a solvent, inert under the reaction conditions, and at room temperature.

In i) when B' has the meaning of bivalent C₁-C₁₀ aliphatic chain, linear or branched when possible, wherein the end of the main chain not linked to the nitrogen atom is linked to W^{I}, W^{I} being as defined above, the synthesis of the compound (VII) can be performed by reacting compound (VI) with hydrated hydrazine in an alcoholic solvent, preferably ethanol, at reflux obtaining compound (VI'): 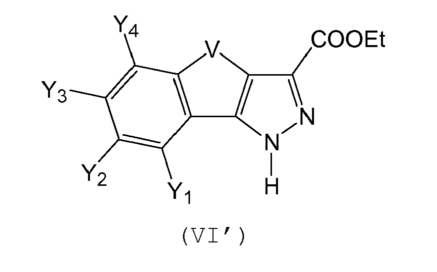 and subsequent alkylation of compound (VI') with W^{I}-B'-Z" in an inert solvent at reflux, preferably in the presence of a base, obtaining compound (VII), W¹ and B' being as defined above and Z" a leaving group, for example bromine, tosyl, mesyl.

In iii) the first process is the preferred one.

Preferably in iii), in the first reaction of the first process, for obtaining the compounds of general formula (I) wherein D'=D2, the ethyl ester of the acid of general formula (II), Al(CH₃)₃, HN(OCH₃)CH₃.HCl is used and as reaction solvent dichloromethane. Preferably both the reactions of said synthesis process described in iii) are initially carried out at a temperature of 0°C and then at room temperature (20-25°C).

In iii), in the second process for obtaining the compounds of general formula (I) wherein D'=D2, preferably Me⁺ is the lithium cation.

Preferably the reduction reaction in iiii) is carried out with lithium hydride and aluminum or with sodium borohydride.

The compounds of formula (III) and (VIIA) are commercially available or their preparation is described in the publications of the technical field.

When in the compounds of formula (I) D' is respectively D'2, or D'3, or D'4, the process for obtaining the compounds of formula (I) as described above can be used, with the proviso that v=1.

The other compounds of formula A', different from the compounds of formula (I), are prepared as described in EP 1,230,244, EP 1,230,222, USP 5,547,975.

A further object of the present invention are acid compounds of formula (II'): 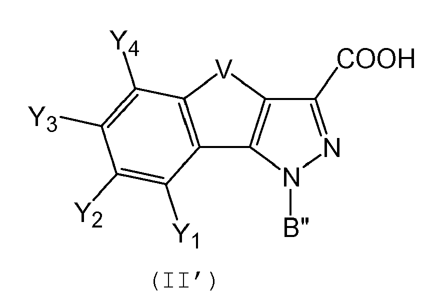 wherein:
V, Y₁, Y₂, Y₃ and Y₄ are as defined above,
B" is hydrogen or a bivalent C₁-C₁₀ aliphatic chain, linear or branched when possible, wherein the main chain end not linked to the nitrogen atom is linked to W^{II}, W^{II} being a group selected from hydrogen, halogen, isothiocyanate, CN, OH, OCH₃, NH₂, SO₂NH₂ or -CH=CH₂.

The preferred acids formula (II') are those wherein:
V is a group selected between A1 and A2 of compound A',
B" is as defined above,
Y₁, Y₂, Y₃ and Y₄, equal to or different from each other, are groups selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, phenyl, cycloalkyl, saturated or unsaturated heterocycle, heteroaryl, isothiocyanate, or amino optionally mono- or bisubstituted with a C₁-C₇ alkyl chain.

It is a further object of the present invention pharmaceutical compositions obtainable from microemulsions containing the compounds of formula A'.

It is a further object of the invention the use of the microemulsions of the invention for preparing pharmaceutical compositions. The pharmaceutical compositions can comprise the microemulsions as such wherein the amount of active principle is that required for the specific pharmaceutical application. If requested the starting microemulsion can be diluted for obtaining the desired concentration.

The pharmaceutical compositions of the invention can also be only in part formed of the microemulsions of the present invention. In this case preferably the microemulsions of the present invention are present in amounts (% by weight) from 0.1% to 95%, more preferably from 5 to 85%, still more preferably from 10 to 60%, depending on the desired amount of active principle in the final formulation.

In the pharmaceutical compositions the component PA) can be present as such or in the salt, hydrate or solvate form, or also as an isomer (geometrical isomer or stereoisomer).

The additives contained in the pharmaceutical compositions comprising the microemulsions are excipients, carriers, dyestuffs, preservatives, aromas, etc., the use of which in the pharmaceutical field is known. The used amounts of these additives and excipients are those known for the specific applications.

The administration of the pharmaceutical compositions can be made by oral, subcutaneous, sublingual, intramuscular, intravenous, topic, transdermal, rectal, ophthalmic, intranasal, vaginal, intraperitoneal route.

When the pharmaceutical compositions are only partly formed of the microemulsions of the present invention, the compositions can comprise for example dispersions, solutions, emulsions, powders, capsules, aerosol, suppositories, tablets, syrups, elixirs, creams, gels, ointments, plasters, etc.. See for example those described in patent application WO 2004/011,468. The pharmaceutical compositions can be prepared according to the known processes of the pharmaceutical technology. For example, the pharmaceutical compositions can be obtained according to the procedures mentioned in USP 6,028,084.

The compounds of formula A', comprising the various isomers and the corresponding hydrates or solvates and pharmaceutically acceptable salts thereof and the respective pharmaeutical compositions, have a high affinity in vitro for the CB1 and/or CB2 cannabinoid receptors. See the examples. More specifically, the compounds of formula A' have a *K*i value for CB1 and/or CB2 cannabinoid receptors lower than 0.5 µM.

It is a further object of the present invention the use of the pharmaceutical compositions of the present invention for the prophylaxis and therapy in mammals and in human beings of the diseases and disorders wherein the receptors of the CB1 and/or CB2 cannabinoids are involved.

A further object of the present invention are the compounds of formula (I) or isomers, or hydrates or solvates or pharmaceuticcally acceptable salts thereof for the prophylaxis and therapy in mammals and in human beings of the diseases and disorders wherein the receptors of the CB1 and/or CB2 cannabinoids are involved.

The diseases and the disorders which can be treated with the pharmaceutical compositions of the present invention are the following: diseases involving immune system cells or immune disorders, osteoporosis, renal ischaemia, inflammatory conditions, pain, post-surgery pain, neuropathic pain, eye diseases, glaucoma, pulmonary diseases as asthma and chronic bronchitis, inflammations such as arthritis, allergies and allergic reactions, such as allergic rhinitis, contact dermatitis, allergic conjunctivitis, anxiety, behavioural disorders, delirium conditions, psychotic disorders in general, schizophrenia, depression, treatment of drug and/or alcohol abuse and/or dependence (for example alcoholism and tabagism), vomit, nausea, vertigoes, in particular in patients submitted to chemotherapy, neuropathies, hemicrania, stress, psychosomatic origin diseases, epilepsy, Tourette syndrome, Parkinson disease, Huntington disease, Alzheimer disease, senile dementia, lateral amyotrophic sclerosis, cognition disorders and memory loss, pathologies associated with appetite (obesity, bulimia), pathologies of the gastrointestinal tract and of the bladder, cardiovascular diseases, urinary, erectile and fertility disorders, neuroinflammatory pathologies such as multiple sclerosis, Guillain-Barré syndrome, viral encephalitis, syndrome associated to demineralization, osteoporosis.

The compounds and the pharmaceutical compositions of the present invention can also be used for reducing metabolic and/or cardiovascular risk factors, also in patients with metabolic syndrome and/or dyslipidemia and in patients with type 2 diabetes.

The compounds of formula (I) and thereof pharmaceutical compositions can also be used for the treatment of eye inflammatory conditions, eye autoimmune diseases, uveitis, uveoretinitis and retina neurodegeneration.

For the activity at a peripheral level, in particular for the reduction of the intraocular pressure, agonist compounds having affinity for the CB1 cannabinoidergic receptors are used. At this purpose the particularly suitable preferred compounds of the present invention of formula (I) are those having affinity values for the CB1 cannabinoidergic receptors, expressed as Ki, lower than 200 nM, preferably lower than 100 nM.

The use of the pharmaceutical compositions of the present invention for the treatment of the various pathologies can be made by applying the known methods employed for said treatments.

In particular the administration of the compositions of the invention must be performed so that the amount of active principle is effective for the specific treatment. The dosages, the administration routes and the posology are determined depending on the disease typology, on the patholopgy severity, on the physical conditions and characteristics of the patient as for example age, weight, response to the active principle, on the pharmacokinetics and toxicology of the active principle for the specific treatment.

The preferred daily dosage is of 0.01-1,000 mg of compound of formula A' per Kg of body weight of the mammal to be treated. In human beings, the preferred daily dosage range is 0.1-1,000 mg of compound for Kg of body weight, still more preferred from 1 to 800 mg.

The present invention relates furthermore to the compounds of formula (I), or their isomers or hydrates or solvates or pharmaceutically acceptable salts thereof, for preparing drugs for the tratment in mammals and in human beings of diseases and disorders wherein the CB1 and/or CB2 cannabinoid receptors ar involved.

Said compounds can therefore be used for the treatment of the same above mentioned diseases and disorders that can be treated with the pharmaceutical compositions of the invention containing the compounds of formula A'.

A further object of the present invention relates to the use of the compounds of formula (I) as a medicament and in particular for the treatment of the above mentioned diseases and disorders.

The compounds of formula (I), containing radioactive isotopes and the pharmaceutical formulations thereof, can be used for identifying and labelling the receptors of the CB1 and/or CB2 cannabinoids in mammals or in human beings.

Besides, the compounds of formula (I) containing in the molecole an hydroxyl group, can be used for obtaining ligands for the cannabinoidergic receptors.

The ligands are detectable by immunochemical methods, to be used in the separation, purification and characterization of the CB1 and/or CB2 cannabinoid receptors in identifying the corresponding active sites.

The following examples are reported for a better understanding of the present invention but are not meant to be limitative of the scope of the invention.

### EXAMPLES

### EXAMPLE 1.1

### Preparation of ethyl 9-chloro-1-(2',4'-dichlorophenyl)-1,4,5,6-tetrahydrobenzo[6,7]cyclohepta[1,2-c]pyrazol-3-carboxylate

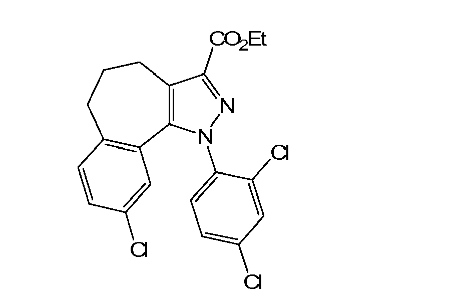

### 1.1a Preparation of ethyl α-(8-chloro-1-oxo-2,3,4,5-tetrahydro-benzocyclohepten-2-yl)-α-oxo-acetate

0.59 grams of metal sodium (25.68 mmoles) were added to 15 ml of absolute ethanol under stirring up to obtain the complete solubilization. 1.88 grams (12.84 mmoles) of diethyloxylate and a solution of 8-chloro-2,3,4,5-tetrahydrobenzocycloheptan-1-one (2.50 g, corresponding to 12.84 mmoles) in absolute ethanol (40 ml) were added to the formerly prepared solution. The reaction mixture was kept under stirring at room temperature for 5 hours and then poured into an ice and HCl 1N mixture, obtaining a white precipitate. The precipitate was filtered, washed with water and dried in the air. 3.67 g (97% yield) of product were obtained corresponding to the compound α-(8-chloro-1-oxo-2,3,4,5-tetrahydro benzocyclohepten-2-yl)-α-oxo-ethyl acetate (Compound 1.1a). Rf = 0.51 (oil ligroin/AcOEt 9.5:0.5 v/v); IR (nujol) (λ= cm⁻¹) 3435, 1731, 1698; ¹H-NMR (CDCl₃) δ 1.41 (t, 3H, J = 7.0 Hz); 2.06 (q, 2H, J = 7.0 Hz); 2.31 (t, 2H, J = 6.4 Hz); 2.71 (t, 2H, J = 7.0 Hz); 4.39 (q, 2H, J = 7.0 Hz); 7.16 (d, 1H, J = 7.8 Hz); 7.42 (dd, 1H, J = 2.0 e 7.8 Hz); 7.60 (d, 1H, J = 2.0 Hz); 15.37 (bs, 1H). Anal. calc. for C₁₅H₁₅ClO₄: C, 61.13; H, 5.13; Cl, 12.03. Found: C, 60.98; H, 5.12; Cl, 12.01.

### 1.1b Preparation of ethyl 9-chloro-1-(2',4'-dichlorophenyl)-1,4,5,6-tetrahydrobenzo[6,7]cyclohepta[1,2-c]pyrazol-3-carboxylate

A mixture of the compound 1.1a (1.0 g corresponding to 3.39 mmoles) and 2,4-dichlorophenylhydrazine hydrochloride (0.83 g corresponding to 3.90 mmoles) in 8 ml of glacial acetic acid was heated at reflux for 8 hours, then cooled at room temperature. A precipitate was formed that was filtered, washed with water and dried in the air to give 0.99 g (68% yield) of 9-chloro-1-(2',4'-dichlorophenyl)-1,4,5,6-tetrahydrobenzo [6,7]cyclohepta[1,2-*c*]pyrazol-3-ethyl carboxylate (compound 1.1b). Rf = 0.43 (oil ligroin/AcOEt 9:1 v/v); IR (nujol) (λ= cm⁻¹) 1709; ¹H-NMR (CDCl₃) δ 1.43 (t, 3H, J = 7.0 Hz); 2.10-2.35 (m, 2H); 2.66 (t, 2H, J = 6.8 Hz); 3.10-3.40 (m, 2H); 4.46 (q, 2H, J = 7.2 Hz); 6.65 (s, 1H); 7.15-7.30 (m, 2H); 7.35-7.50 (m, 2H); 7.57 (d, 1H, J = 9.0 Hz). Anal. calc. for C₂₁H₁₇Cl₃N₂O₂: C, 57.89; H, 3.93; Cl, 24.41; N, 6.43. Found: C, 57.74; H, 3.92; Cl, 24.39; N, 6,41.

### EXAMPLE 1.2

### Preparation of ethyl 9-chloro-1-(4'-methylbenzyl)-1,4,5,6-tetrahydrobenzo [6,7]cyclohepta[1,2-c]pyrazol-3-carboxylate

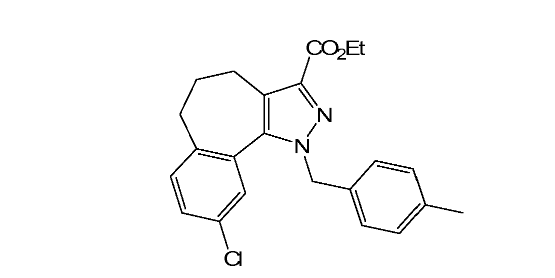

The same procedure described for the preparation of compound 1.1b was repeated, but substituting 2,4-dichloro phenylhydrazine hydrochloride with 4-methylbenzylhydrazine hydrochloride (3.90 mmoles). After filtration, a solid was recovered that after washing and drying, was identified as the compound ethyl 9-chloro-1-(4'-methylbenzyl)-1,4,5,6-tetrahydrobenzo[6,7] cyclohepta[1,2-*c*]pyrazol-3-carboxylate. Yield: 69%. Rf = 0.40 (oil ligroin/AcOEt 8.5:1.5 v/v); IR (nujol) (λ= cm⁻¹) 1719; ¹H-NMR (CDCl₃) δ 1.42 (t, 3H, J = 7.2 Hz); 2.15-2.39 (m, 2H); 2.37 (s, 3H); 2.64 (t, 2H, J = 6.7 Hz); 3.03-3.35 (m, 2H); 3.56 (s, 2H); 4.45 (q, 2H, J = 7.2 Hz); 6.61 (d, 1H, J = 8.0 Hz); 7.02 (dd, 1H, J = 2.0 and 8.0 Hz); 7.25 (d, 1H, J = 2.0 Hz); 7.26-7.34 (m, 4H). Anal. calc. for C₂₃H₂₃ClN₂O₂: C, 69.95; H, 5.87; Cl, 8.98; N, 7.09. Found: C, 69.91; H, 5.86; Cl, 8,96; N, 7.08.

### EXAMPLE 1.3

### Preparation of ethyl 8-chloro-1-(2',4'-dichlorophenyl)-1,4,5,6-tetrahydrobenzo[6,7]cyclohepta[1,2-c]pyrazol-3-carboxylate

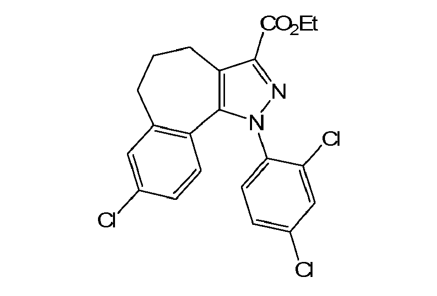

### 1.3a Preparation of ethyl α-(7-chloro-1-oxo-2,3,4,5-tetrahydro-benzocyclohepten-2-yl)-α-oxo-acetate

The same procedure described in example 1.1a was repeated but substituting the compound 8-chloro-2,3,4,5-tetrahydro-benzocycloheptan-1-one with 7-choro-2,3,4,5-tetrahydrobenzo cycloheptan-1-one (12.84 mmoles). The compound ethyl α-(7-choro-1-oxo-2,3,4,5-tetrahydro-benzocyclohepten-2 yl)-α-oxo-acetate (Compound 1.3a) was obtained with a 82% yield. Rf = 0.71 (oil ligroin/AcOEt 1:1 volume/volume); IR (nujol) (λ= cm⁻¹) 3440, 1730, 1680; ¹H-NMR (CDCl₃) δ 1.41 (t, 3H, J = 7.0 Hz); 2.07 (q, 2H, J = 6.8 Hz); 2.32 (t, 2H, J = 6.4 Hz); 2.72 (t, 2H, J = 6.8 Hz); 4.34 (q, 2H, J = 7.0 Hz); 7.22-7.37 (m, 2H); 7.58 (d, 1H, J = 8.2 Hz); 15.37 (bs, 1H). Anal. calc. for C₁₅H₁₅ClO₄: C, 61.13; H, 5.13; Cl, 12.03. Found: C, 61.01; H, 5.11; Cl, 12.00.

### 1.3b Preparation of ethyl 8-chloro-1-(2',4'-dichlorophenyl)-1,4,5,6-tetrahydrobenzo[6,7]cyclohepta[1,2-c]pyrazol-3-carboxylate

The same procedure described in example 1.1b was repeated but the compound reacted with 2,4-dichlorophenylhydrazine hydrochloride (3.90 mmoles) was the compound 1.3a (3.39 mmoles). The compound ethyl 8-chloro-1-(2',4'-dichlorophenyl)-1,4,5,6-tetrahydrobenzo[6,7]cyclohepta[1,2-*c*]pyrazol-3-carboxylate was obtained with a 68% yield. Rf = 0.39 (oil ligroin/AcOEt 8.5:1.5 v/v); IR (nujol) (λ= cm⁻¹) 1724; ¹H-NMR (CDCl₃) δ 1.43 (t, 3H, J = 7.2 Hz); 2.20-2.36 (m, 2H); 2.66 (t, 2H, J = 6.4 Hz); 3.10-3.30 (m, 2H); 4.45 (q, 2H, J = 7.2 Hz); 6.60 (d, 1H, J = 8.4 Hz); 7.02 (dd, 1H, J = 2.0 e 8.4 Hz); 7.31 (d, 1H, J = 2.0 Hz); 7.37-7.42 (m, 2H); 7.54 (d, 1H, J = 9.2 Hz). Anal. calc. for C₂₁H₁₇Cl₃N₂O₂: C, 57.89; H, 3.93; Cl, 24.41; N, 6.43. Found: C, 57.74; H, 3.92; Cl, 24.39; N, 6.41.

### EXAMPLE 1.4

### Preparation of ethyl 8-chloro-1-(4'-methylbenzyl)-1,4,5,6-tetrahydrobenzo[6,7]cyclohepta[1,2-c]pyrazol-3-carboxylate

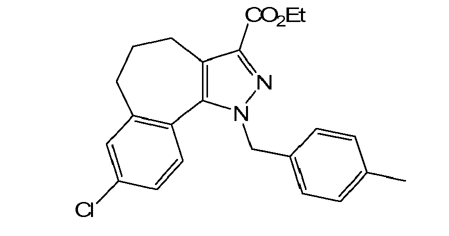

The same procedure described in example 1.1b was repeated but the compound reacting with 4-methylbenzylhydrazine hydrochloride (3.90 mmoles) was the compound 1.3a (3.39 mmoles). The compound ethyl 8-chloro-1-(4'-methylbenzyl)-1,4,5,6-tetrahydro benzo[6,7] cyclohepta-[1,2-*c*]pyrazol-3-carboxylate was obtained with a 73% yield. Rf = 0.42 (oil ligroin/AcOEt 8.5:1.5 v/v); IR (nujol) (λ= cm⁻¹) 1725; ¹H-NMR (CDCl₃) δ 1.41 (t, 3H, J = 7.1 Hz); 2.18-2.38 (m, 2H); 2.36 (s, 3H); 2.65 (t, 2H, J = 6.6 Hz); 3.05-3.32 (m, 2H); 3.55 (s, 2H); 4.44 (q, 2H, J = 7.1 Hz); 6.59 (d, 1H, J = 8.2 Hz); 7.00 (dd, 1H, J = 2.2 and 8.1 Hz); 7.22 (d, 1H, J = 2.1 Hz); 7.24-7.32 (m, 4H). Anal. calc. for C₂₃H₂₃ClN₂O₂: C, 69.95; H, 5.87; Cl, 8.98; N, 7.09. Found: C, 69.88; H, 5.85; Cl, 8.97; N, 7.07.

### EXAMPLE 1.5

### Preparation of ethyl 8-chloro-1-(2',4'-dichlorophenyl)-4,5-dihydrobenzo-1H-6oxa-cyclohepta[1,2-c]pyrazol-3-carboxylate

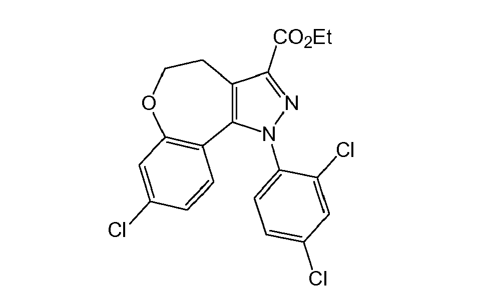

### 1.5a Preparation of the 4-(3-chlorophenoxy)butyric acid

1 eq of NaOH (flakes) was dispersed in 1 eq of 3-chlorophenol and the thus obtained dispersion heated to 170°C up to complete solubilization of the base. 1.4 eq of γ-butyrolactone were dropwise added to the solution, and the reaction mixture maintained at 170°C for 5 hours. The reaction mixture was then poured into ice and then acidified with HCl 6N. The reaction product was extracted with CHCl₃, dehydrated with Na₂SO₄ and concentrated under vacuum. The obtained residue was purified by flash chromatography (oil ligroin/ethyl acetate 4:1 volume/volume). The acid 4-(3-chloro phenoxy)butyric (yellow solid) was obtained with a 47% yield. R_{f} = 0.15 (oil ligroin/ethyl acetate 4:1); m.p. 47°C; IR (nujol) (λ= cm⁻¹) 3223 (OH), 1709 (CO); ¹H-NMR (CDCl₃) δ 2.11 (qu, 2H, J = 6.8 Hz), 2.58 (t, 2H, J = 7.4 Hz), 4.0 (t, 2H, J = 5.6 Hz), 6.76 (d, 1H, J = 8.2 Hz), 6.88 (s, 1H), 6.92 (d, 1H, J = 9.2 Hz), 7.18 (t, 1H, J = 7.8 Hz); Anal. Calc. for C₁₀H₁₁ClO₃: C, 55.98; H, 5.17; Cl, 16.51. Found: C, 55.84; H, 5.16; Cl, 16.50.

### 1.5b Preparation of the compound 8-chloro-1-oxo-2,3,4,5-tetrahydrobenzocycloheptan-5-one

27.96 mmoles of the 4-(3-chlorophenoxy)butyric acid obtained in example 1.5a were added to 48 grams of polyphosphoric acid; the resulting mixture was maintained under stirring at 90°C for 2 hours and then poured on ice. The reaction mixture was extracted with CH₂Cl₂, the pooled organic phases washed with an aqueous solution of Na₂CO₃ at 10%, dehydrated on Na₂SO₄ and concentrated under vacuum. The obtained residue was purified by flash chromatography (oil ligroin/ethyl acetate 9:1 v/v). The compound 8-chloro-1-oxo-2,3,4,5-tetrahydrobenzo-cycloheptan-5-one was separated as an orange coloured oil in a 46% yield. B.p. 46-47°C/27mmHg; ¹H-NMR (CDCl₃) δ 2.22 (qu, 2H, J = 6.4 Hz), 2.89 (t, 2H, J = 6.8 Hz), 4.25 (t, 2H, J = 6.6 Hz), 7.05-7.16 (m, 2H), 7.71 (d, 1H, J = 7. 0 Hz) ; Anal. Calc. for C₁₀H₉ClO₂: C, 61.09; H, 4.61; Cl, 18.03. Found: C, 60.93; H, 4.60; Cl, 18.01.

### 1.5c Preparation of diketoester ethyl γ-(7-chloro-5-oxo-2,3,4,5-tetrahydrobenzocycloheptan-2-yl)-α-oxoacetate

2 eq of metal sodium were added to 5 ml of anhydrous ethanol. The obtained dispersion was maintained under stirring at room temperature up to complete sodium reaction. 1 eq of ethyl oxalate and 30 ml of a solution of the ketonic compound obtained in example 1.5b (1 eq) in anhydrous ethanol were added to the formerly prepared solution. The reaction mixture was maintained under stirring at room temperature for 1.5 hours and then poured on a mixture of ice + HCl 2N. The obtained solution was extracted with ethyl acetate. The organic phase was recovered and washed with water, dehydrated on Na₂SO₄ and concentrated under vacuum. The residue was purified by flash chromatography (oil ligroin/ethyl acetate 4:1 volume/volume). The compound ethyl γ-(7-chloro-5-oxo-2,3,4,5-tetrahydrobenzo-cycloheptan-2-yl)-α-oxoacetate was thus separated in a 90% yield. R_{f} = 0.46 (oil ligroin/ethyl acetate 4:1); m.p. 135 °C; IR (nujol) (λ= cm⁻¹) 1823 (CO), 1713 (CO), 1683 (CO); ¹H-NMR (CDCl₃/DMSO) δ 1.09 (t, 3H, J = 7.2 Hz), 1.27 (t, 3H, J = 6.8 Hz), 3.34-3.38 (m, 2H), 4.17 (q, 2H, J = 7.0 Hz), 6.89 (s, 1H), 7.01 (d, 1H, J = 6.2 Hz), 7.62 (d, 1H, J = 8.4 Hz) ; Anal. Calc. for (C₁₄H₁₃ClO₅) : C, 56.67; H, 4.41; Cl, 11.95. Found: C, 56.58; H, 4.37; Cl, 11.94.

### 1.5d Preparation of the compound ethyl 8-chloro-1-(2',4'-dichlorophenyl)-4,5-dihydrobenzo-1H-6oxa-CyClohepta[1,2-c]-pyrazol-3-carboxylate

1 eq of the diketoester obtained in example 1.5c and 1.1 eq of 2,4-dichlorophenylhydrazine hydrochloride in 50 ml of ethanol were heated at reflux for 90 minutes. The reaction solvent was then removed under vacuum and the obtained residue purified by flash chromatography (oil ligroin/EtOAc 9:1). The compound ethyl 8-chloro-1-(2',4'-dichlorophenyl)-4,5-dihydrobenzo-1*H*-6oxa-cyclo-hepta[1,2-*c*]pyrazol-3-carboxylate was thus obtained as an orange solid (47.5% yield). *R_{f}* = 0.32 (oil ligroin/EtOAc 9:1); m.p. 130-131 °C; IR (nujol) (λ= cm⁻¹) 1712 (CO); ¹H-NMR (CDCl₃) δ 1.42 (t, 3H, J = 7.0 Hz), 3.44 (qu, 2H, J = 5.4 Hz), 4.35-4.51 (m, 4H), 6.65 (d, 1H, J = 8.6 Hz), 6.81 (d, 1H, J = 8.6 Hz), 7.14 (s, 1H) 7.39-7.44 (m, 2H), 7.49 (s, 1H); Anal. Calc. for C₂₀H₁₅Cl₃N₂O₂: C, 54.88; H, 24.30; Cl, 6.40; N, 3.45. Found: C, 54.80; H, 24.26; Cl, 6.39; N, 3.44.

### EXAMPLE 1.6

### Preparation of ethyl 7-chloro-1-(2',4'-dichlorophenyl)-6-methyl-1,4-dihydroindeno[1,2-c]pyrazole-3-carboxylate

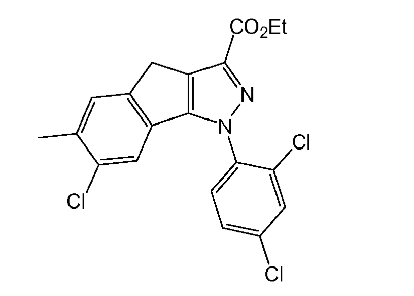

### 1.6a Preparation of ethyl 2-(6-chloro-5-methyl-1-oxo-2,3-dihydro-1H-inden-2-yl)-2-oxoacetate

Metal sodium (0.17 g, 7.5 mmol) was added in small pieces to absolute ethanol (3.5 ml) and the mixture left under stirring until complete solubilization. Diethyloxalate (0.51 ml, 3.75 mmol) was added to the alcohol solution, followed by a dropwise addition of a solution of 6-chloro-5-methylindan-1-one (12.21 mmol) in absolute ethanol (27 ml). The reaction mixture was stirred at room temperature for 9 hours. The reaction was stopped by pouring the liquid phase on a mixture of ice and HCl 1N, followed by extraction with chloroform (3 x 15 ml). The combined extracts were washed with water, dried over anhydrous sodium sulfate, filtered, and evaporated under reduced pressure. The compound ethyl 2-(6-chloro-5-methyl-1-oxo-2,3-dihydro-1*H*-inden-2-yl)-2-oxoacetate was isolated as an orange oil (96% yield), having an analytical grade purity. Rf=0.21 (petroleum ether/ethyl acetate 9/1 v/v); IR (nujol) (λ = cm⁻¹) 3440, 1730, 1680; ¹H-NMR (CDCl₃) δ 1.43 (t, 3H, J = 7.2 Hz); 2.49 (s, 3H); 3.92 (s, 2H); 4.42 (q, 2H, J = 7.2 Hz); 7.42 (s, 1H); 7.82 (s, 1H); 13.20 (bs, 1H). Anal. calc. for C₁₄H₁₃ClO₄: C, 59.90; H, 4.67; Cl, 12.63. Found: C, 58.10; H, 4.71; Cl, 12.67.

### 1.6b Preparation of ethyl 7-chloro-1-(2',4'-dichlorophenyl)-6-methyl-1,4-dihydroindeno[1,2-c]pyrazole-3-carboxylate

A mixture of compound 1.6a (0.9 g, 3.05 mmol) and 2,4-dichlorophenylhydrazine hydrochloride (0.72 g, 3.38 mmol) in ethyl alcohol (21 ml) was stirred at the reflux temperature for 8 hours. The solvent was then removed under reduced pressure and the crude ester was isolated. Purification of said compound by flash chromatography on silica gel, elution solvent petroleum ether/ethyl acetate (8.5/1.5 v/v) gave the compound ethyl 7-chloro-1-(2',4'-dichlorophenyl)-6-methyl-1,4-dihydroindeno[1,2-*c*]pyrazole-3-carboxylate as a yellow solid (99% yield).

IR (nujol) (λ = cm⁻¹) 1725; ¹H-NMR (CDCl₃) δ 1.44 (t, 3H, J = 7.0 Hz); 2.41 (s, 3H); 3.80 (s, 2H); 4.44 (q, 2H, J = 7.2 Hz); 6.93 (s, 1H); 7.43-7.75 (m, 4H). Anal. calc. for C₂₀H₁₅Cl₃N₂O₂: C, 56.96; H, 3.59; Cl, 25.22; N, 6.64. Found: C, 57.16; H, 3.61; Cl, 25.26; N, 6.67.

### EXAMPLE 1.7

### Preparation of ethyl 7-chloro-6-methyl-1-(4'-methylbenzyl)-1,4-di hydroindeno[1,2-c]pyrazole-3-carboxylate

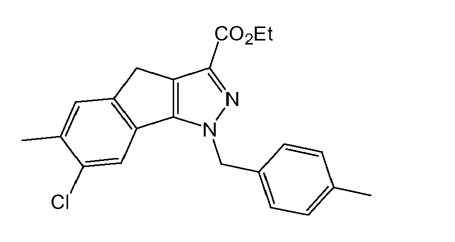

The same procedure described in Example 1.6b was repeated but that compound 1.6a was reacted with 4-methylbenzylhydrazine hydro chloride instead of 2,4-dichlorophenylhydrazine hydrochloride. Yield 95%. IR (nujol) (λ = cm⁻¹) 1724; ¹H-NMR (CDCl₃) δ 1.44 (t, 3H, J = 7.2 Hz); 2.32 (s, 3H); 2.34 (s, 3H); 3.70 (s, 2H) ; 4.45 (q, 2H, J = 7.0 Hz); 5.59 (s, 2H); 6.98-7.39 (m, 6H). Anal. calc. for C₂₂H₂₁ClN₂O₂: C, 69.38; H, 5.56; Cl, 9.31; N, 7.36. Found: C, 69.78; H, 5.53; Cl, 9.34; N, 7.39.

### EXAMPLE 1.8

### Preparation of ethyl 6-chloro-7-methyl-1-(4'-methylbenzyl)-1,4-di hydroindeno[1,2-c]pyrazole-3-carboxylate

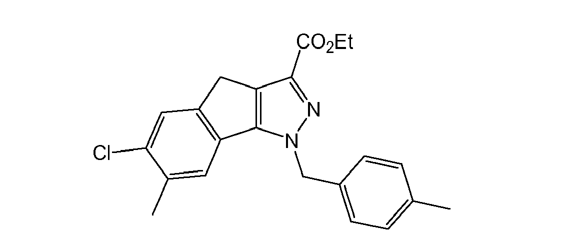

### 1.8a Preparation of ethyl 2-(5-chloro-6-methyl-1-oxo-2,3-dihydro-1H-inden-2-yl)-2-oxoacetate

The same procedure described in ex. 1.6a was repeated, but for dripping in the initial alcoholic solution 5-chloro-6-methylindan-1-one (8.99 mmol) instead of 6-chloro-5-methylindan-1-one. Yield 87%. Rf=0.50 (petroleum ether/ethyl acetate 7/3 v/v); IR (nujol) (λ = cm⁻¹) 3445, 1725, 1685; ¹H-NMR (CDCl₃) δ 1.43 (t, 3H, J = 7.2 Hz); 2.45 (s, 3H); 3.92 (s, 2H); 4.42 (q, 2H, J = 7.2 Hz); 7.54 (s, 1H); 7.71 (s, 1H); 14.50 (bs, 1H). Anal. calc. for C₁₄H₁₃ClO₄: C, 59. 90; H, 4.67; Cl, 12.63. Found: C, 57.60; H, 4.69; Cl, 12.66.

### 1.8b Preparation of ethyl 6-chloro-7-methyl-1-(4'-methylbenzyl)-1,4-dihydroindeno[1,2-c]pyrazole-3-carboxylate

The same procedure described in ex. 1.6b was repeated but for reacting the compound 1.8a with 4-methylbenzylhydrazine hydro chloride instead of 2,4-dichlorophenylhydrazine hydrochloride. Yield 88%. Rf=0.21 (petroleum ether/ethyl acetate 9:1). IR (nujol) (λ = cm⁻¹) 1725; ¹H-NMR (CDCl₃) δ 1.43 (t, 3H, J = 7.2 Hz); 2.31 (s, 3H); 2.38 (s, 3H); 3.69 (s, 2H); 4.45 (q, 2H, J = 7.0 Hz); 5.56 (s, 2H); 7.00-7.40 (m, 6H). Anal. calc. for C₂₂H₂₁ClN₂O₂: C, 69.38; H, 5.56; Cl, 9.31; N, 7.36. Found: C, 69.31; H, 5.54; Cl, 9.30; N, 7.34.

### EXAMPLE 1.9

### Preparation of the ethyl ester of the 1-(2,4-dichlorophenyl)-6-methyl-1H-benzofuro[3,2-c]pyrazole-3-carboxylic acid

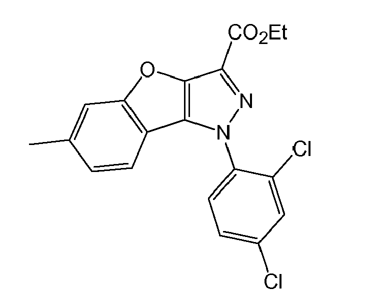

### 1.9a Preparation of 6-methylbenzofuran-3(2H)-one

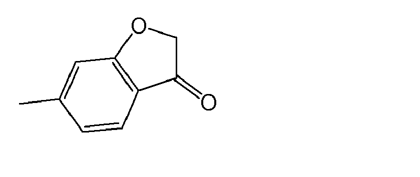

1-bromo-2-(2-hydroxy-4-methylphenyl)acetophenone was obtained according to the synthesis described by L. C. King et al. in J. Org. Chem. 29 (1964) 3459-3461, by reacting 1-(2-hydroxy-4-methyl-phenyl)-ethanone with CuBr₂ in ethyl-acetate at 77 °C.

A solution of 1-bromo-2-(2-hydroxy-4-methylphenyl)-acetophenone (1.0 g, 4.36 mmol) and sodium acetate (0.36 g, 4.38 mmol) in absolute ethanol (10 ml) was refluxed under stirring for 15 hours. The obtained mixture was poured into water and extracted with dichloromethane (3 x 10 ml). The organic phase was dried over Na₂SO₄, then concentrated under reduced pressure to obtain an oil which was purified by flash chromatography (oil ether/ethyl ether 9/1 v/v on silica gel). 0.25 g (38% yield) of a yellow solid, corresponding to 6-methylbenzofuran-3(2H)-one were recovered.

### 1.9b Preparation of ethyl 2-(3-hydroxy-6-methylbenzofuran-2yl)-2-oxoacetate

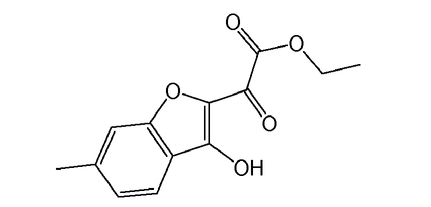

Metal sodium (0.13 g; 2.24 mmol) was added in small pieces to absolute ethanol (3 ml). The suspsension was left under reflux until complete solubilization of sodium. To the so obtained solution diethyloxalate (0.80 ml; 5.87 mmol) was added, followed by dripping a solution of 1-bromo-2-(2-hydroxy-4-methylphenyl)acetophenone (0.39 g; 2.63 mmol) in absolute ethanol (30 ml). The reaction mixture was kept under stirring at room temperature for 20 hours, then poured in a mixture of ice and HCl 1N. The aqueous solution is extracted with chloroform (3 x 20 ml). The organic phase was dried over Na₂SO₄, then concentrated under reduced pressure to obtain an oil which is triturated with oil ether/ethyl ether. 0.47 g (73% yield) of the compound are recovered under the form of a yellow solid. Rf=0.48 (dichloromethane/ acetone 7/3); m.p.: 113-115 °C; IR (nujol) (λ = cm⁻¹) 3406 (OH as tautomer mixture), 1691 (COOEt), 1651 (C=O); ¹H-NMR (CDCl₃) δ 1.50 (t, J=7.2 Hz, 3H), 2.51 (s,3H), 4.56 (q, J=7.2 Hz, 2H), 7.13 (d, J=8.4 Hz, 1H), 7.26 (s, 1H), 7.71 (d, J=8.4 Hz, 1H), 11.87 (brs, 1H); API-ESI (Atmospheric Pressure Ionization - Electron Spray Ionization) calc. for 248.23, found 248.10.

### 1.9c Preparation of (Z)-ethyl 2-(2-(2,4-dichlorophenyl)-hydrazone)-2-(3-hydroxy-6-methylbenzofuran-2-yl)acetate

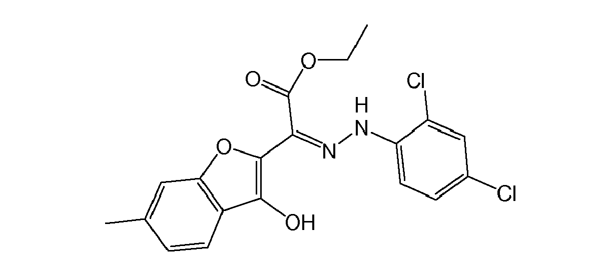

A solution in absolute ethanol (1.15 ml) of the compound obtained in Ex. 1.9b (1.07g; 4.31 mmol)and 2,4-dichlorophenylhydrazine hydrochloride (1.20 g; 5.60 mmol) was prepared. The solution was reacted at the reflux temperature for 1.5 hours, then cooled to room temperature and poured on ice. The resulting precipitate was filtered under reduced pressure, then air dried to obtain 1.37 g (78% yield) of a solid residue corresponding to (Z)-ethyl 2-(2-(2,4-dichlorophenyl)hydrazone)-2-(3-hydroxy-6-methylbenzofuran-2-yl)acetate. Rf=0.42 (oil ether/ethyl acetate 9.5/0.5 v/v on silica gel); m.p.: 190-192 °C; IR (nujol) (λ = cm⁻¹) 3423 (OH as tautomer mixture), 1619 (COOEt); ¹H-NMR (CDCl₃) δ 0.83 (t, J=7.4 Hz, 3H), 2.46 (s, 3H), 4.07 (q, J=7.0 Hz, 2H), 6.93 (d, J=0.8 Hz), 7.35 (s, 2H), 7.60 (d, J=8.6 Hz, 2H), 12.80 (s, 1H); API-ESI calc. for 407.25, found 407.10.

### 1.9d Preparation of the ethyl ester of the 1-(2,4-dichlorophenyl)-6-methyl-1H-benzofuro[3,2-c]pyrazole-3-carboxylic acid

To a solution in toluene (6 ml) of the compound prepared in Ex. 1.9d (0.5g; 1.23 mmol) a catalytic amount of p-toluensulfonic acid (0.023 g, 0.123 mmol) was added. The obtained mixture was reacted at the reflux temperature for 30 hours, then the solvent was removed under reduced pressure. The residue was purified by flash chromatography (oil ether/ethyl ether 8/2 v/v on silica gel). 0.25 g (52% yield) of a yellow solid, corresponding to ethyl ester of 1-(2,4-dichlorophenyl)-6-methyl-1H-benzofuro[3,2-c]pyrazole-3-carboxylic acid were recovered. Rf=0.30 (oil ether/ethyl ether 8/2 volume/volume on silica gel); m.p.: 138-140 °C; IR (nujol) (λ=cm⁻¹) 1635 (COOEt); ¹H-NMR (CDCl₃) δ 1.49 (t, J=7.2 Hz, 3H), 2.51 (s, 3H), 4.55 (q, J=7.4 Hz, 2H), 7.10 (d, J=8.6 Hz, 1H), 7.31 (d, J=8.0 Hz, 1H), 7.43-7.48 (m, 2H), 7.64-7.67 (m, 2H); API-ESI calc. for 389.23, found 389.05.

### EXAMPLE 2.1

### Preparation of the 9-chloro-1-(2',4'-dichlorophenyl)-1,4,5,6-tetrahydrobenzo[6,7]cyclohepta[1,2-c]pyrazol-3-carboxylic acid

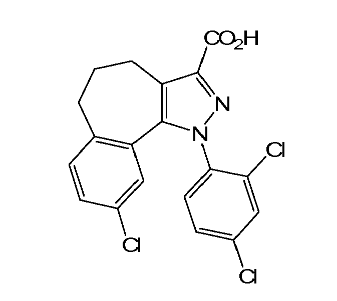

1.00 grams (2.29 mmoles) of the ester obtained in example 1.1b were solubilized in 15 ml of EtOH/H₂O 1:1 (v/v). 1.67 grams (29.77 mmoles) of solid KOH were added to the formerly prepared solution. The reaction mixture was kept under stirring at the reflux temperature for 4 hours and then poured into a mixture of ice + HCl 1N. The so obtained white precipitate was filtered, washed with water and dried in the air obtaining 0.91 g of the 9-chloro-1-(2',4'-dichlorophenyl)-1,4,5,6-tetrahydrobenzo [6,7]cyclohepta[1,2-*c*] pyrazol-3-carboxylic acid (Compound 2.1). Yield 98%. Rf = 0.60 (CHCl₃/MeOH 8.5:1.5 volume/volume); IR (nujol) (λ= cm⁻¹) 3419, 1716; ¹H-NMR (CDCl₃) δ 2.25-2.27 (m, 2H); 2.67 (t, 2H, J = 6.4 Hz); 3.07-3.32 (m, 2H); 4.78 (bs, 1H); 6.65 (d, 1H, J = 1.8 Hz); 7.20-7.32 (m, 2H); 7.40-7.50 (m, 2H); 7.57 (d, 1H, J = 9.0 Hz). Anal. calc. for C₁₉H₁₃Cl₃N₂O₂: C, 55.98; H, 3.21; Cl, 26.09; N, 6.87. Found: C, 55.85; H, 3.19; Cl, 26.05; N, 6.86.

### EXAMPLE 2.2

### Preparation of the 9-chloro-1-(4'-methylbenzyl)-1,4,5,6-tetrahydrobenzo[6,7]cyclohepta[1,2-c]pyrazol-3-carboxylic acid

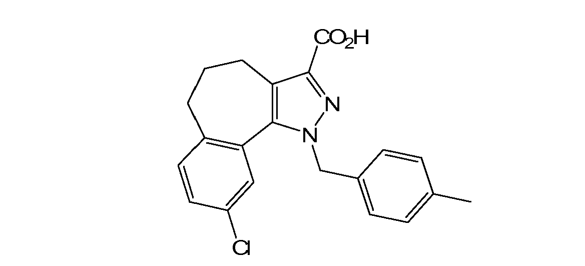

The same procedure described in example 2.1 was repeated, but substituting the ester obtained in example 1.1 with that of example 1.2. The 9-chloro-1-(4'-methylbenzyl)-1,4,5,6-tetrahydro-benzo[6,7]cyclohepta[1,2-*c*]pyrazol-3-carboxylic acid was obtained with a 94% yield. Rf = 0.36 (oil ligroin/AcOEt 8.5:1.5 v/v); IR (nujol) (λ= cm⁻¹) 3422, 1717; ¹H-NMR (CDCl₃) δ 2.18-2.38 (m, 2H); 2.36 (s, 3H); 266 (t, 2H, J = 6.8 Hz); 3.05-3.36 (m, 2H); 3.58 (s, 2H); 6.63 (d, 1H, J = 8.1 Hz); 7.01 (dd, 1H, J = 2.2 and 8.1 Hz); 7.22 (d, 1H, J = 2.1 Hz); 7.24-7.35 (m, 4H). Anal. calc. for C₂₁H₁₉ClN₂O₂: C, 68.76; H, 5.22; Cl, 9.66; N, 7.64. Found: C, 68.61; H, 5.21; Cl, 9.64; N, 7.62.

### EXAMPLE 2.3

### Preparation of the 8-chloro-1-(2',4'-dichlorophenyl)-1,4,5,6-tetrahydrobenzo[6,7]cyclohepta[1,2-c]pyrazol-3-carboxylic acid

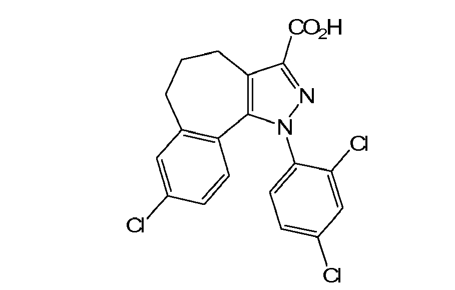

The same procedure described in example 2.1 was repeated, but substituting the ester obtained in example 1.1 with that of example 1.3. The 8-chloro-1-(2',4'-dichlorophenyl)-1,4,5,6-tetrahydrobenzo[6,7]cyclo hepta[1,2-c]pyrazol-3-carboxylic acid was thus obtained with a 94% yield. IR (nujol) (λ= cm⁻¹) 3410, 1715; ¹H-NMR (CDCl₃) δ 2.25-2.30 (m, 2H); 2.68 (t, 2H, J = 6.4 Hz); 3.10-3.23 (m, 2H); 4.50 (bs, 1H); 6.61 (d, 1H, J = 8.4 Hz); 7.03 (dd, 1H, J = 2.2 and 8.2 Hz); 7.32 (d, 1H, J = 2.0 Hz); 7.39-7.44 (m, 2H); 7.52 (d, 1H, J = 8.0 Hz). Anal. calc. for C₁₉H₁₃Cl₃N₂O₂: C, 55.98; H, 3.21; Cl, 26.09; N, 6.87. Found: C, 55.82; H, 3.20; Cl, 26.06; N, 6.85.

### EXAMPLE 2.4

### Preparation of the 8-chloro-1-(4'-methylbenzyl)-1,4,5,6-tetrahydrobenzo[6,7]cyclohepta[1,2-c]pyrazol-3-carboxylic acid

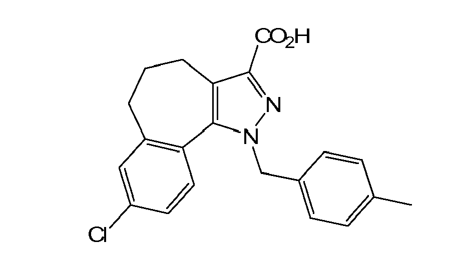

The same procedure described in example 2.1 was repeated, but substituting the ester obtained in example 1.1 with that of example 1.4. The 8-chloro-1-(4'-methylbenzyl)-1,4,5,6-tetrahydrobenzo[6,7]cyclohepta[1,2-*c*]pyrazol-3-carboxylic acid was thus obtained with a 93% yield. IR (nujol) (λ= cm⁻¹) 3412, 1716; ¹H-NMR (CDCl₃) δ 2.15-2.36 (m, 2H); 2.35 (s, 3H); 2.66 (t, 2H, J = 6.8 Hz); 3.04-3.31 (m, 2H); 3.54 (s, 2H); 6.57 (d, 1H, J = 8.1 Hz); 7.01 (dd, 1H, J = 2.0 and 8,1 Hz); 7.23 (d, 1H, J = 2.0 Hz); 7.22-7.31 (m, 4H). Anal. calc. for C₂₁H₁₉ClN₂O₂: C, 68.76; H, 5.22; Cl, 9.66; N, 7.64. Found: C, 68.69; H, 5.20; Cl, 9.65; N, 7.63.

### EXAMPLE 2.5

### Preparation of the 8-chloro-1-(2',4'-dichlorophenyl)-4,5-dihydrobenzo-1H-6oxa-cyclohepta[1,2-c]pyrazol-3-carboxylic acid

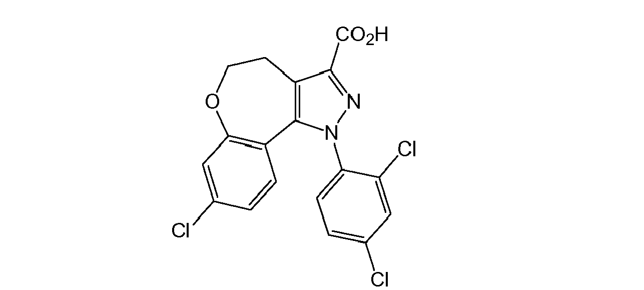

An amount equal to 1 equivalent of the ester obtained in example 1.5 was dispersed in 10 ml of CH₃OH. To said dispersion 7 ml of CH₃OH containing 2 eq of potassium hydroxide were added. The methanol solution thus prepared was maintained at reflux for 12 hours and then poured into a mixture of ice and HCl 1N. A yellow precipitate was thus obtained which was filtered, washed with water and then dried under nitrogen flow. The compound 8-chloro-1-(2',4'-dichlorophenyl)-4,5-dihydrobenzo-1*H*-6oxa-cyclohepta[1,2-*c*]pyrazol-3-carboxylic acid was isolated with a 91.2% yield. *R_{f}*=0.38 (CHCl₃/MeOH 9:1); m.p. 230-231°C; IR (nujol) (λ= cm⁻¹) 1689 (CO); ¹H-NMR (CDCl₃/DMSO) δ 3.10-3.45 (br s, 3H, 10H exchang. with D₂O), 6.67 (d, 1H, J = 8.4 Hz), 6.83 (d, 1H, J = 8.2 Hz), 7.13 (s, 1H), 7.44-7.50 (m, 3H); Anal. Calc. for C₁₈H₁₁Cl₃N₂O₂: C, 52.77; H, 2.70; Cl, 25.96; N, 6.83. Found: C, 52.65; H, 2.69; Cl, 25.94; N, 6.81.

### EXAMPLE 2.6

### Preparation of 7-chloro-1-(2',4'-dichlorophenyl)-6-methyl-1,4-di hydroindeno[1,2-c]pyrazole-3-carboxylic acid

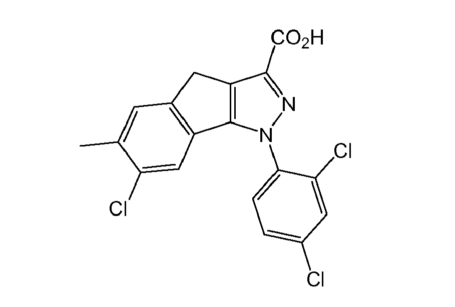

A mixture of ethyl ester 1.6 (0.64 g, 1.47 mmol) and KOH (0.17 g, 2.94 mmol) in methanol (12 ml) was refluxed for 12 hours. After said period of time a solution was formed, that was then cooled to room temperature and poured on a mixture of ice and HCl 1N. A precipitate was separated. The precipitate was filtered, washed with water, and dried under vacuum. It was obtained the compound 7-chloro-1-(2',4'-dichlorophenyl)-6-methyl-1,4-dihydroindeno[1,2-*c*]pyrazole-3-carboxylic acid as a white solid. Yield 98%. IR (nujol) (λ.= cm⁻¹) 3410, 1690; ¹H-NMR (DMSO) δ 2.41 (s, 3H); 3.79 (s, 2H); 6.94 (s, 1H); 7.35-7.75 (m, 4H). Anal. calc. for C₁₈H₁₁Cl₃N₂O₂: C, 54.92; H, 2.82; Cl, 27.02; N, 7.12. Found: C, 54.78; H, 2.80; Cl, 26.99; N, 7.11.

### EXAMPLE 2.7

### Preparation of 7-chloro-6-methyl-1-(4'-methylbenzyl)-1,4-dihydro indeno[1,2-c]pyrazole-3-carboxylic acid

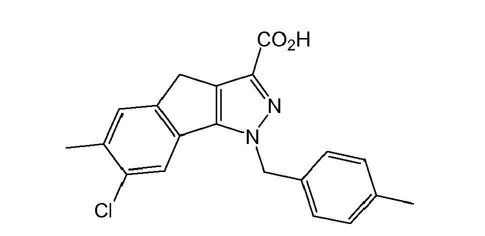

The same procedure of ex. 2.6 was repeated to convert the ethyl ester compound of ex. 1.7 into the corresponding acid. The compound 7-chloro-6-methyl-1-(4'-methylbenzyl)-1,4-dihydroindeno [1,2-c] pyrazole-3-carboxylic acid was obtained (yield 96%). IR (nujol) (λ.= cm⁻¹) 3410, 1690; ¹H-NMR (DMSO) δ 2.27 (s, 3H); 2.36 (s, 3H); 3.66 (s, 2H); 5.67 (s, 2H); 6.99-7.40 (m, 4H); 7.51 (s, 1H); 7.57 (s, 1H); 12.70 (bs, 1H). Anal. calc. for C₂₀H₁₇ClN₂O₂: C, 68.09; H, 4.86; Cl, 10.05; N, 7.94. Found: C, 68.02; H, 4.84; Cl, 10.03; N, 7.93.

### EXAMPLE 2.8

### Preparation of 6-chloro-7-methyl-1-(4'-methylbenzyl)-1,4-dihydro indeno[1,2-c]pyrazole-3-carboxylic acid

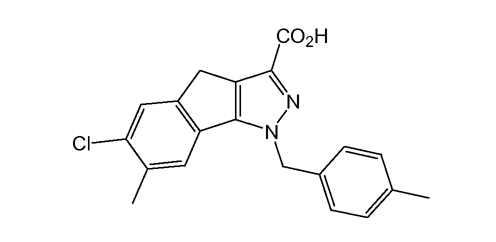

The same procedure of ex. 2.6 was repeated to convert the ethyl ester of ex. 1.8 into the corresponding acid. The compound 6-chloro-7-methyl-1-(4'-methylbenzyl)-1,4-dihydroindeno[1,2-*c*] pyrazole-3-carboxylic acid was isolated (yield 85%). IR (nujol) (λ.= cm⁻¹) 3410, 1690; ¹H-NMR (DMSO) δ . 2.26 (s, 3H); 2.36 (s, 3H); 3.66 (s, 2H); 5.67 (s, 2H); 7.16 (m, 4H); 7.40-7.70 (m, 2H); 12.70 (bs, 1H). Anal. calc. for C₂₀H₁₇ClN₂O₂: C, 68.09; H, 4.86; Cl, 10.05; N, 7.94. Found: C, 68.07; H, 4.85; Cl, 10.02; N, 7.92.

### EXAMPLE 2.9

### Preparation of 1-(2,4-dichlorophenyl)-6-methyl-1H-benzofuro-[3,2-c]pyrazole-3-carboxylic acid

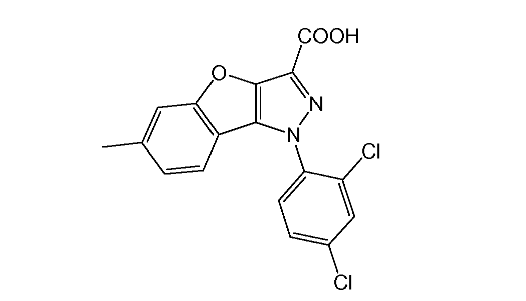

The compound obtained in Ex. 1.9 (0.17 g, 0.44 mmol) and potassium hydroxide (0.32 g, 5.7 mmol) were reacted in ethanol/water 1/1 (v/v) solution (5.6) ml at the reflux temperature for 4 hours. The reacted mixture was cooled to room temperature and then poured into a mixture of ice and HCl 1N. A precipitate was formed, that was filtered under reduced pressure, washed with water and air-dried. A solid residue corresponding to 1-(2,4-dichlorophenyl)-6-methyl-1H-benzofuro[3,2-c]pyrazole-3-carboxylic acid was obtained. Yield was quantitative. Rf=0.30 (chloroform/methanol 8/2 v/v on silica gel); m.p.: 228-230°C; IR (nujol) (λ = cm⁻¹) 3417 (OH), 1637 (COOH); ¹H-NMR (CDCl₃) δ 2.51 (s, 3H), 3.57 (bs, 1H), 7.13 (d, J=8.2 Hz, 2H), 7.34 (d, J=7.8 Hz), 7.45-7.55 (m, 2H), 7.67-7.72 (m, 2H); API-ESI calc. for 361.18, found 360.15.

### EXAMPLE 3.1

### Preparation of N-myrtanyl-9-chloro-1-(2',4'-dichlorophenyl)-1,4,5,6-tetrahydrobenzo[6,7]cyclohepta[1,2-c]pyrazol-3-carboxamide

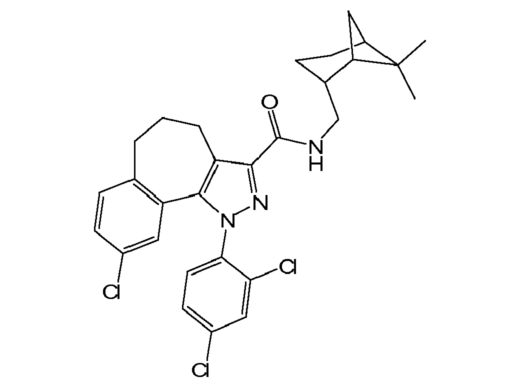

0.70 grams (1.72 mmoles) of the acid obtained in example 2.1, *N*-(3-dimethylamminopropyl)-*N'*-ethylcarbodiimide hydrochloride (EDC) (1.2 eq) and hydrate 1-hydroxybenzotriazol (HOBt) (1.2 eq) were added to 6 ml of CH₂Cl₂. The obtained mixture was maintained under stirring at room temperature for one hour. To the mixture a solution of (-)-*cis*-myrtanylamine (2 eq) in CH₂Cl₂ (6 ml) was dropwise added. The resulting reaction mixture was maintained under stirring at room temperature for 14 hours and then concentrated under vacuum. The residue was purified by flash chromatography (oil ligroin /AcOEt 8.5:1.5 v/v) to obtain the compound N-myrtanyl-9-chloro-1-(2',4'-dichlorophenyl) -1,4,5,6-tetrahydrobenzo-[6,7]-cyclohepta [1,2-*c*]pyrazol-3-carboxamide with a 87% yield. Rf = 0.50 (oil ligroin/AcOEt 6:4 v/v); IR (nujol) (λ= cm⁻¹) 3405, 1664; ¹H-NMR (CDCl₃) δ 1.08 (s, 3H); 1.20 (s, 3H); 1.53-1.61 (m, 5H); 1.84-2.03 (m, 4H); 2.19-2.30 (m, 4H); 2.63 (t, 2H, J = 6.4 Hz); 3.37-3.46 (m, 2H); 6.61 (s, 1H); 6.93 (bt, 1H, J = 5.5 Hz); 6.98-7.10 (m, 1H); 7.17-7.23 (m, 2H); 7.49-7.55 (m, 2H). Anal. calc. for C₂₉H₃₀Cl₃N₃O: C, 64.15; H, 5.57; Cl, 19.59; N, 7.74. Found: C, 64.03; H, 5.56; Cl, 19.57; N, 7.73.

### EXAMPLE 3.2

### Preparation of N-myrtanyl-9-chloro-1-(4'-methylbenzyl)-1,4,5,6-tetrahydrobenzo[6,7]cyclohepta[1,2-c]pyrazol-3-carboxamide

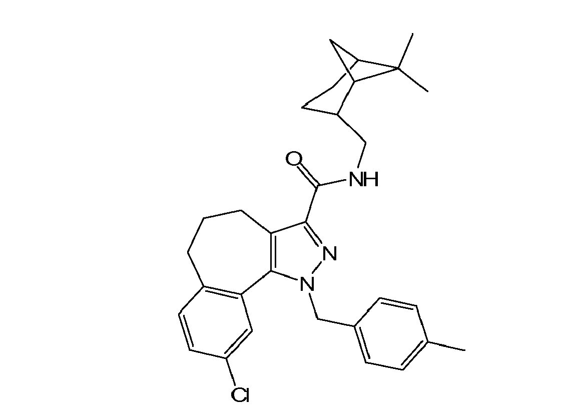

The same procedure described in example 3.1 was repeated, but substituting the acid obtained in example 2.1 with the compound obtained in example 2.2. At the end of the reaction the compound *N*-myrtanyl-9-chloro-1-(4'-methylbenzyl)-1,4,5,6-tetrahydrobenzo[6,7]cyclo-hepta[1,2-*c*] pyrazol-3-carboxamide was obtained with a 60% yield. Rf = 0.48 (oil ligroin/AcOEt 7:3 v/v); IR (nujol) (λ= cm⁻¹) 3408, 1659; ¹H-NMR (CDCl₃) δ 1.15 (s, 3H); 1.19 (s, 3H); 1.90-2.14 (m, 11H); 2.31 (s, 3H); 2.42-2.46 (m, 2H); 2.85-2.89 (m, 2H); 3.39-3.45 (m, 2H); 5.38 (s, 2H); 6.94-7.22 (m, 7H). Anal. calc. for C₂₉H₃₀Cl₃N₃O: C, 64.15; H, 5.57; Cl, 19.59; N, 7.74. Found: C, 64.03; H, 5.56; Cl, 19.57; N, 7.73.

### EXAMPLE 3.3

### Preparation of N-myrtanyl-8-chloro-1-(2',4'-dichlorophenyl)-4,5-dihydrobenzo-1H-6oxa-cyclohepta[1,2-c]pyrazol-3-carboxamide

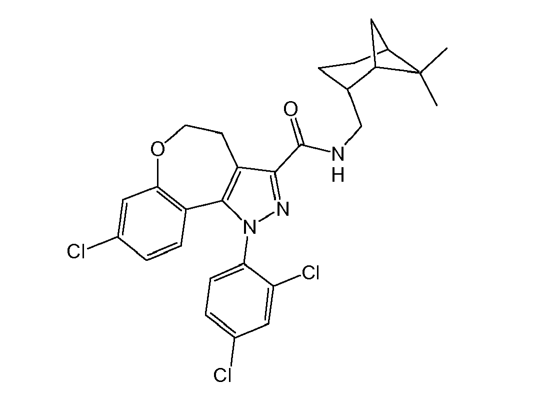

An amount equal to 1 eq of the acid obtained in example 2.5, *N*-(3-dimethylamminopropyl)-*N'*-ethylcarbodiimide hydrochloride (EDC) (1.2 eq) and 1-hydroxybenzotriazole hydrate (HOBt) (1.2 eq) were added to 2 ml of CH₂Cl₂. The obtained mixture was kept under stirring at room temperature for 30 minutes. Then a solution of (-)-*cis*-myrtanylamine (2 eq) in CH₂Cl₂ (2 ml) was dropwise added to the mixture. The resulting reaction mixture was kept under stirring at room temperature for 10 hours and then concentrated under vacuum. The residue from concentration was purified by flash chromatography (oil ligroin /AcOEt 9:1 volume/volume) obtaining the compound *N*-myrtanyl-8-chloro-1-(2',4'-dichlorophenyl)-4,5-dihydrobenzo-1*H*-6oxa-cyclo-hepta[1,2-*c*] pyrazol-3-carboxamide (white solid) with a 90% yield. Rf = 0.31 (oil ligroin/AcOEt 9:1 v/v); m.p. 142-143 142-143 °C; IR (nujol) (λ= cm⁻¹) 3423 (NH), 1676 (CO); ¹H-NMR (CDCl₃) δ 0.82-0.94 (m, 1H), 1.07 (s, 3H), 1.19 (s, 3H), 1.45-1.62 (m, 1H), 1.82-2.04 (m, 4H), 2.21-2.43 (m, 2H), 3.26-3.57 (m, 4H), 4.35-4.42 (m, 2H), 6.60 (d, 1H, J= 8.6 Hz), 6.81 (d, 1H, J= 8.6 Hz), 6.90-6.97 (m, 1H), 7.14 (s, 1H), 7.31-7.39 (m, 2H), 7.54 (br s, 1H, NH, exchang. with D₂O); ¹³C-NMR (CDCl₃) δ 19.84 (CH₂), 23.22 (CH₃), 26.00 (CH₂), 27.08 (CH₂), 27.96 (CH₃), 33.26 (CH₂), 38.70 (C), 41.32 (CH), 41.50 (CH), 43.80 (CH), 44.57 (CH₂), 73.56 (CH₂), 120.03 (C), 122.75 (CH), 123.44 (CH), 128.01 (CH), 128.33 (CH), 130.32 (CH), 130.67 (CH), 132.93 (C), 134.61 (C), 136.05 (C), 137.16 (C), 139.05 (C), 144.51 (C), 159.62 (C), 162.25 (CO); Anal. Calc. for C₂₈H₂₇Cl₃N₃O₂: C, 61.83; H, 5.00; Cl, 19.55; N, 7.72. Found: C, 61.79; H, 4.99; Cl, 19.53; N, 7.71.

### EXAMPLE 3.4

### Preparation of N-myrtanyl-8-chloro-1-(2',4'-dichlorophenyl)-1,4,5,6-tetrahydrobenzo[6,7]cyclohepta[1,2-c]pyrazol-3-carboxamide

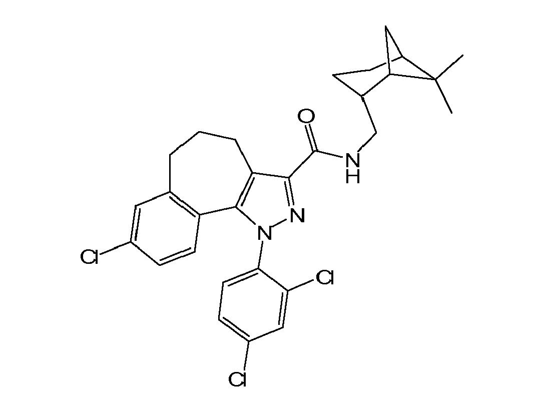

The same procedure described in example 3.1 was repeated, but substituting the acid obtained in example 2.1 with the compound obtained in example 2.3. At the end of the reaction the compound *N*-myrtanyl-8-chloro-1-(2',4'-dichlorophenyl)-1,4,5,6-tetrahydrobenzo[6,7]cyclohepta[1,2-*c*] pyrazol-3-carboxamide was obtained with a 81% yield. Rf = 0.48 (oil ligroin/AcOEt 6:4 v/v); IR (nujol) (λ= cm⁻¹) 3400, 1662; ¹H-NMR (CDCl₃) δ 1.07 (s, 3H); 1.21 (s, 3H); 1.52-1.63 (m, 5H); 1.82-2.05 (m, 4H); 2.15-2.29 (m, 4H); 2.64 (t, 2H, J = 6.6 Hz); 3.35-3.45 (m, 2H); 6.59 (d, 1H, J = 8.0 Hz); 7.00 (dd, 1H, J = 2.2 and 8.0 Hz); 7.25-7.32 (m, 1H); 7.37-7.43 (m, 3H); 7.51 (bt, 1H, J = 5.4 Hz). Anal. calc. for C₂₉H₃₀Cl₃N₃O: C, 64.15; H, 5.57; Cl, 19.59; N, 7.74. Found: C, 64.09; H, 5.55; Cl, 19.56; N, 7.72.

### EXAMPLE 3.5

### Preparation of N-cyclohexylmethyl-8-chloro-1-(2',4'-dichloro

### phenyl)-1,4,5,6-tetrahydrobenzo[6,7]cyclohepta[1,2-c]pyrazole-3-carboxamide

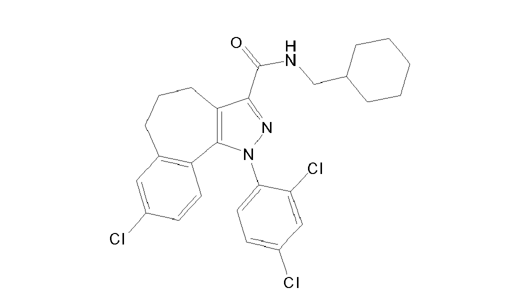

A mixture of the carboxylic acid compound of ex. 2.3 (0.2 g, 0.49 mmol), of *N*-(3-dimethylaminopropyl)-*N'*-ethylcarbodiimidehydro chloride (EDC) (1.2 eq) and 1-hydroxybenzotriazole hydrate (HOBt) (1.2 eq) in CH₂Cl₂ (5 ml) was stirred at room temperature for 1 hour. A solution of cyclohexylmethylamine (2 eq) in CH₂Cl₂ (3 ml) was then dripped and the reaction mixture stirred at room temperature for 14 hours. The solvent was removed under reduced pressure. The residue was purified by flash chromatography (petroleum ether/ethyl acetate 9/1 v/v) obtaining 0.11 g (44% yield) of the compound *N*-cyclohexylmethyl-8-chloro-1-(2',4'-dichlorophenyl)-1,4,5,6-tetrahydrobenzo[6,7]cyclohepta[1,2-*c*] pyrazole-3-carboxamide as a white solid. Rf=0.45 (petroleum ether/ethyl acetate 9:1 volume/volume). IR (nujol) (λ= cm⁻¹) 3410, 1670; ¹H-NMR (CDCl₃) δ 0.92-1.03 (m, 2H); 1.10-1.32 (m, 4H); 1.53-1.84 (m, 5H); 2.20-2.31 (m, 2H); 2.60-2.72 (m, 3H); 2.80-3.15 (m, 1H); 3.20-3.30 (m, 2H); 6.58 (d, 1H, J = 8.0 Hz); 6.97-7.08 (m, 2H); 7.30 (d, 1H, J = 1.6 Hz); 7.40 (dd, 1H, J = 1.7 and 8.4 Hz); 7.42-7.48 (m, 2H). Anal. calc. for C₂₆H₂₆Cl₃N₃O: C, 62.10; H, 5.21; Cl, 21.15; N, 8.36. Found: C, 62.03; H, 5.20; Cl, 21.13; N, 8.34.

### EXAMPLE 3.6

### Preparation of N-(1-adamantylmethyl)-8-chloro-1-(2',4'-dichloro phenyl)-1,4,5,6-tetrahydrobenzo[6,7]cyclohepta[1,2-c]pyrazole-3-carboxamide

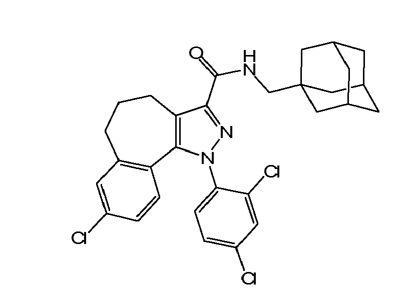

The same procedure of ex. 3.5 was repeated, but the carboxylic acid of ex. 2.3 was reacted with 1-adamantylmethylamine instead of cyclohexylmethylamine. Yield 59%. Rf=0.41 (petroleum ether/ethyl acetate 9/1 v/v). IR (nujol) (λ = cm⁻¹) 3405, 1669; ¹H-NMR (CDCl₃) δ 1.57 (bs, 6H); 1.61-1.75 (m, 6H); 1.95-2.02 (m, 3H); 2.20-2.30 (m, 2H); 2.63-2.73 (m, 3H); 2.82-3.18 (m, 3H); 6.58 (d, 1H, J = 8.4 Hz); 6.97-7.09 (m, 2H); 7.30 (bs, 1H); 7.40 (dd, 1H, J = 1.9 and 8.4 Hz); 7.44-7.50 (m, 2H). Anal. calc. for C₃₀H₃₀Cl₃N₃O: C, 64.93; H, 5.45; Cl, 19.17; N, 7.57. Found: C, 64.81; H, 5.44; Cl, 19.10; N, 7.55.

### EXAMPLE 3.7

### Preparation of N-tetrahydrofurfuryl-8-chloro-1-(2',4'-dichloro phenyl)-1,4,5,6-tetrahydrobenzo[6,7]cyclohepta[1,2-c]pyrazole-3-carboxamide

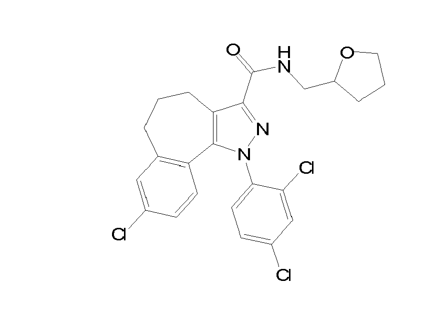

The same procedure of ex. 3.5 was repeated but using tetrahydrofurfurylamine instead of cyclohexylmethylamine. Yield 54%. Rf=0.38 (petroleum ether/ethyl acetate 7/3 v/v). IR (nujol) (λ = cm⁻¹) 3409, 1667; ¹H-NMR (CDCl₃) δ 1.58-1.74 (m, 1H); 1.84-1.96 (m, 2H); 1.97-2.08 (m, 1H); 2.19-2.31 (m, 2H); 2.61-2.72 (m, 2H); 2.75-3.28 (m, 2H); 3.31-3.41 (m, 1H); 3.66-3.81 (m, 2H); 3.84-3.94 (m, 1H); 4.04-4.12 (m, 1H); 6.57 (d, 1H, J = 8.0 Hz); 7.00 (dd, 1H, J = 1.9 and 8.4 Hz); 7.27-7.32 (m, 2H); 7.39 (dd, 1H, J = 1.9 and 8.4 Hz); 7.41-7.48 (m, 2H). Anal. calc. for C₂₄H₂₂Cl₃N₃O₂: C, 58.73; H, 4.52; Cl, 21.67; N, 8.56. Found: C, 58.69; H, 4.51; Cl, 21.65; N, 8.54.

### EXAMPLE 3.8

### Preparation of N-myrtanyl-7-chloro-1-(2',4'-dichlorophenyl)-6-methyl-1,4-dihydroindeno[1,2-c]pyrazole-3-carboxamide

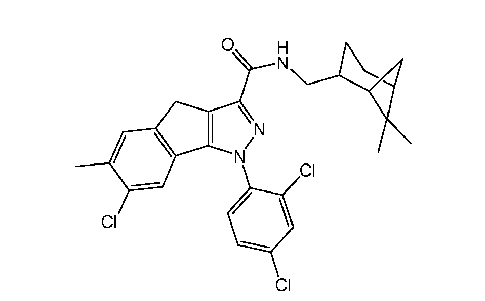

The same procedure of ex. 3.5 was followed but using in the first reaction synthesis the carboxylic acid compound of ex 2.6 instead of that of ex. 2.3, and substituting the dropwise addition of cyclohexylmethylamine with that of (-)-*cis-*myrtanylamine. Yield 78%. Rf=0.26 (petroleum ether/ethyl acetate 9/1 v/v). IR (nujol) (λ = cm⁻¹) 3378, 1683; ¹H-NMR (CDCl₃) δ 0.90-1.75 (m, 11H); 1.75-2.20 (m, 4H); 2.41 (s, 3H); 3.30-3.63 (m, 2H); 3.84 (s, 2H); 3.87-4.00 (m, 1H); 6.94 (s, 1H); 7.40-7.62 (m, 3H); 7.68 (d, 1H, J = 1.8 Hz). Anal. calc. for C₂₈H₂₈Cl₃N₃O: C, 63.58; H, 5.34; Cl, 20.11; N, 7.94. Found: C, 63.98; H, 5.37; Cl, 20.31; N, 7.98.

### EXAMPLE 3.9

### Preparation of N-(1-cyclohexylethyl)-7-chloro-1-(2',4'-dichloro phenyl)-6-methyl-1,4-dihydroindeno[1,2-c]pyrazole-3-carboxamide

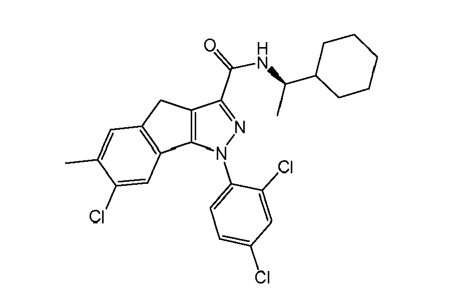

The same procedure of ex. 3.8 was repeated but that the solution dripped in the reaction mixture contained R-(-)-1-cyclohexylethylamine instead of (-)-*cis*-myrtanylamine. Yield 20%. Rf=0.49 (petroleum ether/ethyl acetate 9/1 v/v). IR (nujol) (λ = cm⁻¹) 3305, 1646; ¹H-NMR (CDCl₃) δ 1.00-2.94 (m, 14H); 2.41 (s, 3H); 3.84 (s, 2H); 3.97-4.20 (m, 1H); 6.77 (d, 1H, J = 9.0 Hz); 6.94 (s, 1H); 7.43 (s, 1H); 7.48 (dd, 1H, J = 1.8 and 8.6 Hz); 7.56 (d, 1H, J = 8.6 Hz); 7.68 (d, 1H, J = 2.0 Hz). Anal. calc. for C₂₆H₂₆Cl₃N₃O: C, 62.10; H, 5.21; Cl, 21.15; N, 8.36. Found: C, 62.40; H, 5.23; Cl, 21.18; N, 8.38.

### EXAMPLE 3.10

### Preparation of N-(1-adamantylmethyl)-7-chloro-1-(2',4'-dichlorophenyl)-6-methyl-1,4-dihydroindeno[1,2-c]pyrazole-3-carboxamide

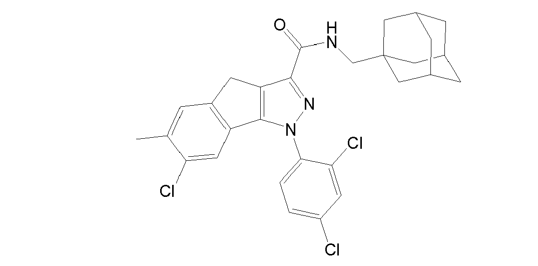

The same procedure of ex. 3.8 was repeated but that the solution dripped in the reaction mixture contained 1-adamantylmethylamine instead of (-)-*cis*-myrtanylamine. Yield 58%. Rf=0.35 (petroleum ether/ethyl acetate 9/1 v/v). IR (nujol) (λ = cm⁻¹) 3252, 1643; ¹H-NMR (CDCl₃) δ 1.32-2.14 (m, 15H); 2.41 (s, 3H); 3.14 (d, 2H, J = 6.7 Hz); 3.84 (s, 2H); 6.95 (s, 1H); 6.99 (bs, 1H); 7.43 (s, 1H); 7.48 (dd, 1H, J = 2.2 and 8.6 Hz); 7.56 (d, 1H, J = 8.6 Hz); 7.68 (d, 1H, J = 1.8 Hz). Anal. calc. for C₂₉H₂₈Cl₃N₃O: C, 64.39; H, 5.22; Cl, 19.66; N, 7.77. Found: C, 64.69; H, 5.25; Cl, 19.69; N, 8.06.

### EXAMPLE 3.11

### Preparation of N-myrtanyl-7-chloro-6-methyl-1-(4'-methylbenzyl)-1,4-dihydroindeno[1,2-c]pyrazole-3-carboxamide

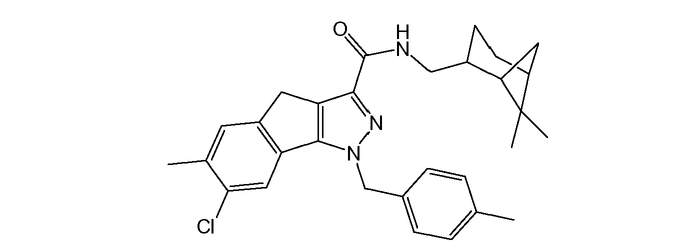

The same procedure of ex. 3.5 was repeated but that the carboxylic acid prepared in ex. 2.7 instead of that of ex. 2.3 was reacted, and the solution dripped in the reaction mixture contained (-)-*cis*-myrtanylamine instead of cyclohexylmethylamine. Yield 50%. Rf=0.45 (petroleum ether/ethyl acetate 8/2 v/v). IR (nujol) (λ = cm⁻¹) 3356, 1679; ¹H-NMR (CDCl₃) δ 1.02 (s, 3H); 1.14 (s, 3H); 1.40-1.70 (m, 2H); 1.75-2.05 (m, 4H); 2.25 (s, 3H); 2.31 (s, 3H); 3.25-3.55 (m, 2H); 3.68 (s, 2H); 5.41 (s, 2H); 6.86 (bs, 1H); 7.00-7.30 (m, 6H). Anal. calc. for C₃₀H₃₄ClN₃O: C, 73.83; H, 7.02; Cl, 7.26; N, 8.61. Found: C, 73.77; H, 7.00; Cl, 7.25; N, 8.60.

### EXAMPLE 3.12

### Preparation of N-myrtanyl-6-chloro-7-methyl-1-(4'-methylbenzyl)-1,4-dihydroindeno[1,2-c]pyrazole-3-carboxamide

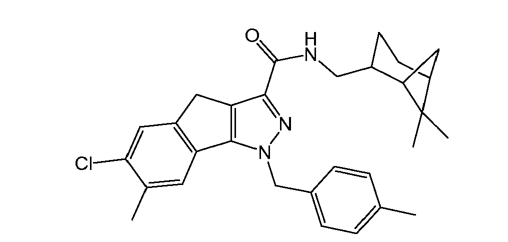

The same procedure of ex. 3.5 was repeated but that the carboxylic acid prepared in ex. 2.8 instead of that of ex. 2.3 was reacted, and the solution dripped in the reaction mixture contained (-)-*cis*-myrtanylamine instead of cyclohexylmethylamine. Yield 68%. Rf=0.30 (petroleum ether/ethyl acetate 9/1 v/v). IR (nujol) (λ = cm⁻¹) 3381, 1674; ¹H-NMR (CDCl₃) δ 1.09 (s, 3H); 1.21 (s, 3H); 1.50-1.80 (m, 2H); 1.90-2.10 (m, 4H); 2.22-2.50 (m, 6H); 3.25-3.60 (m, 2H); 3.76 (s, 2H); 5.51 (s, 2H); 6.92 (bs, 1H); 7.00-7.50 (m, 6H). Anal. calc. for C₃₀H₃₄ClN₃O: C, 73.83; H, 7.02; Cl, 7.26; N, 8.61. Found: C, 73.80; H, 7.01; Cl, 7.24; N, 8.58.

### EXAMPLE 3.13

### Preparation of 8-chloro-1-(2',4'-dichlorophenyl)-3-(1-oxo-2-cyclo hexyleth-1-yl)-1,4,5,6-tetrahydrobenzo[6,7]-cyclohepta-[1,2-c] pyrazole

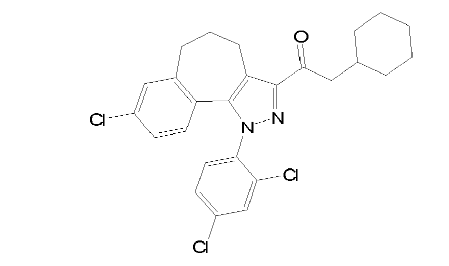

### 3.13a Preparation of N-methoxy-N-methyl-8-chloro-1-(2',4'-dichloro phenyl)-1,4,5,6-tetrahydrobenzo[6,7]cyclohepta[1,2-c]pyrazole-3-carboxamide

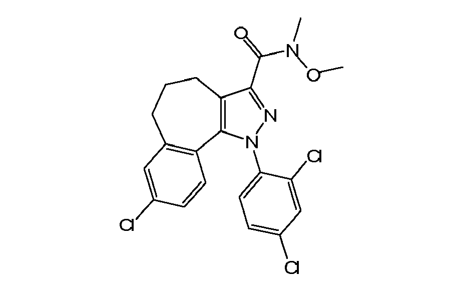

Trimethylaluminum (0.92 ml of a 2 M solution of the compound in hexane, 1.84 mmol) was added dropwise to a suspension of dimethylhydroxylamine hydrochloride (0.18 g, 1.84 mmol) in CH₂Cl₂ (3 ml) at 0 °C. The reaction mixture was stirred at 0 °C for 45 minutes, then at room temperature for 40 minutes. After this period of time a clear solution was obtained. Under stirring to said solution a solution in CH₂Cl₂ (2 ml) of the compound obtained in Ex. 1.3 (0.4 g, 0.92 mmol) was added dropwise. Stirring was continued for further 4 hours at room temperature. The reaction mixture was cooled to 0 °C, and 10% HCl was carefully added dropwise. The mixture was extracted with CH₂Cl₂, washed with water, brine, dried over Na₂SO₄, and filtered. The residue isolated after evaporation of the solvent under reduced pressure was purified by flash chromatography (petroleum ether/ethyl acetate 7/3 volume/volume) obtaining 0.33 g of the compound *N*-methoxy-*N*-methyl-8-chloro-1-(2',4'-dichlorophenyl)-1,4,5,6-tetrahydro-benzo [6,7]cyclohepta[1,2-*c*]pyrazole-3-carboxamide as a white solid (80% yield). Rf=0.38 (petroleum ether/ethyl acetate 7/3). IR (nujol) (λ = cm⁻¹) 1681; ¹H-NMR (CDCl₃) δ 2.21-2.30 (m, 2H); 2.64-2.75 (m, 4H); 3.46 (s, 3H); 3.80 (s, 3H); 6.60 (d, 1H, J = 8.3 Hz); 7.02 (dd, 1H, J = 2.2 and 8.3 Hz); 7.30 (d, 1H, J = 1.6 Hz); 7.36 (dd, 1H, J = 2.2 and 8.3 Hz); 7.40 (d, 1H, J = 8.3 Hz); 7.45 (d, 1H, J = 2.2 Hz). Anal. calc. for C₂₁H₁₈Cl₃N₃O₂: C, 55.96; H, 4.03; Cl, 23.60; N, 9.32. Found: C, 55.82; H, 4.02; Cl, 23.57; N, 9.30.

### 3.13b Preparation of 8-chloro-1-(2',4'-dichlorophenyl)-3-(1-oxo-2-cyclohexyleth-1-yl)-1,4,5,6-tetrahydrobenzo[6,7]-cyclohepta[1,2-c]pyrazole

3.86 ml of a 0.5 M cyclohexylmethylmagnesium bromide solution in THF were added dropwise at 0°C under a nitrogen atmosphere to 6 ml of THF solution containing 0.29 g (0.64 mmol) of the compound obtained in ex. 3.13a. The temperature of the reaction mixture was slowly raised to room temperature and stirred at this temperature for 24 hours. At the end of this period the temperature of the mixture was lowered to 0°C. 15 ml of a saturated NH₄Cl aqueous solution previously conditioned at 0 °C were added dropwise. The temperature of the reaction mixture was slowly raised to room temperature and then diluted with ethylacetate (15 ml). The aqueous and the organic phases were thus separated. The aqueous layer was extracted with ethylacetate (3x10 ml), and the combined organic layers were washed with water, dried (Na₂SO₄), and filtered. After evaporation of the solvent under reduced pressure a residue was recovered, that was purified by flash chromatography (petroleum ether/diethyl ether 9/1 v/v). 80 mg (26% yield) of compound 8-chloro-1-(2',4'-dichlorophenyl)-3-(1-oxo-2-cyclohexyl eth-1-yl)-1,4,5,6-tetrahydrobenzo[6,7]-cyclohepta[1,2-*c*]pyrazole were recovered as a white solid. Rf=0.56 (petroleum ether/diethyl ether 9/1). IR (nujol) (λ = cm⁻¹) 1685; ¹H-NMR (CDCl₃) δ 0.98-1.11 (m, 2H); 1.13-1.38 (m, 4H); 1.62-1.81 (m, 4H); 1.97-2.10 (m, 1H); 2.18-2.29 (m, 2H); 2.62-2.71 (m, 2H); 2.85-3.18 (m, 4H); 6.58 (d, 1H, J = 8.3 Hz); 7.00 (dd, 1H, J = 2.2 and 8.3 Hz); 7.30 (d, 1H, J = 2.2 Hz); 7.40 (dd, 1H, J = 2.2 and 8.3 Hz); 7.44-7.48 (m, 2H). Anal. calc. for C₂₆H₂₅Cl₃N₂O: C, 64.01; H, 5.17; Cl, 21.80; N, 5.74. Found: C, 63.89; H, 5.16; Cl, 21.77; N, 5.72.

### EXAMPLE 3.14

### Preparation of 8-chloro-1-(2',4'-dichlorophenyl)-3-(1-hydroxy-2-cyclohexyleth-1-yl)-1,4,5,6-tetrahydrobenzo[6,7]cyclohepta[1,2-c] pyrazole

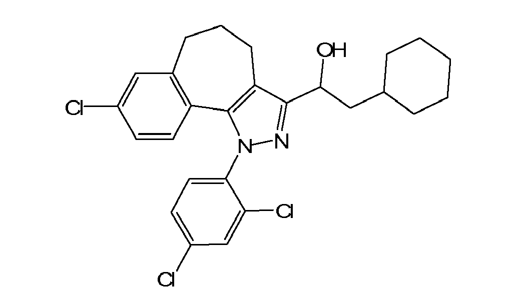

To a suspension in methyl alcohol (3 ml) of the keto compound prepared in Example 3.13 (60 mg, 0.12 mmol) sodium borohydride (10 mg, 0.25 mmol) was added. The mixture was stirred at room temperature for 2 hours, then diluted with CHCl₃ and washed with water. The organic layer was recovered and dried over anhydrous sodium sulfate. The organic phase was then filtered and concentrated under reduced pressure. 60 mg (99% yield) of the compound 8-chloro-1-(2',4'-dichlorophenyl)-3-(1-hydroxy-2-cyclo hexyleth-1-yl)-1,4,5,6-tetrahydrobenzo-[6,7]cyclohepta[1,2-c] pyrazole were recovered as a white solid. Rf=0.34 (petroleum ether/ethyl acetate 8/2 volume/volume). IR (nujol) (λ = cm⁻¹) 3315; ¹H-NMR (CDCl₃) δ 0.84-1.05 (m, 2H); 1.08-1.24 (m, 4H); 1.34-1.78 (m, 5H); 1.80-1.90 (m, 2H); 2.14-2.26 (m, 2H); 2.46-2.72 (m, 4H); 5.00 (bs, 1H); 6.60 (d, 1H, J = 8.3 Hz); 7.01 (dd, 1H, J = 2.2 and 8.3 Hz); 7.29 (d, 1H, J = 2.2 Hz); 7.35 (dd, 1H, J = 2.2 and 8.9 Hz); 7.40-7.45 (m, 2H). Anal. calc. for C₂₆H₂₇Cl₃N₂O: C, 63.75; H, 5.56; Cl, 21.71; N, 5.72. Found: C, 63.68; H, 5.55; Cl, 21.69; N, 5.71.

### EXAMPLE 3.15

### Preparation of N-myrtanyl-1-(2',4'-dichlorophenyl)-6-methyl-1,4-dihydroindeno[1,2-c]pyrazole-3-carboxamide

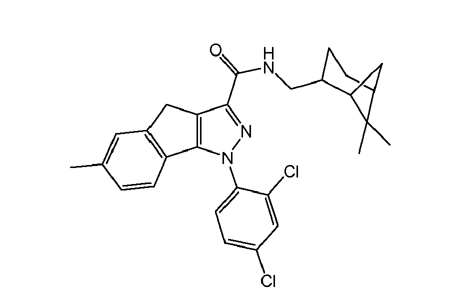

The same procedure of ex. 3.5 was repeated but that the starting mixture contained 1-(2',4'-dichlorophenyl)-6-methyl-1,4-dihydro indeno[1,2-*c*]pyrazole-3-carboxylic acid, prepared as reported in literature (Mussinu J.M., Ruiu S., Mulè A.C., Pau A., Carai M.A.M., Loriga G., Murineddu G., Pinna G.A., Bioorg. Med. Chem. 2003, 11, 251-263) instead of the carboxylic acid prepared in the ex. 2.3 and that the solution dripped in the reaction mixture contained (-)-*cis-*myrtanylamine instead of cyclohexyl methyl amine. Yield 56%. Rf = 0.50 (petroleum ether/ethyl acetate 8.5/1.5 v/v). IR (nujol) (λ = cm⁻¹) 3350, 1685; ¹H-NMR (CDCl₃) δ 0.93 (d, 1H, J = 12.0 Hz); 1.11 (s,3H), 1.23 (s,3H), 1.55-1.68 (m, 3H); 1.84-2.09 (m, 4H); 2.30-2.45 (m, 1H); 2.42 (s, 3H); 3.38-3.46 (m, 1H); 3.50-3.58 (m, 1H); 3.88 (s, 2H); 6.90 (d, 1H, J = 7.9 Hz); 6.97 (bt, 1H, J = 6.4 Hz); 7.06 (bd, 1H, J = 7.7 Hz); 7.40 (bs, 1H); 7.49 (dd, 1H, J = 2.0 and 7.9 Hz); 7.57 (d, 1H, J = 7.8 Hz); 7.68 (d, 1H, J = 2.2 Hz). Anal. calc. for C₂₈H₂₉Cl₂N₃O: C, 68.01; H, 5.91; Cl, 14.34; N, 8.50. Found: C, 67.89; H, 5.90; Cl, 14.31; N, 8.48.

### EXAMPLE 3.16

### Preparation of N-cyclohexylmethyl-1-(2',4'-dichlorophenyl)-6-methyl 1,4-dihydroindeno[1,2-c]pyrazole-3-carboxamide

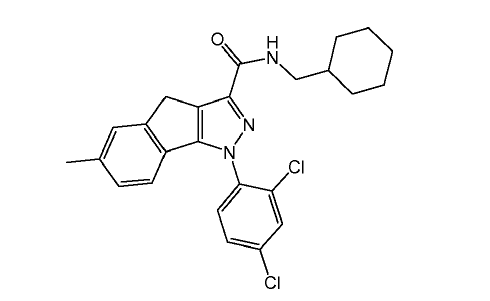

The same procedure of ex. 3.15 was repeated but that the solution dripped in the reaction mixture contained cyclohexylmethylamine instead of (-)-*cis*-myrtanylamine. Yield 84%. Rf=0.26 (petroleum ether/ethyl acetate 9:1 volume/volume). IR (nujol) (λ = cm⁻¹) 3360, 1685; ¹H-NMR (CDCl₃) δ 0.93-1.05 (m, 2H); 1.10-1.30 (m, 3H); 1.53-1.84 (m, 6H); 2.39 (s, 3H); 3.25-3.32 (m, 2H); 3.85 (s, 2H); 6.87 (d, 1H, J = 7.8 Hz); 6.98 (bt, 1H, J = 6.6 Hz); 7.03 (bd, 1H, J = 7.8 Hz); 7.37 (bs, 1H); 7.45 (dd, 1H, J = 2.2 and 8.1 Hz); 7.54 (d, 1H, J = 7.9 Hz); 7.63 (d, 1H, J = 2.2 Hz). Anal. calc. for C₂₅H₂₅Cl₂N₃O: C, 66.08; H, 5.55; Cl, 15.60; N, 9.25. Found: C, 66.01; H, 5.54; Cl, 15.58; N, 9.24.

### EXAMPLE 3.17

### Preparation of N-(1-adamantylmethyl)-1-(2',4'-dichlorophenyl)-6-methyl-1,4-dihydroindeno[1,2-c]pyrazole-3-carboxamide

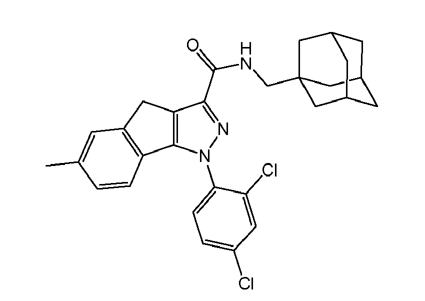

The same procedure of ex. 3.15 was repeated but that the solution dripped in the reaction mixture contained 1-adamantylmethylamine instead of (-)-*cis*-myrtanylamine. Yield 77%. Rf=0.31 (petroleum ether/ethyl acetate 9/1 v/v). IR (nujol) (λ = cm⁻¹) 3374, 1682; ¹H-NMR (CDCl₃) δ 1.55-1.76 (m, 12H); 1.98 (bs, 3H); 2.38 (s, 3H); 3.14 (d, 2H, J = 7.8 Hz); 3.85 (s, 2H); 6.88 (d, 1H, J = 7.8 Hz); 6.97 (bt, 1H, J = 6.4 Hz); 7.02 (bd, 1H, J = 7.7 Hz); 7.37 (bs, 1H); 7.45 (dd, 1H, J = 2.1 and 8.0 Hz); 7.55 (d, 1H, J = 7.8 Hz); 7.65 (d, 1H, J = 2.0 Hz). Anal. calc. for C₂₉H₂₉Cl₂N₃O: C, 68.77; H, 5.77; Cl, 14.00; N, 8.30. Found: C, 68.72; H, 5.76; Cl, 13.98; N, 8.28.

### EXAMPLE 3.18

### Preparation of 1-(2',4'-dichlorophenyl)-6-methyl-3-(1-oxo-2-cyclo hexyleth-1-yl)-1,4-dihydroindeno[1,2-c]pyrazole

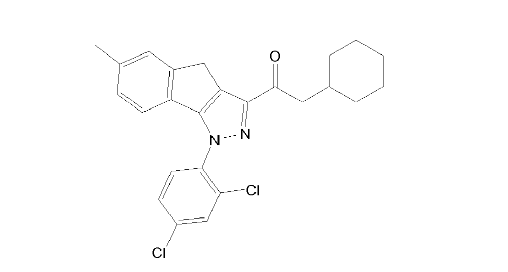

### 3.18a Preparation of N-methoxy-N-methyl-1-(2',4'-dichlorophenyl)-6-methyl-1,4-dihydroindeno[1,2-c]pyrazole-3-carboxamide

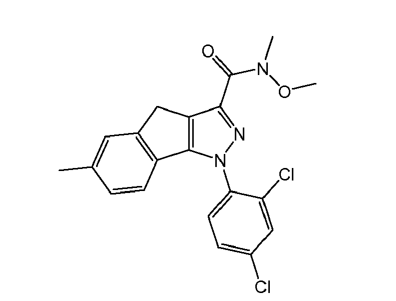

The same procedure of ex. 3.13a was repeated but that instead of the ester compound of ex. 1.3, ethyl 1-(2',4'-dichlorophenyl)-6-methyl-1,4-dihydroindeno[1,2-*c*]pyrazole-3-carboxylate, prepared according to the procedure reported by Mussinu J.M. et al. in *Bioorg. Med. Chem.* 2003, *11*, 251-263, was used. Yield 44%. Rf=0.31 (petroleum ether/ethyl acetate 7.5/2.5 v/v). IR (nujol) (λ = cm⁻¹) 1684; ¹H-NMR (CDCl₃) δ 2.40 (s, 3H); 3.54 (bs, 3H); 3.80 (s, 2H); 3.82 (s, 3H); 6.90 (d, 1H, J = 7.8 Hz); 7.04 (bd, 1H, J = 7.7 Hz); 7.37 (bs, 1H); 7.44 (dd, 1H, J = 2.2 and 8.5 Hz); 7.56 (d, 1H, J = 8.5 Hz); 7. 65 (d, 1H, J = 2.2 Hz). Anal. calc. for C₂₀H₁₇Cl₂N₃O₂: C, 59.71; H, 4.26; Cl, 17.63; N, 10.45. Found: C, 59.67; H, 4.25; Cl, 17.62; N, 10.43.

### 3.18b Preparation of 1-(2',4'-dichlorophenyl)-6-methyl-3-(1-oxo-2-cyclohexyleth-1-yl)-1,4-dihydroindeno[1,2-c]pyrazole

The same procedure of ex. 3.13b was repeated but using the compound of ex. 3.18a instead of the carboxamide compound of ex. 3.13a. Yield 70%. Rf=0.62 (petroleum ether/diethyl ether 9:1 v/v). IR (nujol) (λ = cm⁻¹) 1686; ¹H-NMR (CDCl₃) δ 1.00-1.40 (m, 5H); 1.56-1.85 (m, 5H); 2.02-2.16 (m, 1H); 2.40 (s, 3H); 2.96 (d, 2H, J = 6.9 Hz); 3.82 (s, 2H); 6.89 (d, 1H, J = 7.8 Hz); 7.04 (bd, 1H, J = 7.5 Hz); 7.38 (bs, 1H); 7.47 (dd, 1H, J = 2.2 and 8.4 Hz); 7.57 (d, 1H, J = 8.4 Hz); 7.66 (d, 1H, J = 2.1 Hz). Anal. calc. for C₂₅H₂₄Cl₂N₂O: C, 68.34; H, 5.51; Cl, 16.14; N, 6.38. Found: C, 68.26; H, 5.50; Cl, 16.12; N, 6.37.

### EXAMPLE 3.19

### Preparation of 1-(2',4'-dichlorophenyl)-6-methyl-3-(1-hydroxy-2-cyclohexyleth-1-yl)-1,4-dihydroindeno[1,2-c]pyrazole

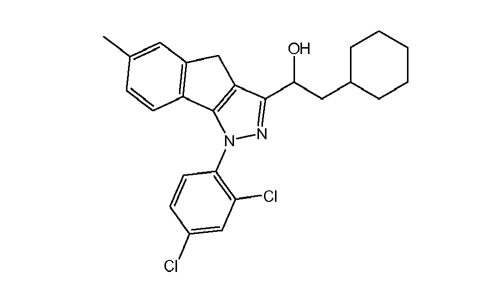

The same procedure of ex. 3.14 was repeated but using the keto compound of ex. 3.18 instead of the keto compound of ex. 3.13. Yield 99%. Rf=0.39 (petroleum ether/ethyl acetate 8/2 v/v). IR (nujol) (λ = cm⁻¹) 3325; ¹H-NMR (CDCl₃) δ 0.90-1.32 (m, 6H); 1.49-1.60 (m, 1H); 1.61-1.92 (m, 7H); 2.39 (s, 3H); 3.65 (d, 2H, J = 3.1 Hz); 4.99-5.05 (m, 1H); 6.88 (d, 1H, J = 7.8 Hz); 7.03 (bd, 1H, J = 7.7 Hz); 7.33 (bs, 1H); 7.40 (dd, 1H, J = 2.3 and 8.4 Hz); 7.50 (d, 1H, J = 8.4 Hz); 7.61 (d, 1H, J = 2.2 Hz). Anal. calc. for C₂₅H₂₆Cl₂N₂O: C, 68.03; H, 5.94; Cl, 16.09; N, 6.35. Found: C, 67.94; H, 5.93; Cl, 16.03; N, 6.34.

### EXAMPLE 3.20

### Preparation of N-piperidinyl-1-(2,4-dichlorophenyl)-6-methyl-1H-benzofuro[3,2-c]pyrazole-3-carboxamide

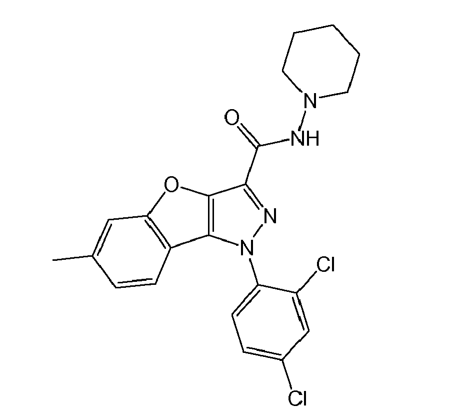

To the solution in methylene chloride (2 ml) of the acid obtained in ex. 2.9 (0.09 g; 0.25 mmol) HOBt (0.04 g; 0.30 mmol) and EDC (0.06 g, 0.30 mmol) were added. The resulting mixture was stirred at room temperature for 1 hour, then a solution of aminopiperidine (0.50 mmol) in 3 ml of CH₂Cl₂, was added. The resulting mixture was stirred at room temperature for 22 hours, then the solvent was removed under reduced pressure. The oily residue was purified by flash chromatography (oil ether/ethyl ether 6/4 v/v on silica gel). 0.09 g (81% yield) of a white solid corresponding to N-piperidinyl-1-(2,4-dichlorophenyl)-6-methyl-1H-benzofuro[3,2-c] pyrazole-3-carboxamide were recovered. Rf=0.10 (oil ether/ethyl ether 6/4 on silica gel); m.p.: 155-157°C; IR (nujol) (λ = cm⁻¹) 3434 (NH), 1648 (C=O); ¹H-NMR (CDCl₃) δ 0.86 (m, 2H), 1.25-1.98 (m, 4H), 2.49 (s, 3H) 2.92 (m, 4H), 7.09 (d, J=7.6 Hz, 1H), 7.29 (m, 2 H), 7.47 (m, 2H), 7.62 (m, 2H); ¹³C-NMR (CDCl₃) δ 21.9, 23.2, 25.2, 57.0, 113.9, 119.0, 124.4, 125.4, 128.2, 128.8, 129.2, 130.3, 130.5, 135.6, 136.0, 136.2, 137.8, 157.5, 162.9; API-ESI calc. for 443.33, found 443.10.

### EXAMPLE 3.21

### Preparation of N-pirrolidinyl-1-(2,4-dichlorophenyl)-6-methyl-1H-benzofuro[3,2-c]pyrazole-3-carboxamide

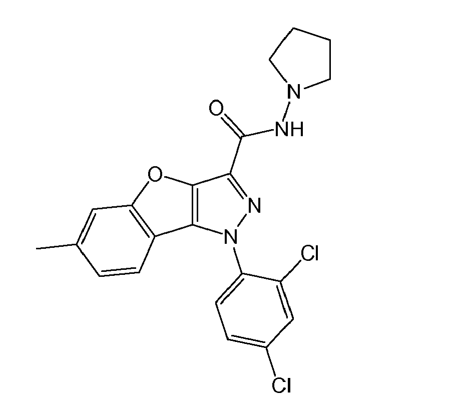

The same procedure described in ex. 3.20 was repeated, but the acid prepared in the ex. 2.9 was reacted with aminopirrolidine instead of aminopiperidine. Yield 94%. m.p. 90-92°C; IR (nujol) (λ = cm⁻¹) 3405, 1677; ¹H-NMR (CDCl₃) δ 1.78-1.94 (m, 5H); 2.50 (s,3H); 3.07 (m, 4H); 7.09 (d, 1H, J = 7.6 Hz); 7.27 (m, 1H); 7.48 (m, 2H); 7.63 (m, 2H). API-ESI calc. 429.30, found: 429.05.

### EXAMPLE 3.22

### Preparation of N-morpholin-4yl-1-(2,4-dichlorophenyl)-6-methyl-1H-benzofuro[3,2-c]pyrazole-3-carboxamide

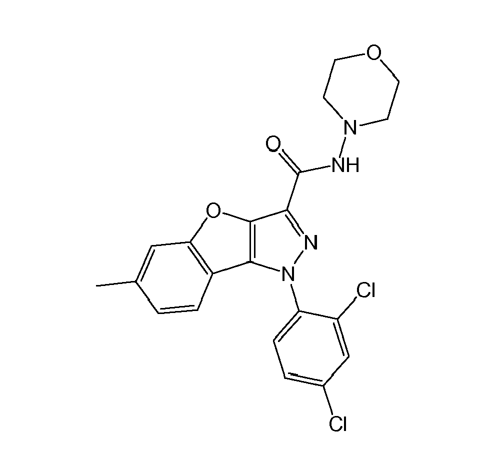

The same procedure described in ex. 3.20 was repeated but the acid prepared in ex. 2.9 was reacted with morpholin-4-ylamine instead of aminopiperidine. Yield 99%. m.p. 178-180°C; IR (nujol) (λ=cm⁻¹) 3430, 1673; ¹H-NMR (CDCl₃) δ 2.50 (s,3H); 3.02 (m, 4H); 3.88 (m, 4H); 7.10 (d, 1H, J = 7.8 Hz); 7.29 (d, 1H, J = 8.2 Hz); 7.46-7.51 (m, 2H); 7.59-7.67 (m, 2H). API-ESI calc. 445.30; found: 445.15.

### EXAMPLE 3.23

### Preparation of N-cyclohexyl-1-(2,4-dichlorophenyl)-6-methyl-1H-benzofuro[3,2-c]pyrazole-3-carboxamide

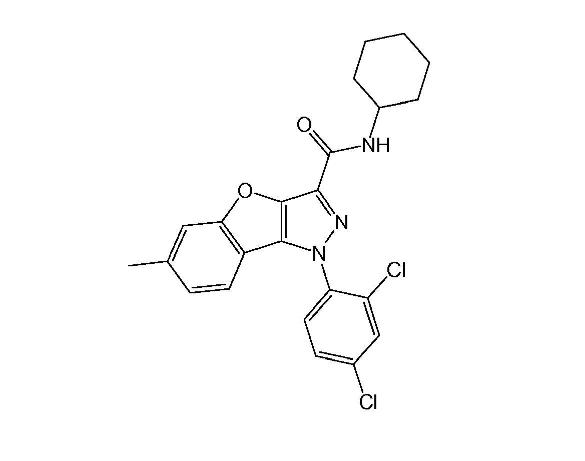

The same procedure described in ex. 3.20 was repeated but the acid prepared in ex. 2.9 was reacted with cyclohexylamine instead of aminopiperidine. Yield 99%. m.p. 140-142°C; IR (nujol) (λ = cm⁻¹) 3409, 1665; ¹H-NMR (CDCl₃) δ 0.79-2.08 (m, 10H); 2.50 (s,3H); 4.05 (m, 1H); 6.73 (d, 1H, J = 8.4 Hz); 7.09 (d, 1H, J = 7.8 Hz); 7.28 (d, 1H, J = 8.1 Hz); 7.47 (m, 2H); 7.58(m, 2H); ¹³C-NMR (CDCl₃) 21.9, 24.8, 25.6, 33.1, 47.9, 113.9, 119.1, 124.4, 128.3, 129.2, 129.9, 130.5, 135.6, 137.7, 159.3, 162.9. API-ESI calc. 442.34; Found: 442.20.

### EXAMPLE 3.24

### Preparation of N-2-isopropyl-5-methyl-cyclohexyl-1-(2,4-dichloro phenyl)-6-methyl-1H-benzofuro[3,2-c]pyrazole-3-carboxamide

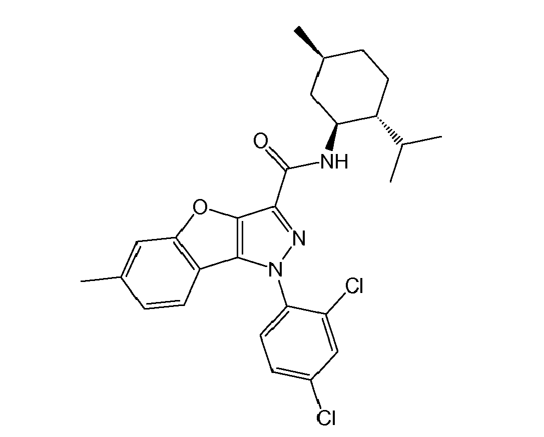

The same procedure described in ex. 3.20 was followed but the acid prepared in ex. 2.9 was reacted with 2-isopropyl-5-methyl-cyclohexylamine instead of aminopiperidine. Yield 96%. m.p. 70-72°C; IR (nujol) (λ = cm⁻¹) 3403, 1666; ¹H-NMR (CDCl₃) δ 0.86-0.99 (m, 9H); 1.14-1.32 (m, 4H); 1.50-1.74 (m, 3H); 2.03-2.14 (m, 2H), 2.50 (s,3H); 4.70 (m, 1H); 6.58 (d, 1H, J = 9.8 Hz); 7.09 (d, 1H, J = 8.0 Hz); 7.30 (d, 1H, J = 8.2 Hz); 7.47 (dd, 2H, J = 2.2, 10.6 Hz); 7.60-7.73 (m, 2H); ¹³C-NMR (CDCl₃) δ 16.2,21.2, 22.0, 22.2, 23.8, 26.9, 31.9, 34.5, 43.1, 48.2, 49.7, 113.9, 119.1, 124.4, 128.3, 129.2, 130.6, 135.6, 137.8, 159.5, 163.0. API-ESI calc. 498.44; found: 498.25.

### EXAMPLE 3.25

### Preparation of N-2,6,6-trimethyl-bicyclo[3.1.1]hept-3yl-1-(2,4-di chlorophenyl)-6-methyl-1H-benzofuro[3,2-c]pyrazole-3-carboxamide

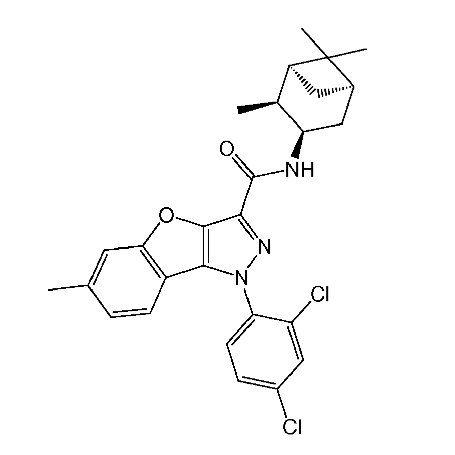

The same procedure described in ex. 3.20 was repeated but the acid prepared in ex. 2.9 was reacted with 2,6,6-trimethyl-bicyclo [3.1.1]hept-3ylamine instead of aminopiperidine. Yield 89%. m.p. 85-87°C; IR (nujol) (λ = cm⁻¹) 3407, 1666; ¹H-NMR (CDCl₃) δ 1.11 (s, 3H), 1.25(s, 6H), 1.69-2.00 (m, 5H), 2.49 (s, 3H), 2.70 (t, J=12 Hz, 2H), 4.53 (q, J=6.6 Hz, 1H), 6.76 (d, J=8.4 Hz, 1H), 7.09 (d, J=8.2 Hz, 1H), 7.29 (d, J=7.8 Hz, 1H), 7.45-7.49 (m, 2H), 7.60-7.66 (m, 2H). API-ESI calc. 496.43; found: 496.15.

### EXAMPLE 3.26

### Preparation of N-1,3,3-trimethyl-bicyclo[2.2.1]hept-2yl-1-(2,4-dichlorophenyl)-6-methyl-1H-benzofuro[3,2-c]pyrazole-3-carboxamide

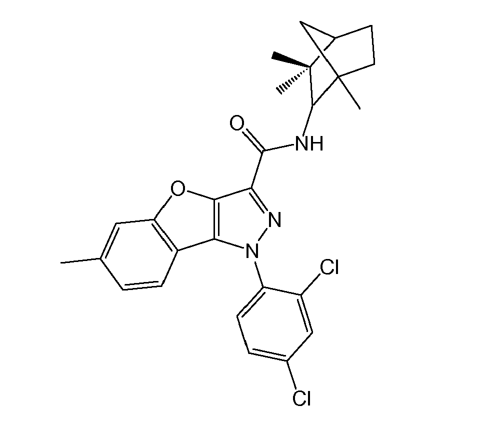

The same procedure described in ex. 3.20 was repeated but the acid prepared in ex. 2.9 was reacted with 1,3,3-trimethyl-bicyclo [2.2.1]hept-2yl-amine instead of aminopiperidine. Yield 96%. m.p. 84-86°C; IR (nujol) (λ = cm⁻¹) 3416, 1671; ¹H-NMR (CDCl₃) δ 0.82-1.80 (m, 16H), 2.50 (s, 3H), 3.88 (d, J= 9.4 Hz, 1H), 6.92 (d, J=10.8 Hz, 1H), 7.09 (d, J=8.0 Hz, 1H), 7.32 (d, J=8.2 Hz, 1H) 7.48 (m,2H), 7.60 (m, 2H); ¹³C-NMR (CDCl₃) δ 19.7, 21.2, 21.9, 26.0, 27.3, 30.9, 39.5, 42.7, 48.1, 48.6, 63.0, 113.9, 119.1, 124.4, 128.2, 129.1, 130.5, 135.4, 136.2, 137.7, 141.5, 146.7, 160.7, 162.9. API-ESI calc. 496.43; found: 496.35.

### EXAMPLE 3.27

### Preparation of N-1,7,7-trimethyl-bicyclo[2.2.1]hept-2yl-1-(2,4-dichlorophenyl)-6-methyl-1H-benzofuro[3,2-c]pyrazole-3-carboxamide

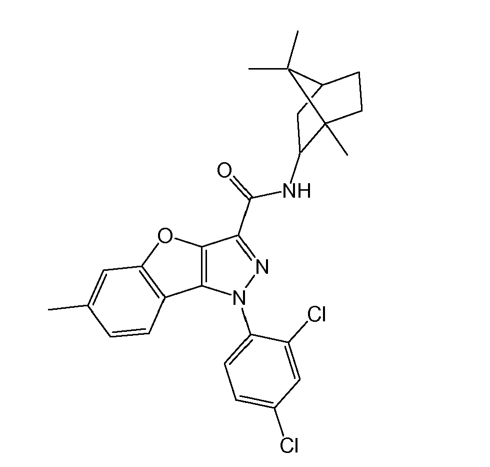

The same procedure described in ex. 3.20 was repeated but the acid prepared in ex. 2.9 was reacted with 1,7,7-trimethyl-bicyclo [2.2.1]hept-2ylamine instead of aminopiperidine. Yield 89%. m.p. 78-80°C; IR (nujol) (λ = cm⁻¹) 3414, 1669; ¹H-NMR (CDCl₃) δ 0.92 (s, 6H), 1.02 (s, 3H), 1.26-1.74 (m, 7H), 2.49 (s, 3H), 4.53 (m, 1H), 6.88 (d, J=9.2 Hz, 1H), 7.09 (d, J=8.00 Hz, 1H), 7.29 (m, 1H), 7.48 (dd, J=2.2, 11.0 Hz, 2H), 7.65 (m, 2H); ¹³C-NMR (CDCl₃) δ 13.8, 18.7, 19.9, 21.9, 28.1, 28.4, 37.5, 44.9, 48.2, 49.9, 53.6, 113.9, 119.1, 124.4, 128.3, 129.3, 130.5, 135.5, 136.1, 136.7, 137.7, 146.3, 160.2, 162.9. API-ESI calc. 496.43; found: 496.15.

### EXAMPLE 3.28

### Preparation of N-adamantan-1yl-1-(2,4-dichlorophenyl)-6-methyl-1H-benzofuro[3,2-c]pyrazole-3-carboxamide

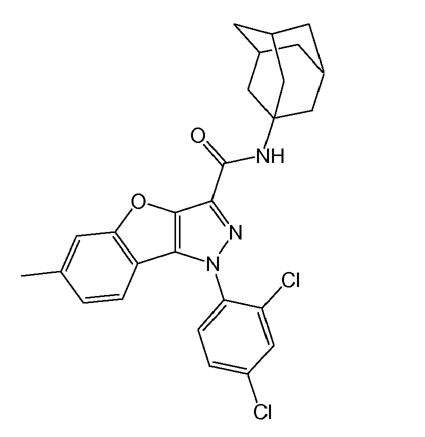

The same procedure described in ex. 3.20 was repeated but reacting the acid prepared in ex. 2.9 with adamantan-1-ylamine instead of aminopiperidine. Yield 81%. m.p. 210-212°C; IR (nujol) (λ = cm⁻¹) 3399, 1670; ¹H-NMR (CDCl₃) δ 1.68 (m, 7H), 2.18 (m, 8H), 2.49 (s, 3H), 6.58 (s, 1H), 7.08 (d, J=8.2 Hz, 1H), 7.29 (dd, J=7.8, 10.4 Hz, 1H), 7.46 (dd, J=2.2, 10.8 Hz, 2H), 7.62 (m, 2H). API-ESI calc. 494.41; found: 494.15.

### EXAMPLE 3.29

### Preparation of N-adamantan-2yl-1-(2,4-dichlorophenyl)-6-methyl-1H-benzofuro[3,2-c]pyrazole-3-carboxamide

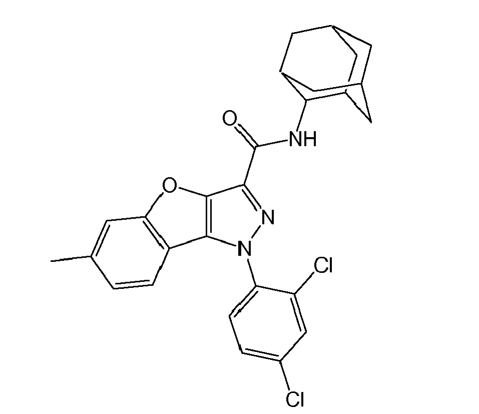

The same procedure described in ex. 3.20 was repeated but the acid prepared in ex. 2.9 was reacted with adamantan-2-ylamine instead of aminopiperidine. Yield 76%. m.p. 209-211°C; IR (nujol) (λ = cm⁻¹) 3417, 1664; ¹H-NMR (CDCl₃) δ 1.66-2.10 (m, 15 H), 2.50 (s, 3H), 4.30 (m, 1H), 7.09 (d, J=8.2 Hz, 1H), 7.20-7.33 (m, 3H), 7.48 (dd, J=2.2, 8.4 Hz, 2H), 7.64 (dd, J=2.2, 12,0 Hz, 1H). API-ESI calc. 494.41; found: 494.15.

### .EXAMPLE 4

### Affinity of the compounds of the invention towards the CB1 and CB2 cannabinoidergic receptors

The affinity of the compounds towards the CB1 and CB2 cannabinoidergic receptors was evaluated in vitro by radioreceptor binding studies using the following method.

The technique of the receptor binding allows to establish if, and with which affinity and specificity, a specific compound binds to a particular receptor. To evaluate the affinity of a specific compound to a particular receptor it is necessary to challenge in a particular tissue preparation wherein those specific receptors are present the compound to be tested with a radioactive labelled compound whose affinity for the same receptors is known. The ability of the compound under test to displace the radioactive compound from the receptor site gives an index of the affinity of the compound under test for that specific receptor. The amount of radioactivity present in the receptor-compound complex allows furthermore to stimate with great accuracy the amount of compound bound to the receptor. By said method it is therefore possible to stablish quickly the affinity of a new compound towards a specific receptor and thus to determine its pharmacological activity. With the same experimental protocol it is possible to evaluate the affinity of the compound towards other receptors and thus establish its specificity degree toward said other receptors.

The receptor binding technique, besides being used for the screening of new molecules with pharmacological activity, can give useful information on possible changes at receptor level, related for example to a prolonged exposure to drugs and/or to particular pathologies. In these conditions, indeed, changes in the amount of the receptors, or conformational changes can take place that alter the binding affinity of the agonists or antagonists, therefore affecting the functionality of the receptors themselves.

The experimentation has been carried out according to the guide lines of the European Community for the animal experimentation (EEC n. 86/609), by using laboratory animals (mice) lodged twenty per cage, under standard stabulation conditions (temperature 22±2°C, relative humidity 60%, artificial lighting with light/dark cycle of 12 hours). The food and water were ad libitum.

The procedure adopted, based on the use of the compound [³H]-CP-55,940 (New England Nuclear, Boston, MA, USA), requires the use of the mouse brain as biological tissue for the evaluation of the affinity towards the CB1 receptors and of the mouse spleen for the affinity assay for the CB2 receptors.

The animals were sacrificed by cervical dislocation and the complete brain (excluding the cerebellum) and the spleen were quickly dissected and kept in ice.

The tissue was homogeneized in 15 volumes (weight/- volume) of TME buffer (50 Mm Tris, 1 mM EDTA and 3 mM MgCl₂, pH 7.4) by an Ultra-Turrax and subsequently centrifuged for 10 minutes at 1086 x g in a centrifuge refrigerated at 4°C. The recovered supernatant was centrifuged at 45,000 x g for 30 minutes at 4°C by using a Beckman SW41 rotor and the obtained pellet was resuspended in 50 volumes of TME.

The thus obtained membranes (50-80 µg of proteins) were incubated in the presence of 1 nM of [³H]-CP55.940 for 1 hour at 30°C in a final volume of 0.5 ml of TME buffer containing 5 mg/ml of bovine serum albumin (BSA). The non specific binding was measured in the presence of CP55.940 at a 1 µM concentration.

All the experiments were carried out in polypropylene test tubes pretreated with Sigma-Cote (Sigma Chemical Co. Ltd., Poole, UK) for reducing non specific binding.

In order to determine the competitive inhibition binding curves, eight different concentrations of each compound were used. As reference compounds SR141716A was used for the CB1 receptors and SR144528 was used for the CB2 receptors.

Incubation was stopped by addition of TME buffer (at 4°C) containing 5 mg/ml of BSA, and subsequent filtration under vacuum by Whatman GFC filters pretreated with 0.5% of polyethylamine (PEI) and by using a filtering device (Brandell, Gaithersburg, MD, USA). The filters were washed 3 times with 5 ml of Tris HCl buffer (pH 7.4, 4°C) containing 1 mg/ml of BSA and separately placed in plastic vials containing 4 ml of scintillating liquid (Ultima Gold MV, Packard).

The radioactivity present in the filters was determined by a scintillator spectrophotometer (Tricarb^{®} 2100, Packard, Meridien, USA).

Protein determination was carried out by the Bradford method by using the protocol and the reactants supplied by Bio-Rad (Milano, Italy).

The experiments were carried out in triplicate and the results confirmed in five independent experiments.

The affinity of the compounds towards the CB1 and CB2 receptors has been expressed in Ki terms.

The Ki values, obtained with the compounds of the present invention in the test in vitro, are reported in Table 1. By comparison, in the Table, the affinity values of the reference compounds SR144528 and SR141716A (Rimonobant^{®}) are reported.

The Table shows that the compounds of the present invention have activity on the CB1 and/or CB2 receptors comparable with that of the reference compounds.

**TABLE 1**

| Example 4 : in vitro activity of the compounds of the invention on CB1 and CB2 receptors | | |
|---|---|---|
| Compound example | CB1 (brain) Ki (nM) | CB2 (spleen) Ki (nM) |
| 3.1 | 20.0±5.0 | 23.3±1.6 |
| 3.3 | 21.7±3.0 | 325±38 |
| 3.4 | 2.6±0.17 | 19.6±1.5 |
| 3.8 | 2500±740 | 62.0±9.3 |
| 3.15 | 350±76 | 2.3±0.5 |
| 3.20 | 1788±330 | 12.9±1.1 |
| 3.21 | 2722±652 | 29.5±8.0 |
| 3.22 | 4064±64 | 37.7±6.3 |
| 3.23 | 1247±129 | 15.6±4.0 |
| 3.24 | 38.2±6.0 | 2.3±0.3 |
| 3.25 | 2398±629 | 3.7±0.3 |
| 3.26 | 257±7 | 2.5±0.3 |
| 3.27 | 469±64 | 3.5±0.6 |
| 3.28 | 363±92 | 28.0±6.6 |
| 3.29 | 537±132 | 17.5±0.9 |
| SR144528 (comp) | 70±10 | 0.28±0.04 |
| SR141716A (comp) | 1.8±0.075 | 514±30 |

### EXAMPLE 4.1 (COMPARISON)

### 4.1.a Preparation of 1-(2,4-dichlorophenyl)-6-methyl-1H-benzofuro [3,2-c]pyrazole-3-carboxamide

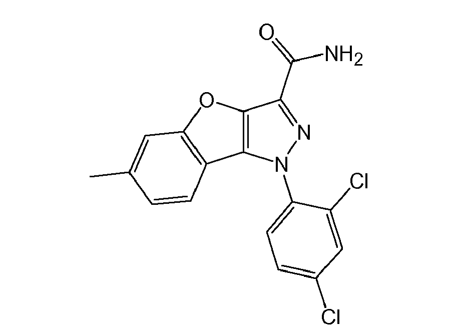

To a solution of the acid obtained in ex. 2.9 (0.09 g; 0.25 mmol) in methylene chloride (2 ml) HOBt (0.04 g; 0.30 mmol) and EDC (0.06g, 0.30 mmol) were added. The mixture was kept under stirring at room temperature for 1 hour, then 10 eq of NH₄OH (30% w in water) was added dropwise. The resulting mixture was stirred at room temperature for 30 minutes. The organic solution was washed with brine, dried over Na₂SO₄, then concentrated under reduced pressure. A crude product was obtained, that was purified by flash chromatography (oil ether/ethyl ether 10/3 v/v on silica gel) to give 1-(2,4-dichlorophenyl)-6-methyl-1*H*-benzofuro[3,2-c]pyrazole-3 - carboxamide. Yield 90%. M.p.: 185-186°C; IR (nujol) (λ = cm⁻¹) 3548 (NH₂), 3287 (NH₂), 1648 (C=O); ¹H-NMR (CDCl₃) δ 2.34 (s, 3H) 6.93 (d, 1H), 7.17 (s, 1H), 7.37 (m, 1 H), 7.50 (m, 2H), 7.69 (m, 2H), 7.77 (m, 1H), 8.31 (br s, NH₂); ¹³C-NMR (CDCl₃) δ 21.6, 106.9, 111.6, 116.7, 120.8, 122.7, 123.6, 127.5, 131.0, 132.9, 133.2, 134.9, 141.3, 141.7, 145.0, 156.4, 161.6; API-ESI calc. for 360.19; found 360.05.

### 4.1.b Determination of the affinity of 1-(2,4-dichlorophenyl)-6-methyl-1H-benzofuro[3,2-c]pyrazole-3-carboxamide for CB1 and CB2 receptors

The affinity of 1-(2,4-dichlorophenyl)-6-methyl-1*H-*benzofuro[3,2-c]pyrazole-3-carboxamide for CB1 and CB2 receptors was determined according to the method reported in ex. 4.

It was found that said compound did not show any significant affinity towards both CB1 and CB2 receptors, as the Ki values for this compound were higher than 3.000 nM for both CB1 and CB2 receptors.

The example shows that the presence of specific substituents at the 3 position of the pyrazole ring in the condensed tricyclic structure, is critical for providing affinity, as in the case of the compounds of formula (I), for CB1 and/or CB2 receptors.

### EXAMPLE 5

### Preparation of a microemulsion containing the compounds of the invention

17.7 mg of the compound obtained in example 3.1 were solubilized in a mixture formed of ethanol (44.2 mg), Miglyol^{®}810N (88.4 mg) and Imvitor^{®}308 (88.4 mg). To the obtained solution 663.1 mg of the nonionic surfactant Solutol^{®}HS15 and 98.2 mg of physiological solution were added under stirring.

A composition in the form of a microemulsion was obtained which at temperatures comprised between 25°C and 37°C was liquid and isotropic.

The microemulsion composition (% by weight) is the following:

| | |
|---|---|
| Ethanol | 4.42 |
| Miglyol^{®}810N | 8.84 |
| Imvitor^{®}308 | 8.84 |
| Solutol^{®}HS15 | 66.31 |
| Compound ex. 3.1 | 1.77 |
| Physiological solution | 9.82 |

### EXAMPLE 6

### Preparation of a microemulsion containing the compounds of the invention

The microemulsion of example 5 was diluted with physiological solution up to a final composition containing the following components (% by weight):

| | |
|---|---|
| Ethanol | 2.23 |
| Miglyol^{®}810N | 4.45 |
| Imvitor^{®}308 | 4.45 |
| Solutol^{®}HS15 | 33.44 |
| Compound ex. 3.1 | 0.89 |
| Physiological solution | 54,54 |

The final sample was a microemulsion. At temperatures comprised between 25°C and 37°C was liquid and isotropic.

### EXAMPLE 7

### Preparation of a microemulsion containing the compounds of the invention

99.1 mg of the compound obtained in example 3.3 were solubilized in ethanol (54.1 mg) Miglyol^{®}810N (108.1 mg). 648.6 mg of the nonionic surfactant Solutol^{®}HS15 and 90.1 mg of physiological solution were added under stirring to the formerly prepared solution.

A composition in the form of a microemulsion was obtained which at temperatures in the range 25°C-37°C is liquid and isotropic, corresponding to a microemulsion.

The microemulsion composition (% by weight) is the following:

| | |
|---|---|
| Ethanol | 5.41 |
| Miglyol^{®}810N | 10.81 |
| Solutol^{®}HS15 | 64.86 |
| Compound ex. 3.3 | 9.91 |
| Physiological solution | 9.01 |

### EXAMPLE 8

### Preparation of a microemulsion containing the compounds of the invention

The microemulsion of example 7 was diluted with physiological solution up to a final composition containing the following components (% by weight):

| | |
|---|---|
| Ethanol | 0.65 |
| Miglyol^{®}810N | 1.29 |
| Solutol^{®}HS15 | 7.77 |
| Compoundo ex. 3.3 | 1.19 |
| Physiological solution | 89.10 |

The final sample was a microemulsion. At temperatures comprised between 25°C and 37°C was liquid and isotropic.

### EXAMPLE 9

### Preparation of a microemulsion containing a compound of formula A': N-piperidinyl-[8-chloro-1-(2',4'-dichlorophenyl)-1,4,5,6-tetrahydrobenzo[6,7]cyclohepta[1,2-c]pyrazol-3-carboxamide]

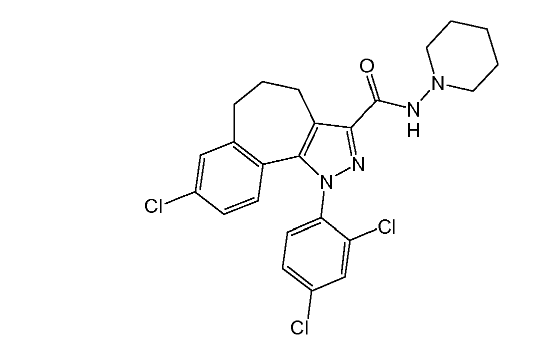

The compound has CB1 antagonist activity and was prepared as described by S. Ruiu et al. in J.P.E.T., Vol. 306, 2003, 363-370.

10 mg of the compound were solubilized in 6 mg of ethanol and 10 mg of Miglyol^{®}812N. 60 mg of the nonionic surfactant Solutol^{®}HS15 and 0.914 grams of physiological solution were added under stirring to the obtained soution.

A composition was obtained which at temperatures in the range 25°C-37°C was liquid and isotropic, corresponding to a microemulsion.

The microemulsion composition (% by weight) is the following:

| | |
|---|---|
| Ethanol | 0.60 |
| Miglyol^{®}812N | 1.00 |
| Solutol^{®}HS15 | 6.00 |
| CB1 antagonist compound | 1.00 |
| Physiological solution | 91.40 |

### EXAMPLE 10

### Preparation of a microemulsion of the invention containing a compound of formula A': N-piperidinyl-[6-methyl-1-(2',4'-dichlorophenyl)-1,4-dihydroindeno[1,2-c]pyrazol-3-carboxamide]

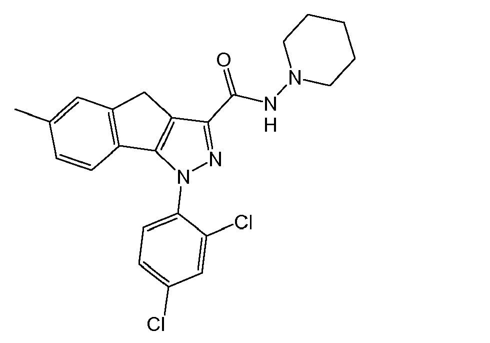

The compound has CB2 agonist activity and has been prepared as described by J.M. Mussinu et al al. in Bioorg. Med. Chem., 11 (2003) 251-263.

5 mg of the compound were solubilized in 5 mg of ethanol and 10 mg of Miglyol^{®}810N. 60 mg of the nonionic surfactant Solutol^{®}HS15 and 0.920 grams of a physiological solution were added under stirring to the obtained soution.

A composition was obtained which at temperatures in the range 25°C-37°C was liquid and isotropic, corresponding to a microemulsion.

The microemulsion composition (% by weight) is the following:

| | |
|---|---|
| Ethanol | 0.50 |
| Miglyol^{®}810N | 1.00 |
| Solutol^{®}HS15 | 6.00 |
| CB2 agonist compound | 0.50 |
| Physiological solution | 92.00 |

### EXAMPLE 11

### Preparation of a microemulsion of the invention containing a compound of formula A': N-piperidinyl-[7-bromo-1-(2',4'-dichlorophenyl)-4,5-dihydro-1H-benzo[g]indazo1-3-carboxamide]

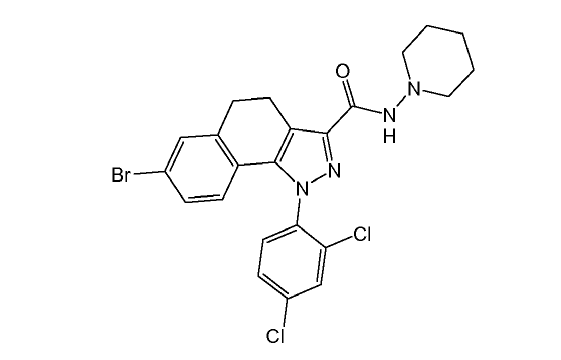

The compound has affinity for the CB1 receptors and was obtained as described by G.Murineddu et al. in Bioorg. Med. Chem., 13 (2005) 3309-3320.

5 mg of the obtained compound were solubilized in 5 mg of ethanol and 10 mg of Miglyol^{®}812N. 60 mg of the nonionic surfactant Solutol^{®}HS15 and 0.920 grams of a physiological solution were added under stirring to the obtained soution. A composition was obtained which at temperatures in the range 25°C-37°C results liquid and isotropic, corresponding to a microemulsion.

The microemulsion composition (% by weight) is the following:

| | |
|---|---|
| Ethanol | 0.50 |
| Miglyol^{®}812N | 1.00 |
| Solutol^{®}HS15 | 6.00 |
| Compound with affinity for the CB1 receptors | 0.50 |
| Physiological solution | 92.00 |

### EXAMPLE 12 Comparative

The same preparation described in example 7 was repeated but without adding the Miglyol^{®}810N oil. The ratios by weight among the other components are therefore equal to those of example 7. The ratio surfactant Solutol^{®} HS15)/active principle (compound ex. 3.3) is of 6.54. An heterogeneous composition is formed, appearing as an opalescent liquid phase containing a bottom sediment.

The composition aspect does not either change by diluting with physiological solution for obtaining the same concentration of the compound of example 3.1 of the microemulsion of example 7 (9.91%).

### EXAMPLE 13 Comparative

The same preparation described in example 9 was repeated but without adding the Miglyol^{®}812N oil. The ratios by weight among the other components are therefore equal to those of example 9. The ratio between the surfactant (Solutol^{®} HS15)/CB1 antagonist compound is of 6.00. An heterogeneous composition is obtained, appearing as an opalescent liquid phase containing a sediment bottom.

The composition aspect does not change by diluting with physiological solution in order to obtain the concentration of the CB1 antagonist compound as in the microemulsion of example 9 (1,00%).

Furthermore the composition aspect does not either change also by adding further amounts of surfactant up to a final ratio surfactant/(CB1 antagonist compound) equal to 12.

## Claims

1. Microemulsions of pharmaceutical compositions comprising the following components (% by weight):
S) from 0.01 to 95% of one or more pharmaceutically acceptable compounds, selected from the following classes:
- surfactants, selected from non-ionic, anionic, cationic and amphoteric surfactants, optionally containing fluorine atoms,
- polymers forming organized structures, such as aggregates, micelles, liquid crystals, vesicles, in the liquid in which they are solubilized,
O) from 0.01 to 95% of one or more oils selected from the following classes of pharmaceutically acceptable compounds:
- C₄-C₃₂ acid esters, linear or branched, optionally containing one or more unsaturations of ethylenic type,
- C₄-C₃₂ acid, linear or branched, optionally containing one or more unsaturations of ethylenic type, that are included when the composition has a pH such that the acid is not converted into the corresponding salt,
PA) from 0.001 to 90% of condensed tricyclic compounds with affinity for the cannabinoidergic CB1 and/or CB2 receptors of formula A': 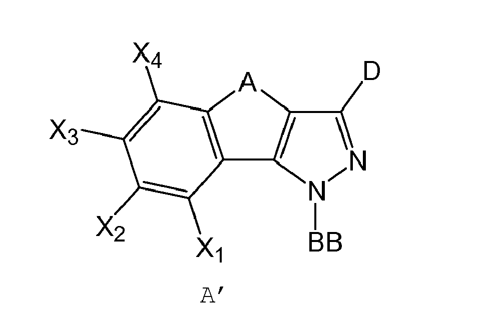 wherein:
A is a group selected from:
A1: -(CH₂)ₜ-,
A2: -(CH₂)ᵣ-O-(CH₂)ₛ-
A3: -(CH₂)ᵣ-S(O)ₚ-(CH₂)ₛ-
wherein
t is an integer equal to 1, 2 or 3,
p is an integer equal to 0, 1 or 2,
r and s, equal to or different from each other,
are integers equal to 0, 1 or 2 with the pro-
viso that r+s is equal to 0, 1, 2 or 3,
BB is selected from phenyl, arylalkyl, arylalkenyl, heteroaryl, heteroaryl-alkyl, or a bivalent C₁-C₁₀ aliphatic chain, linear or branched when possible, wherein the chain end not linked to the nitrogen atom is linked to W, W being selected from hydrogen, halogen, isothio-cyanate, CN, OH, OCH₃, NH₂, SO₂NH₂ or -CH=CH₂,
X₁, X₂, X₃ and X₄, equal to or different from each other, are selected from hydrogen, halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
D is a group selected from the following:
D1: -(CH₂)-O-(CH₂)_{z}-(Z')ᵥ-R"
wherein z is an integer equal to 1 or 2, v is an integer equal to 0 or 1, Z' is a bivalent C₁-C₈ aliphatic chain, R" is selected from C₃-C₁₅ cycloalkyl, saturated or unsaturated heterocycle, aryl, or heteroaryl,
D2: -C(O)-(2')ᵥ-R"
wherein v, Z' and R" are as defined above,
D3: -CH(OH)-(Z')ᵥ-R"
wherein v, Z' and R" are as defined above,
D4: -C(O)-NH-(Z')ᵥ-T'
wherein v and Z' are as defined above and T' is a group selected from:
- C₁-C₈ alkyl, C₁-C₇ haloalkyl with the proviso that in both cases in D4 v = 0,
- C₃-C₁₅ cycloalkyl,
- monocyclic aryl or monocyclic heteroaryl,
- NR₁R₂, wherein R₁ and R₂, equal to or different from each other, are: hydrogen, C₁-C₇ alkyl, C₁-C₇ haloalkyl, heteroaryl, heteroarylalkyl, aryl, arylalkyl or arylalkenyl,
or R₁ and R₂ with the nitrogen atom form a saturated or unsaturated heterocycle from 5 to 10 atoms,
- C₃-C₁₅ heterocycloalkyl, containing one or more heteroatoms, equal to or different from each other selected from N, O, S, with the proviso that Z' is linked to one carbon atom of the heterocycloalkyl ring,
AD) from 0 to 60% by weight of one or more compounds selected from the following classes:
modifiers of the water and/or oil polarity,
modifiers of the curvature of the film of component S),
co-surfactants,
WA) from 0.01 to 99.9% of water or of a saline aqueous solution
the sum of the components of the microemulsion being 100%,
wherein the ratio by weight S)/PA) is lower than at least 10% with respect to the ratio by weight S)/PA) of a microemulsion prepared starting from the same component amounts but without component O).

2. Microemulsions according to claim 1, wherein in component S) the surfactants containing fluorine atoms have (per)fluorinated chains.

3. Microemulsions according to claims 1-2, wherein in component PA), BB is phenyl, arylalkyl, arylalkenyl, heteroaryl, heteroarylalkyl, and BB is substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

4. Microemulsions according to claims 1-3, wherein X₁, X₂, X₃ or X₄ have the meaning of phenyl, cycloalkyl, saturated or unsaturated heterocycle, heteroaryl, and said phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

5. Microemulsions according to claims 1-4, wherein R" is substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio, or C₁-C₇ alkoxy.

6. Microemulsions according to claims 1-5, wherein T' is C₃-C₁₅ cycloalkyl, monocyclic aryl, monocyclic heteroaryl, C₃-C₁₅ heterocycloalkyl, and is optionally substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, said phenyl and benzyl substituents are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

7. Microemulsions according to claims 1-6, wherein R₁ and R₂ are heteroaryl, heteroarylalkyl, aryl, arylalkyl or arylalkenyl, or R₁ and R₂ with the nitrogen atom form an heterocycle, said aromatic rings or the heterocycle being substituted with one or more groups equal to or different from each other selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, said phenyl and benzyl substituents are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

8. Microemulsions according to claims 1-7, wherein in the compounds PA):
A represents a group selected between A1 and A2,
BB is a substituent selected from phenyl, benzyl, monocyclic heteroaryl, monocyclic heterocycloalkyl, bivalent C₄-C₁₀ aliphatic chain, linear or branched when possible,
wherein the chain end not linked to the nitrogen atom is linked to W, W being selected from hydrogen, halogen, isothiocyanate, CN, OH, OCH₃, NH₂, SO₂NH₂ or -CH=CH₂,
said phenyl, benzyl, monocyclic heteroaryl and monocyclic heterocycloalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl,
amino optionally mono- or bisubstituted with a C₁-C₇ alkyl chain,
X₁, X₂, X₃ and X₄ are as defined above,
D is selected from D2, D3 or D4.

9. Microemulsions according to claims 1-8, wherein in compounds PA):
A is selected between A1 and A2,
BB is selected from phenyl, benzyl, thiophene, bivalent C₄-C₁₀ aliphatic chain, linear or branched, wherein the end of the main chain not linked to the nitrogen atom is linked to W, W selected from hydrogen, halogen, OH, OCH₃, NH₂, SO₂NH₂, said phenyl, benzyl and thiophene being optionally substituted with one or more groups,
equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or
bisubstituted with a C₁-C₇ alkyl chain,
X₁, X₂, X₃ and X₄, equal to or different from each other,
are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, cycloalkyl, saturated or unsaturated heterocycle, heteroaryl, amino optionally mono- or bisubstituted with a C₁-C₃ alkyl chain,
D is a group selected from the following:
D2 with the proviso that Z' is selected from -CH₂- or -CH(CH₃)-, R" is as above defined wherein C₃-C₁₅ cycloalkyl, saturated or unsaturated heterocycle, aryl, or heteroaryl are optionally substituted with one or more groups, equal to or different from each other, selected frm SO₂NH₂, halogen, C₁-C₃ alkyl, C₁-C₃ haloalkyl, C₁-C₃ haloalkoxy, C₁-C₃ alkylthio or C₁-C₃ alkoxy,
D4 with the proviso that Z' is selected from -CH₂- or -CH(CH₃)-, and T' is a group selected from the following:
- C₃-C₁₅ cycloalkyl,
- monocyclic aryl or monocyclic heteroaryl,
- NR₁R₂, wherein R₁ and R₂, equal to or different from each other, with the nitrogen atom form a saturated or unsaturated heterocycle from 5 to 10 atoms,
- C₃-C₁₅ heterocycloalkyl, wherein Z' is linked to one carbon atom of the heterocycloalkyl ring,
T' is substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, said phenyl and benzyl substituents being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain.

10. Microemulsions according to claims 1-9 having the following composition (% by weight):
- Component S) from 0.01 to 90%,
- Component O) from 0.01 to 90%,
- Component PA) from 0.001 to 50%,
- Component AD) from 0 to 30%,
- Component WA) from 0.1 to 99.9%,
the sum of the components being 100%.

11. Microemulsions according to claims 1-10 having the following composition (% by weight):
- Component S) from 0.01 to 80%,
- Component O) from 0.01 to 70%,
- Component PA) from 0.05 to 40%,
- Component AD) from 0 to 20%,
- Component WA) from 10 to 99.9%,
the sum of the components being 100%.

12. Microemulsions according to claims 1-11 having the following composition (% by weight):
- Component S) from 0.01 to 70%,
- Component O) from 0.01 to 50%,
- Component PA) from 0.05 to 30%,
- Component AD) from 0 to 15%,
- Component WA) from 20 to 99.9%,
the sum of the components being 100%.

13. Microemulsions according to claims 1-12, wherein component S) is selected from non-ionic and anionic surfactants.

14. Microemulsions according to claims 1-13, wherein component S) is selected from the polymers soluble in component W and/or in component O).

15. Microemulsions according to claims 1-14, wherein component O) is selected from the esters of acids with an alcohol having a C₁-C₅ aliphatic chain or a polyoxyethylene chain, or with glycerine.

16. Microemulsions according to claims 1-15, wherein the compounds A' are in the form of geometrical isomers and/or stereoisomers, when one or more chiral centres are present.

17. A process for preparing the microemulsions of claims 1-15 comprising the following steps:
(IP) solubilization of the compound component PA) in component O),
(IIP) addition of component S) to the solution in oil obtained in (IP),
(IIIP) optional addition of component AD) to the oily phase obtained in (IIP),
(IVP) addition of water or of a saline solution to the oily phase obtained in (IIP) or optionally in (IIIP), obtaining a limpid phase.

18. Condensed tricyclic compounds having a condensed ring structure, containing a phenyl and a pyrazole ring joined by a central ring comprising from five to eigth atoms, showing affinity for the CB1 and/or CB2 receptors, with central and/or the peripheral activity, having formula (I): 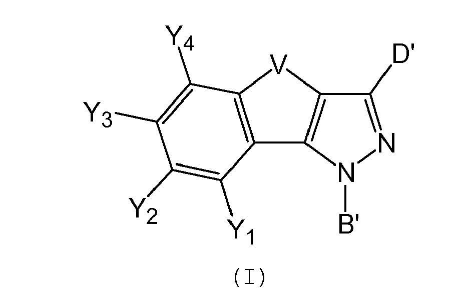 wherein:
B' is selected from phenyl, arylalkyl, arylalkenyl, heteroaryl, heteroarylalkyl, or a bivalent C₁-C₁₀ aliphatic chain, linear or branched when possible, wherein the end of the main chain not linked to the nitrogen atom is linked to W^{I}, W^{I} selected from hydrogen, halogen, isothiocyanate, CN, OH, OCH₃, NH₂, SO₂NH₂ or -CH=CH₂,
Y₁, Y₂, Y₃ and Y₄, equal to or different from each other, have the same meaning of X₁, X₂, X₃ and X₄ as defined in formula A',
V has the same meanings of A as defined in formula A', when B' is a bivalent C₁-C₁₀ aliphatic chain, linear or branched when possible, wherein the chain end not linked to the nitrogen atom is linked to W^{I} as defined above, D' has the same meanings of D as defined in formula A', when B' is selected from phenyl, arylalkyl, arylalkenyl, heteroaryl, heteroarylalkyl, D' is selected from the following groups:
D'2: -C(O)-Z'-R"
wherein Z' and R" are as defined in formula A',
D'3: -CH(OH)-Z'-R"
wherein Z' and R" are as defined in formula A',
D'4: -C(O)-NH-Z'-T'
Z' and T' being as defined in formula A', but excluding from the meanings of T' C₁-C₈ alkyl, C₁-C₇ haloalkyl and, when in D'4 Z'= -CH₂-, T' is not 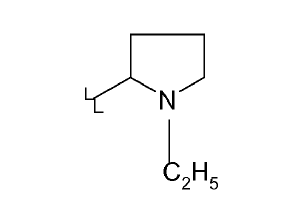 D"2: -C(O)-R", with the proviso that V=A2,
D"3: -CH(OH)-R", with the proviso that V=A2,
D"4: -C(O)-NH- T', with the proviso that V is selected from: -O-, -CH₂-O-.

19. Isomeric forms, both geometrical and stereoisomers, and mixtures thereof, of the compounds according to claim 18.

20. Compounds according to claims 18-19, wherein B' is selected from phenyl, arylalkyl, arylalkenyl, heteroaryl, heteroarylalkyl, and is optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

21. Compounds according to claims 18-20, wherein Y_{1,} Y₂, Y₃ or Y₄ are selected from phenyl, cycloalkyl, saturated or unsaturated heterocycle, heteroaryl, said phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

22. Compounds according to claims 18-21, wherein R" is substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio or C₁-C₇ alkoxy.

23. Compounds according to claims 18-22, wherein T' is optionally substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, said phenyl and benzyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

24. Compounds according to claims 18-23, wherein R₁ and R₂ of T' are aromatic rings selected from heteroaryl, heteroarylalkyl, aryl, arylalkyl or arylalkenyl, or R₁ and R₂ with the nitrogen atom form an heterocycle, the armatic rings or the heterocycle can have one or more substituents, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothocyanate, phenyl, benzyl, amino optionally mono- or bisubstituted with a C₁-C₇ alkyl chain, said phenyl and benzyl optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

25. Compounds according to claims 18-24, wherein:
B' is selected from phenyl, benzyl, monocyclic heteroaryl, monocyclic heteroarylalkyl or a bivalent C₁-C₁₀ aliphatic chain, linear or branched when possible, wherein the chain end not linked to the nitroogen atom is linked to W^{I}, W^{I} being selected from hydrogen, halogen, isothiocyanate, CN, OH, OCH₃, NH₂, SO₂NH₂ or -CH=CH₂, said phenyl, benzyl, monocyclic heteroaryl and monocyclic heteroarylalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
Y₁, Y₂, Y₃ and Y₄, equal to or different from each other, have the same meanings of X₁, X₂, X₃ and X₄ as defined above in formula A',
V represents a group selected from A1 or A2,
when B' has the meaning of bivalent C₁-C₁₀ aliphatic chain, linear or branched when possible, wherein the chain end not linked to the nitrogen atom is linked to W^{I} as defined above, D' has the same meanings of D as defined in formula A',
when B' is selected from phenyl, benzyl, monocyclic heteroaryl or monocyclic heteroarylalkyl, D' has the meanings of D'2, D'4, D"2 or D"4.

26. Compounds according to claims 18-25, wherein:
B' is selected from phenyl, benzyl, thiophene or a bivalent C₄-C₁₀ aliphatic chain, linear or branched when possible, wherein the chain end not linked to the nitrogen atom is linked to W^{I}, W^{I} selected from hydrogen, halogen, isothiocyanate, CN, OH, OCH₃, NH₂, SO₂NH₂ or -CH=CH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, have the same meanings of X₁, X₂, X₃ and X₄ as defined above in formula A',
V represents a group selected from A1 or A2,
when B' is a bivalent C₄-C₁₀ aliphatic chain, linear or branched when possible, wherein the chain end not linked to the nitrogen atom is linked to W^{I} as defined above,
D' has the same meanings of D as defined in formula A', when B' is selected from phenyl, benzyl or thiophene, D' is selected from D'2, D'4, D"2 or D"4.

27. Compounds according to claims 18-26, wherein:
B' is selected from phenyl, benzyl, thiophene, a bivalent C₄-C₁₀ aliphatic chain, linear or branched when possible, wherein the chain end not linked to the nitrogen atom is linked to W^{I}, W^{I} selected from hydrogen, halogen, OH, OCH₃, NH₂, SO₂NH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ aliphatic chain,
V is selected between A1 or A2 of compound A',
when B' has the meaning of bivalent C₄-C₁₀ aliphatic chain, linear or branched when possible, wherein the chain end not linked to the nitrogen atom is linked to W^{I}, D' has the same meanings of D as defined in formula A',
when B' is selected from phenyl, benzyl or thiophene, D' is selected from D'2, D'4, D"2 or D"4, with the proviso that:
Z' is -CH₂- or -CH(CH₃)-,
R" is selected from C₃-C₁₅ cycloalkyl, saturated or unsaturated heterocycle, aryl, or heteroaryl, said C₃-C₁₅ cycloalkyl, saturated or unsaturated heterocycle, aryl and heteroaryl being optionally substituted with one or more groups, equal to or different from each other, selected from SO₂NH₂, halogen, C₁-C₃ alkyl, C₁-C₃ haloalkyl, C₁-C₃ haloalkoxy, C₁-C₃ alkylthio, C₁-C₃ alkoxy,
T' is selected from:
- C₃-C₁₅ cycloalkyl,
- monocyclic aryl when one of the following alternative conditions is met:
V is different from A1, or
independently from V, B' is different from phenyl,
benzyl or thiophene,
- NR₁R₂, wherein R₁ and R₂, equal to or different from each other, form with the nitrogen atom a saturated or unsaturated heterocycle from 5 to 10 atoms,
- C₃-C₁₅ heterocycloalkyl, wherein Z' is linked to one carbon atom of the heterocycloalkyl.

28. Compounds according to claims 18-27, wherein T' is substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, said phenyl and benzyl optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain.

29. Compounds according to claims 18-28, selected from the following: 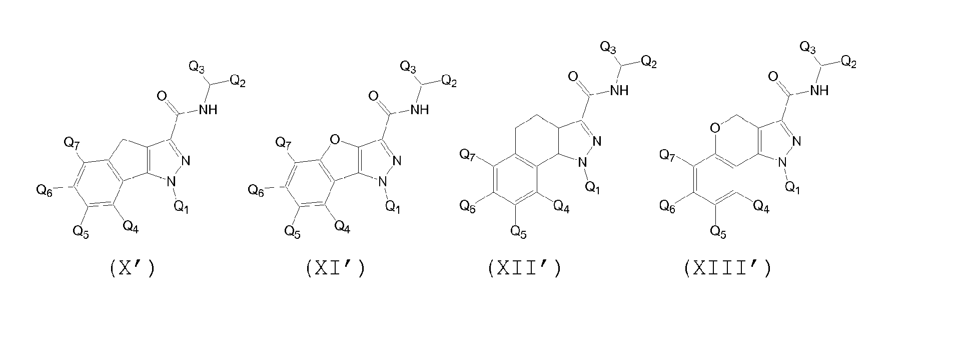 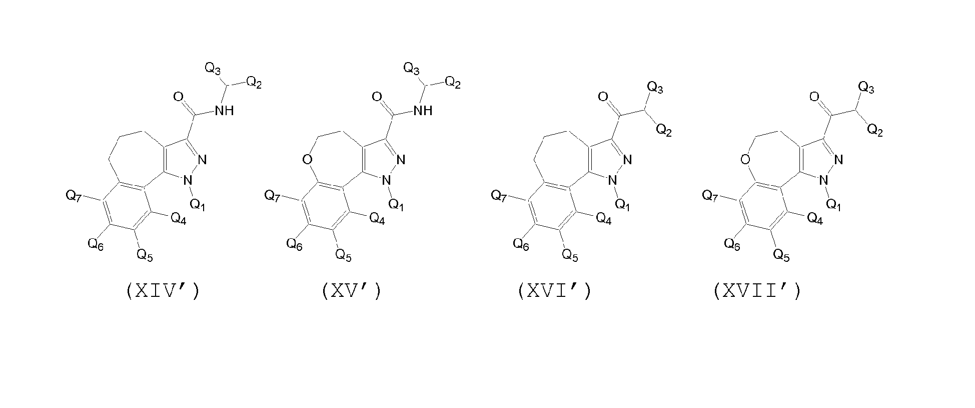 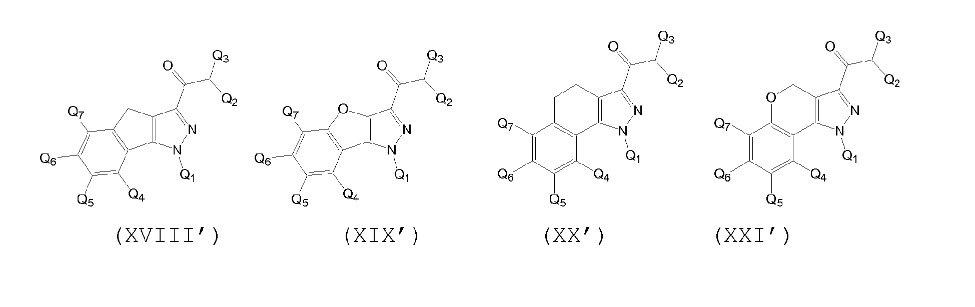 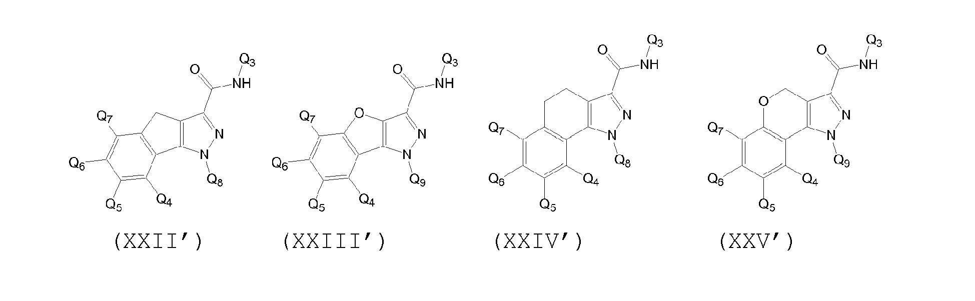 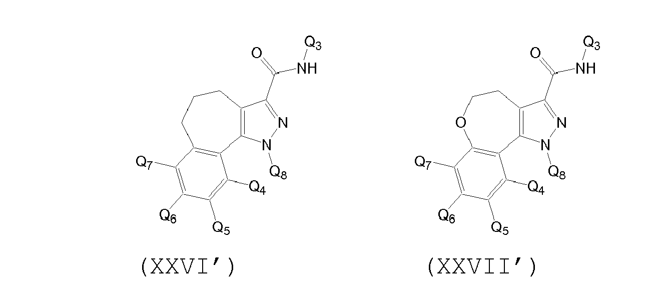 wherein:
Q, has the following meanings:
Q₁A: bivalent C₄-C₁₀ aliphatic chain, linear or branched when possible, wherein the end of the chain not linked to the nitrogen atom is linked to W^{IV}, W^{IV} selected from hydrogen, halogen, OH, OCH₃, NH₂ or SO₂NH₂,
Q₁B, selected from phenyl and benzyl, optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, or amino optionally mono- or bisubstituted with a C₁-C₃ alkyl chain,
Q₈ is as Q₁A as defined above,
Q₉ is as Q₁ as defined above,
Q₂ is selected from hydrogen, methyl,
Q₄, Q₅, Q₆, Q₇, equal to or different from each other, are selected from hydrogen, halogen, C₁-C₃ alkyl, C₁-C₃ alkoxy, C₁-C₃ haloalkyl, cyano, SO₂NH₂, isothiocyanate, phenyl, cycloalkyl, saturated or unsaturated heterocycle, thiophene, amino optionally mono- or bisubstituted with a C₁-C₃ alkyl chain, said phenyl, cycloalkyl, saturated or unsaturated heterocycle and thiophene optionally substituted with one or more groups, equal to or different from each other, selected from halogen, C₁-C₃ alkyl, C₁-C₃ alkoxy, C₁-C₃ haloalkyl, cyano, SO₂NH₂, isothiocyanate, amino optionally mono- or bisubstituted with a C₁-C₃ alkyl chain,
Q₃ is selected from the following structures:

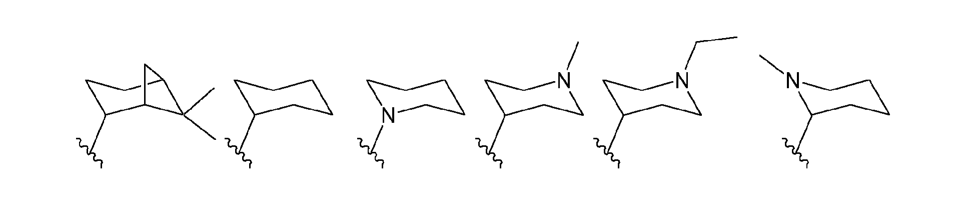

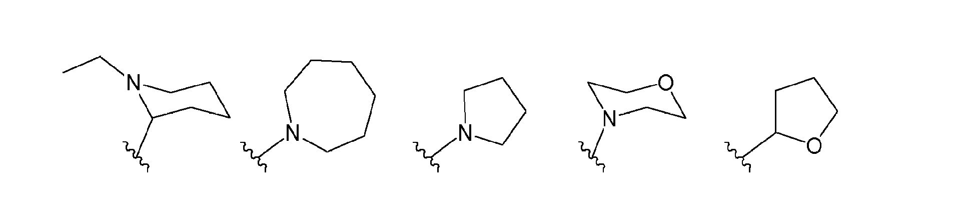

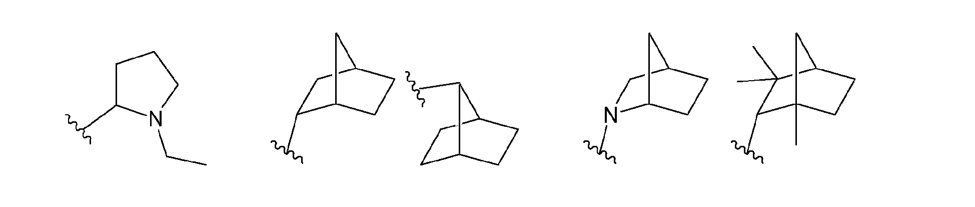

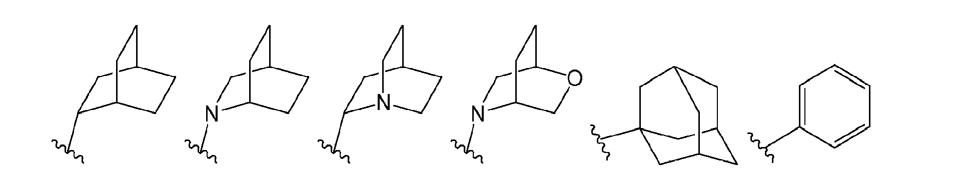

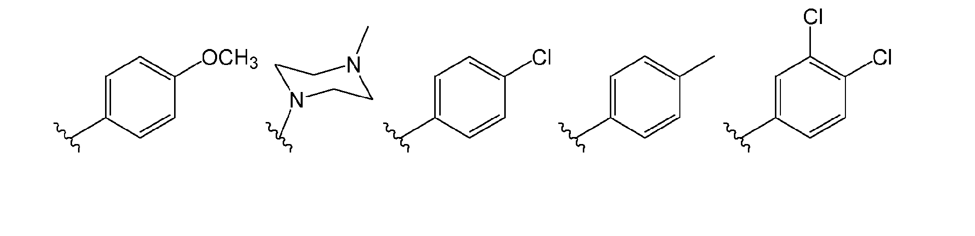

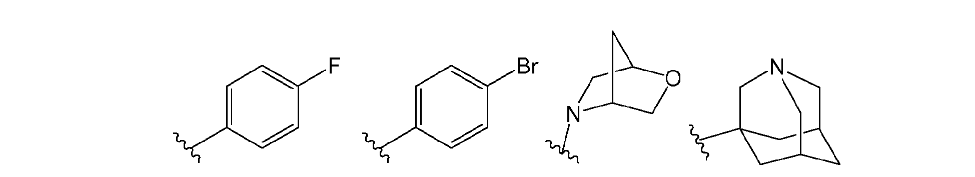

30. Compounds according to claims 18-29, selected from the following:

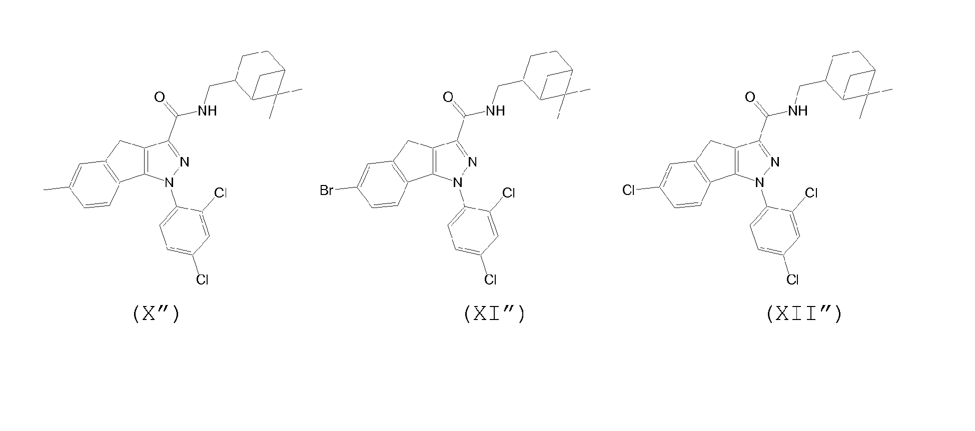

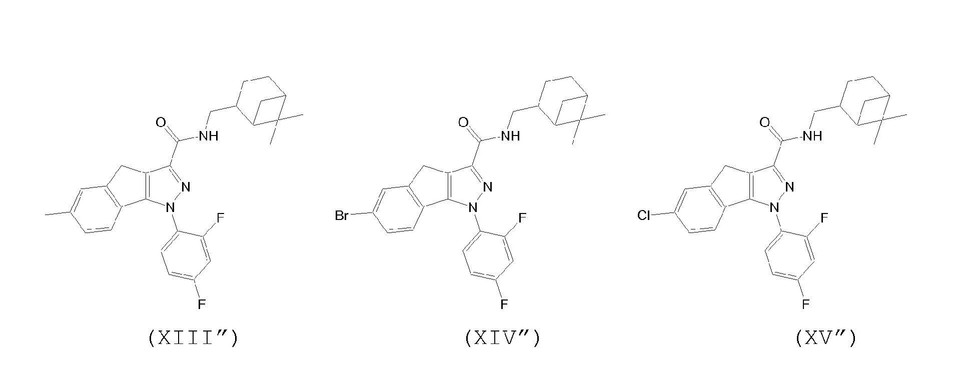

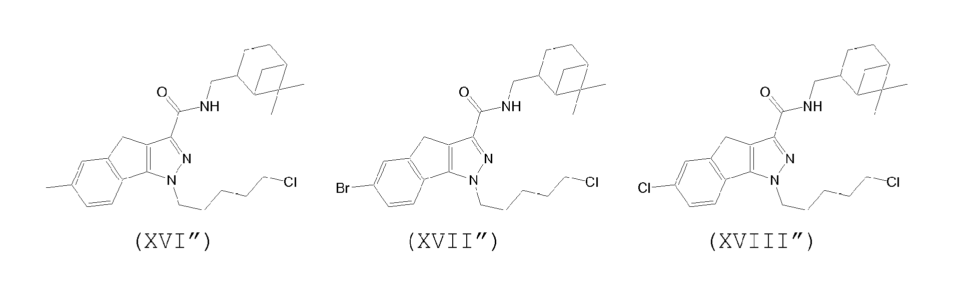

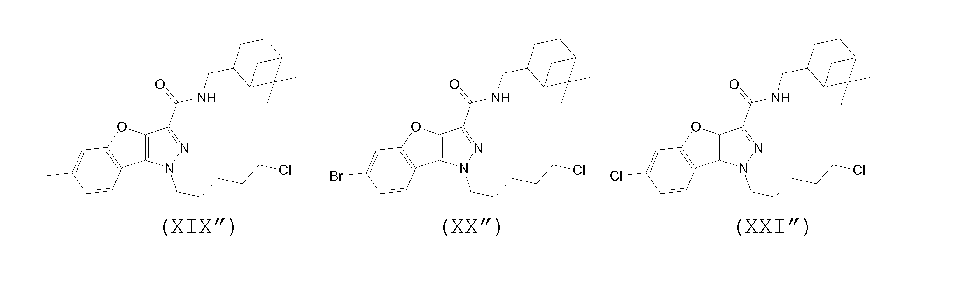

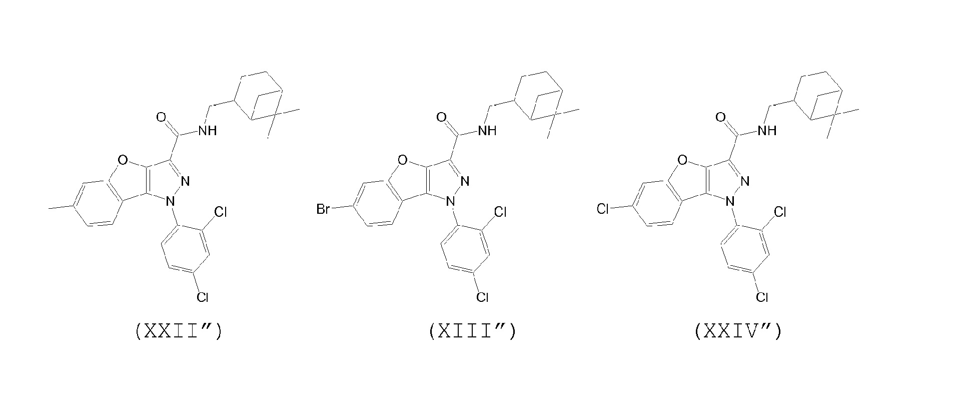

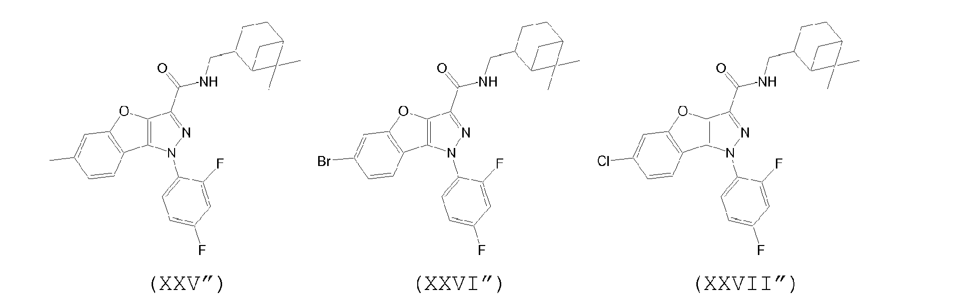

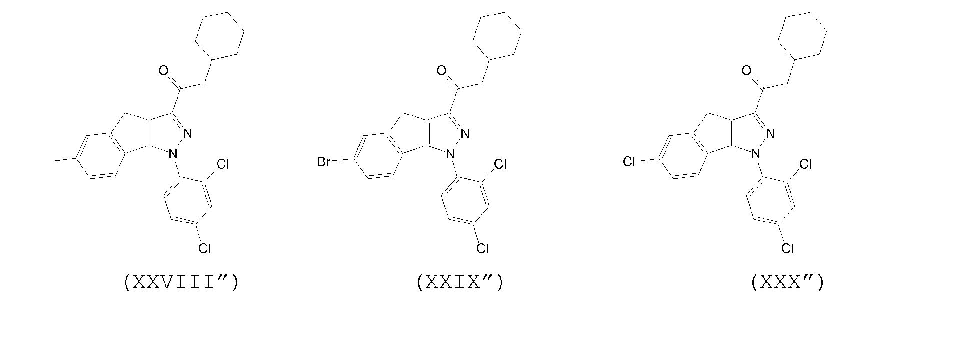

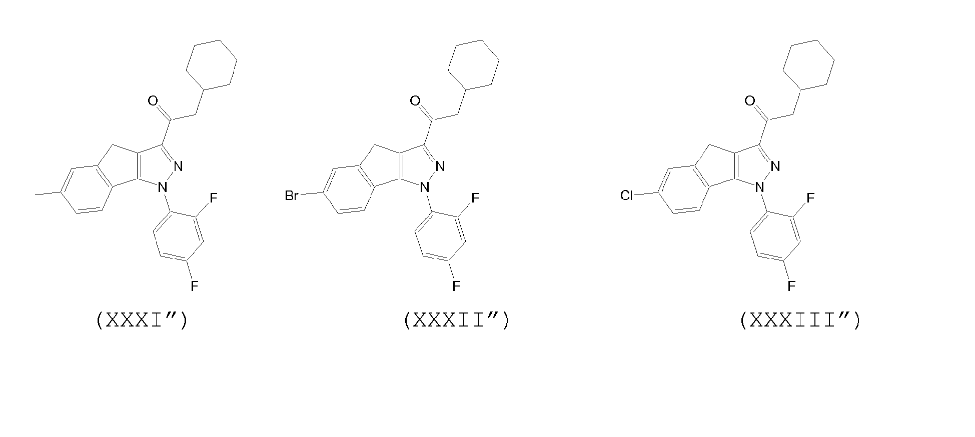

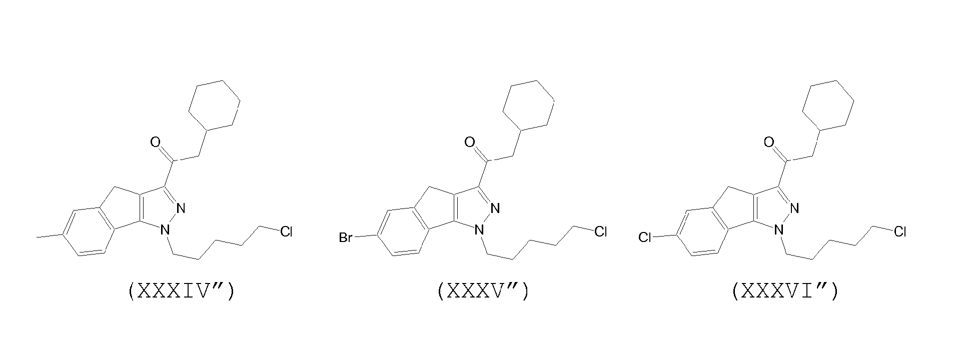

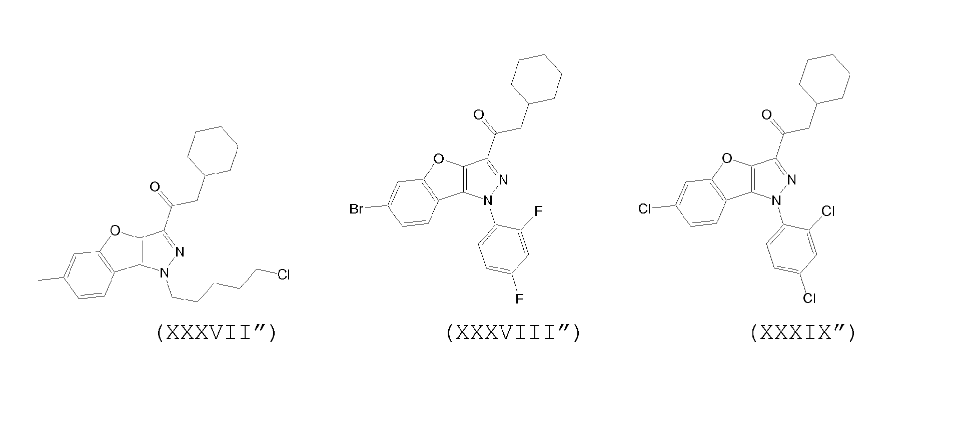

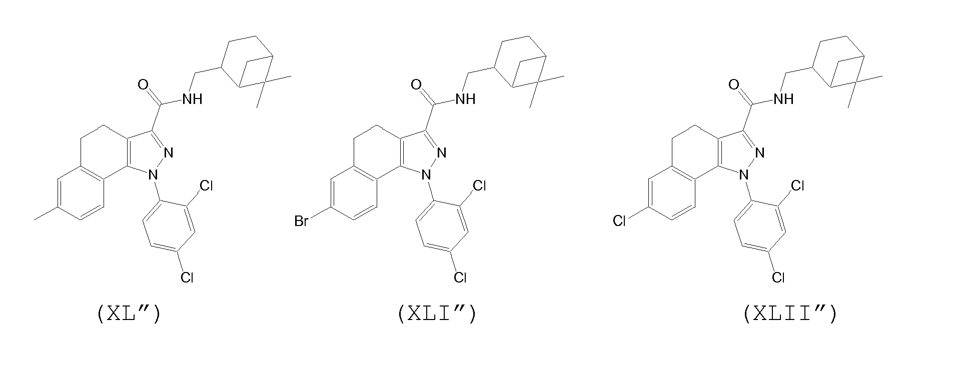

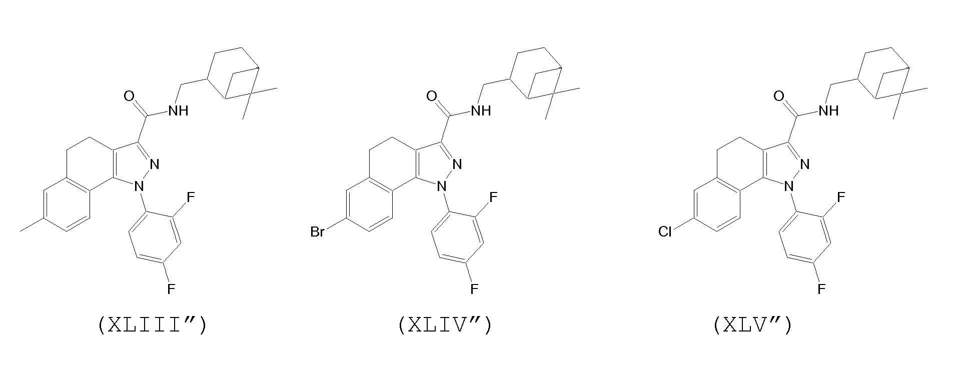

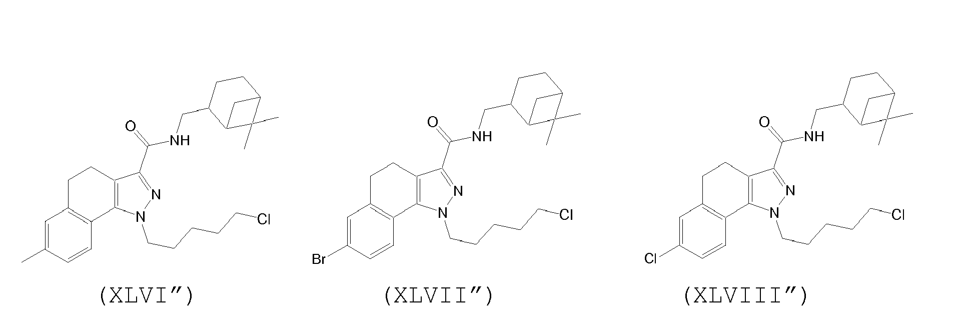

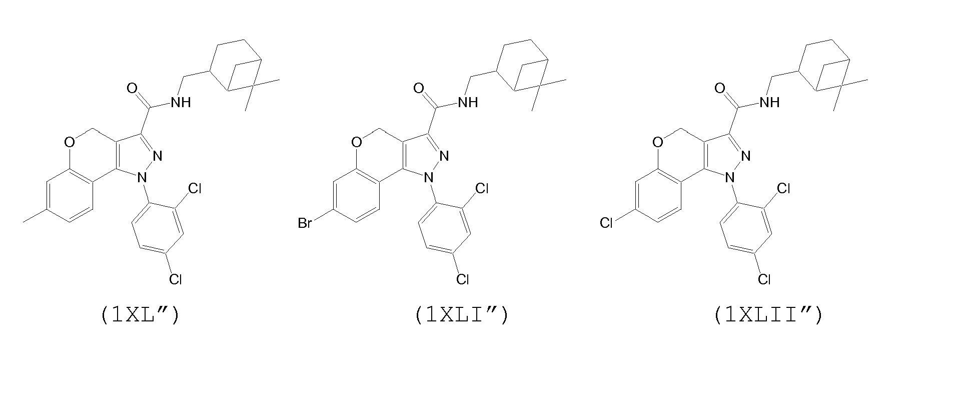

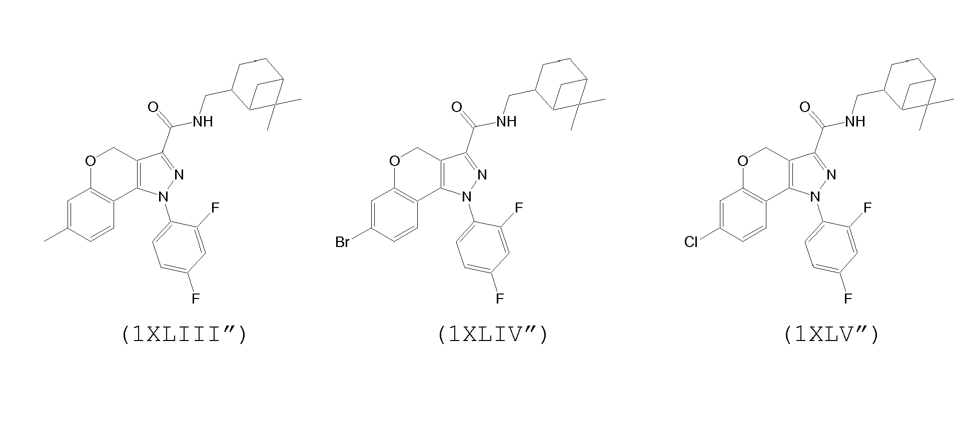

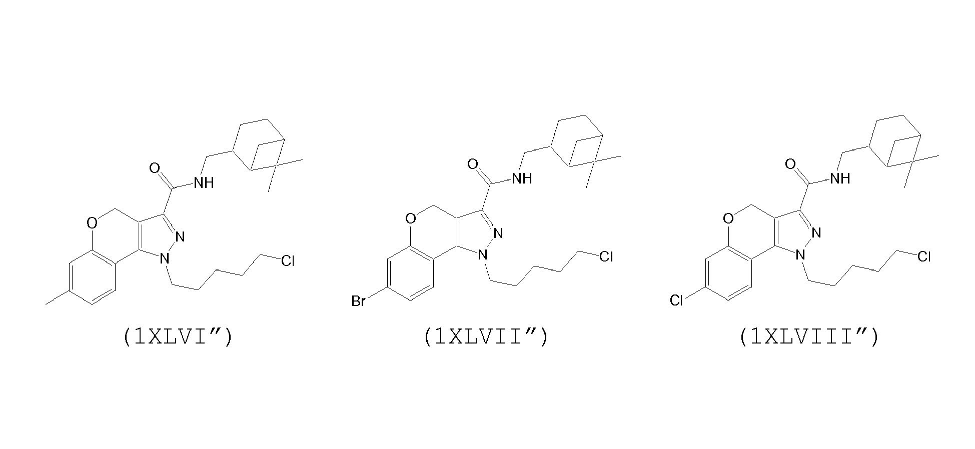

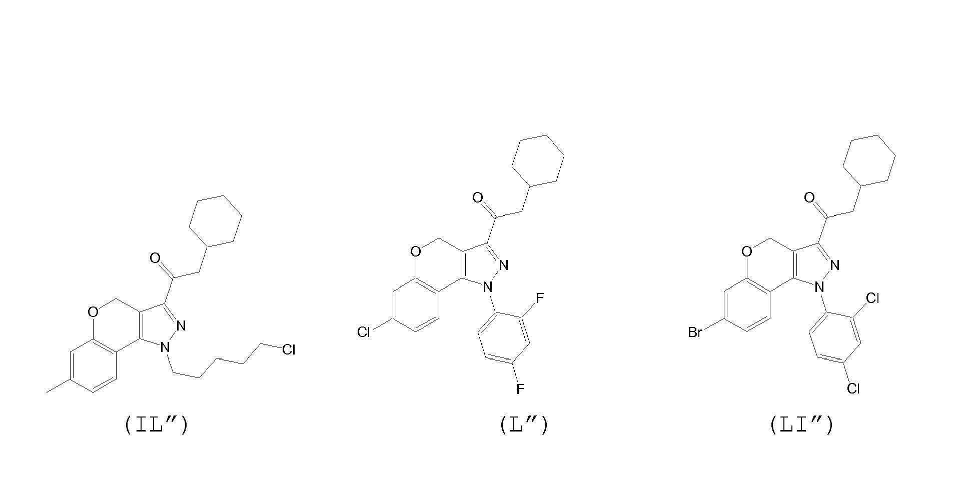

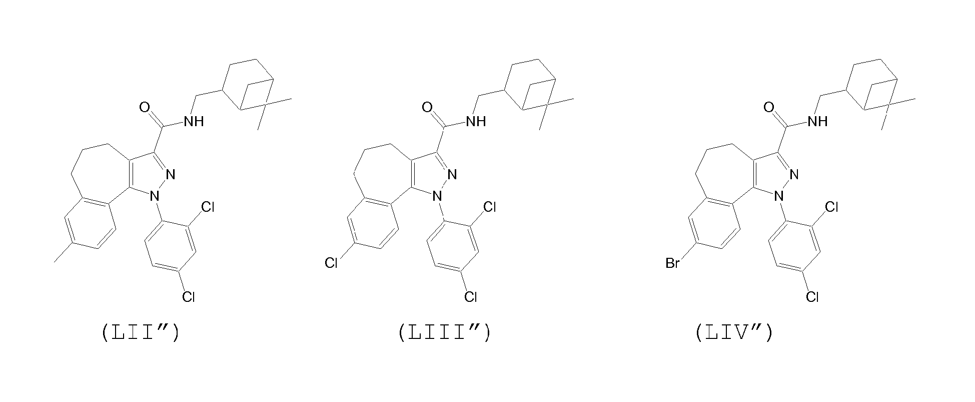

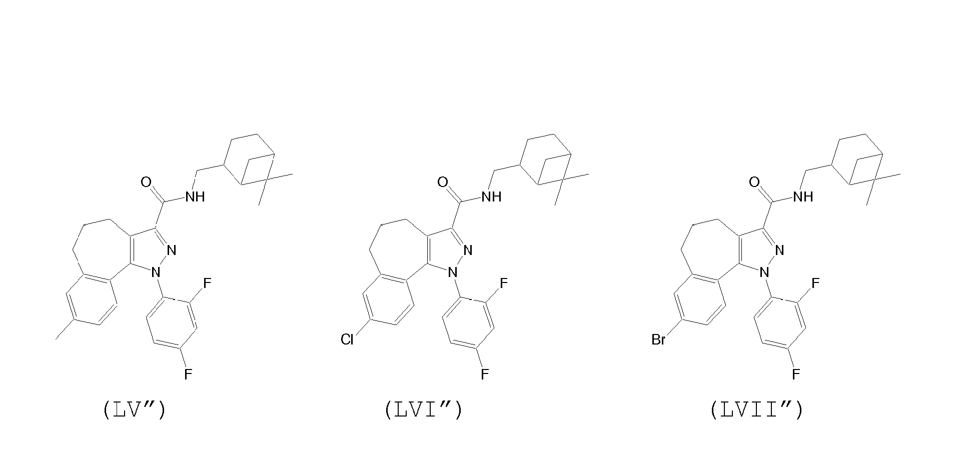

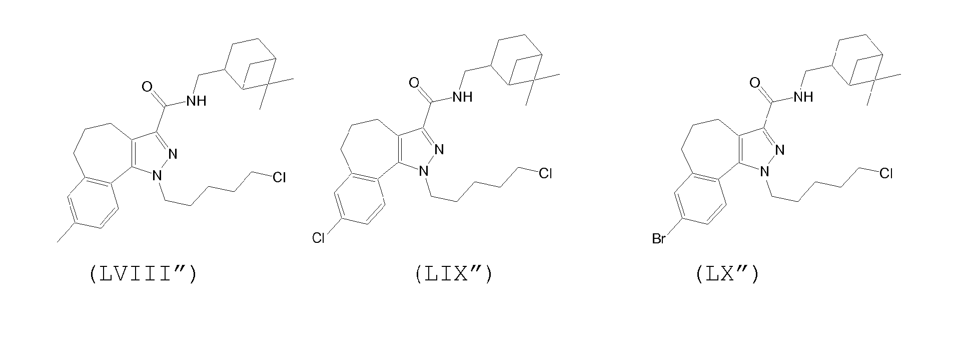

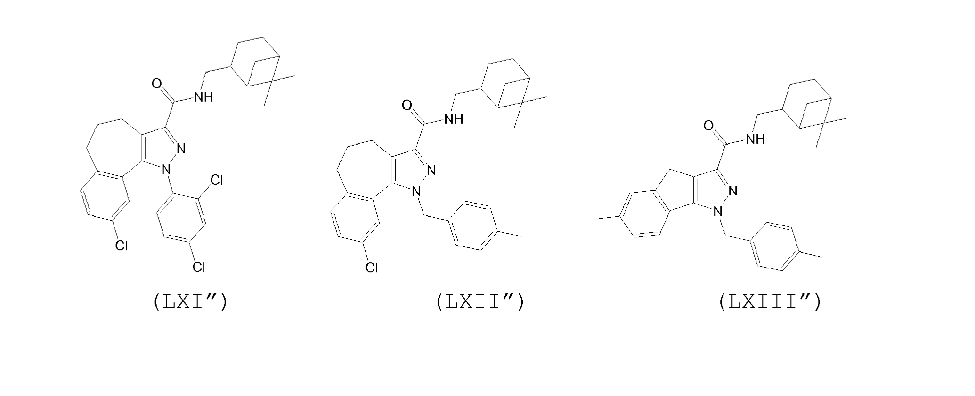

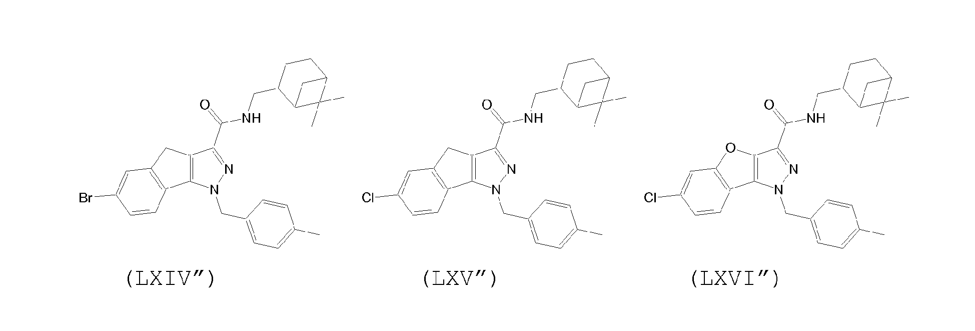

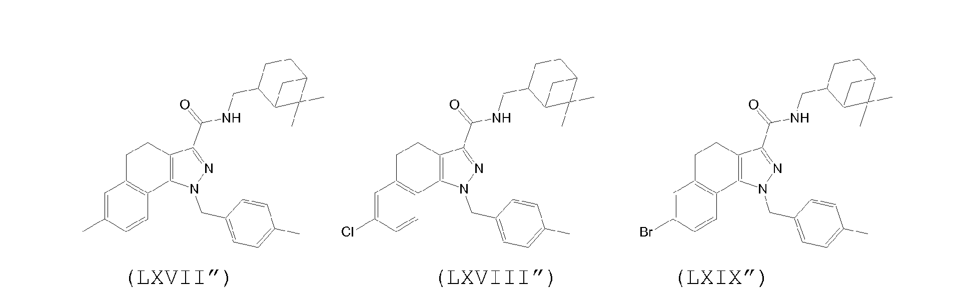

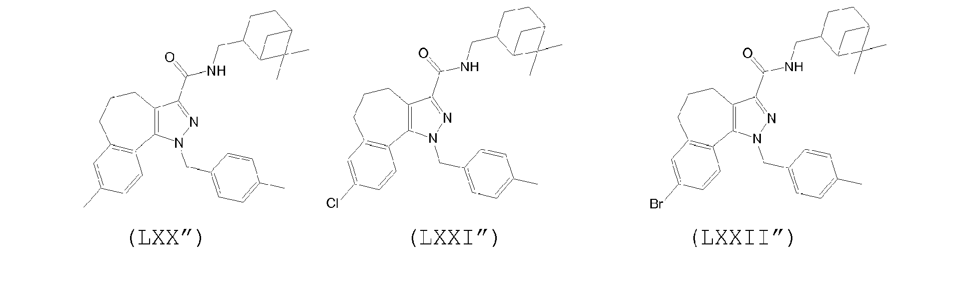

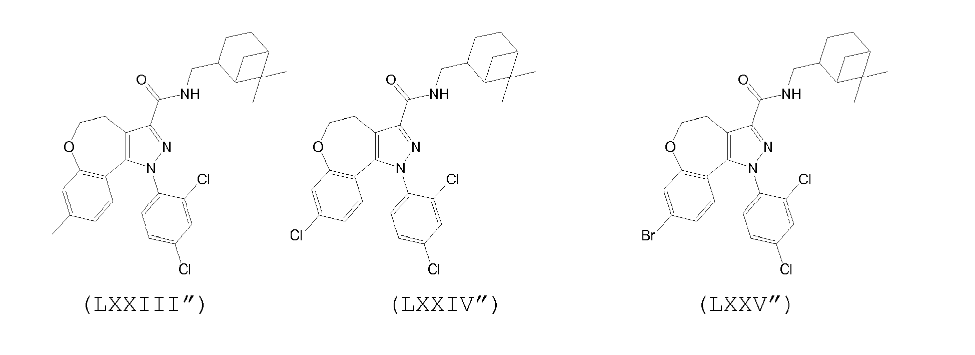

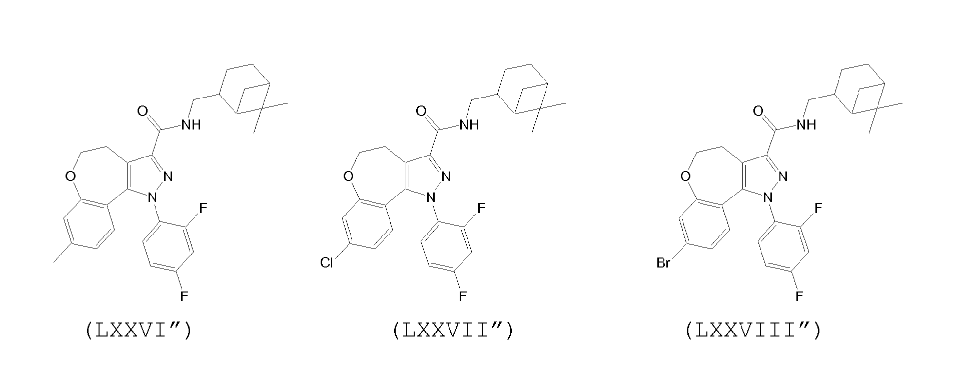

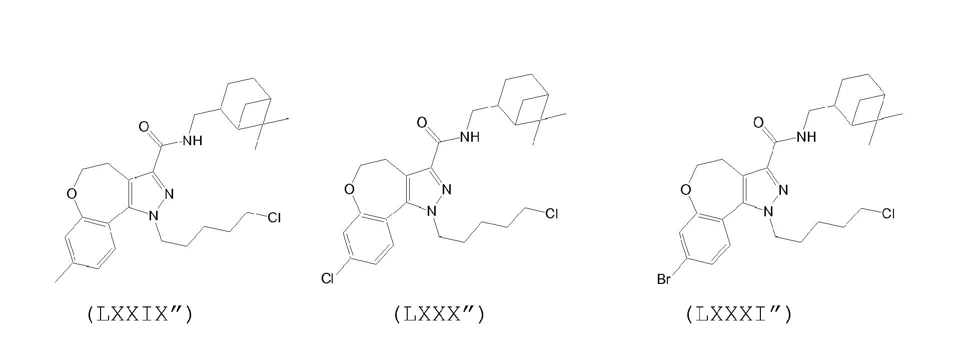

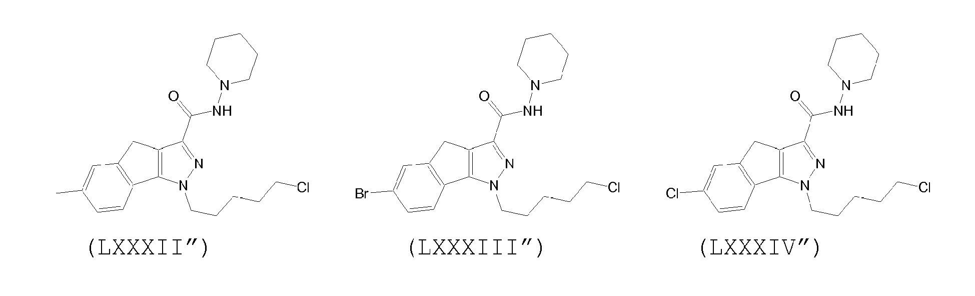

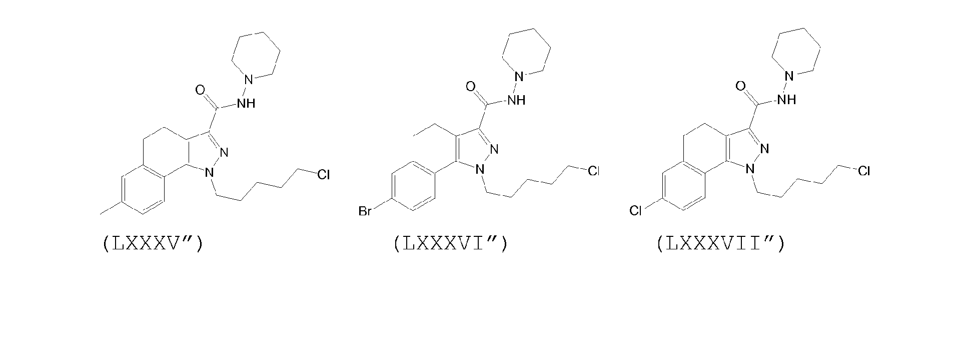

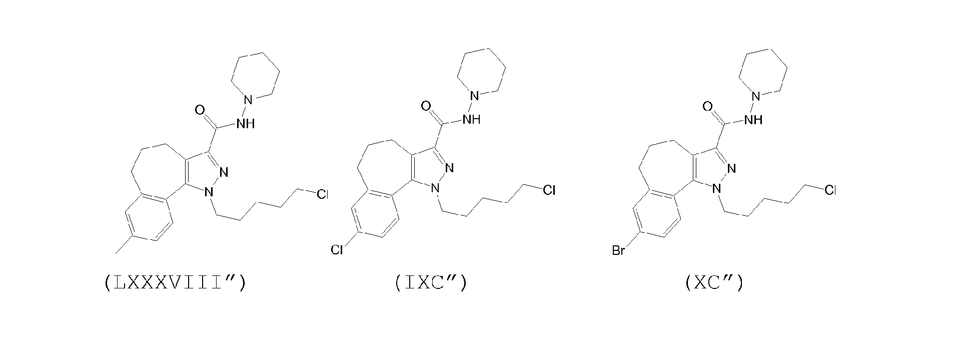

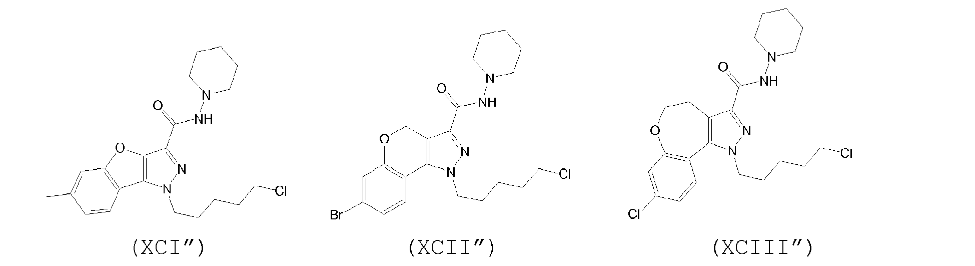

31. Compounds according to claim 30, selected from the following:
(XVI"), (XVII"), (XVIII"), (XIX"), (XX"), (XXI"), (XXXIV"), (XXXV"), (XXXVI"), (XXXVII"), (XLVI"), (XLVII"), (XLVI"), (XLVII"), (XLVIII"), (1XLVI"), (1XLVII"), (1XLVIII"), (IL"), (LVIII"), (LVIX"), (LX"), (LXXIX"), (LXXX"), (LXXXI"), (LXXXII"), (LXXXIII"), (LXXXIV"), (LXXXV"), (LXXXVI"), (LXXXVII"), (LXXXVIII"), (IXC"), (XC"), (XCI"), (XCII"), (XCII").

32. Hydrates, solvates and pharmaceutically acceptable salts of the compounds according to claims 18-31.

33. Metabolites of the compounds of claims 18-32 administered to an individual or to an animal.

34. A process for preparing compounds according to claims 18-32 comprising:
i) synthesis of the acid of formula (II), or optionally of a reactive derivative thereof, said reactive derivative selected from acyl halides, anhydrides, mixed anhydrides, imidazolides, ester-amide adducts, linear or branched C₁-C₄ alkyl esters, 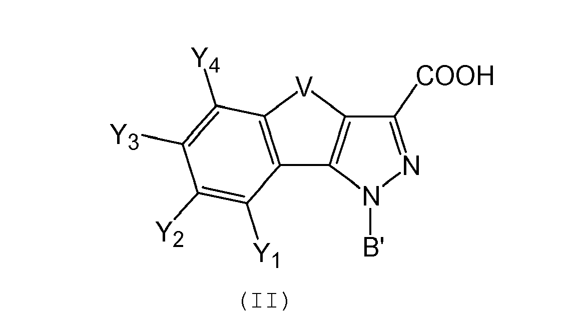 comprising the following steps:
- preparation α-hydroxy-γ-ketoesters of formula (IV), wherein V, Y₁, Y₂, Y₃ and Y₄ are as above defined, by reacting a compound of formula (III) with sodium alkoxide (RONa) and diethyloxalate in a C₁-C₃ alcoholic solvent, at reflux: 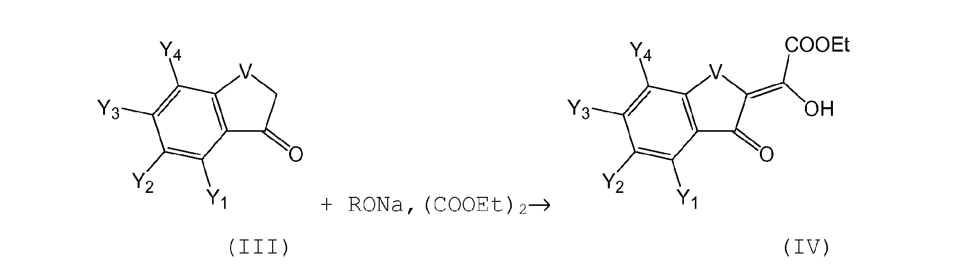
- reaction of the compounds of formula (IV) with an hydrazine of formula (VI) wherein B' is as above defined, said compound (VI) being optionally in the form of an hydrochloride salt thereof in an alcoholic solvent or in acetic acid, at reflux, to yield the tricyclic compound of formula (VII): 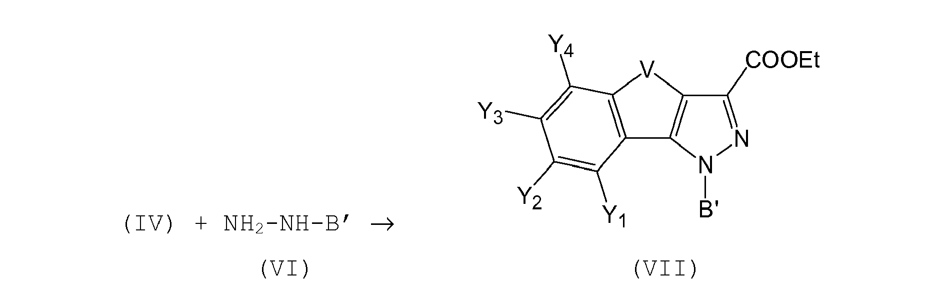
- alkaline hydrolysis with alkaline hydroxides in hydroalcoholic solution of the compound of formula (VII) at reflux, to obtain the acid of formula (II);
- optionally, formation of a reactive derivative of the acid of formula (II),
ii) if D' is an ethereal group D1 in formula (I), the acid of formula (II) or an ester thereof, is reduced in a first step to primary alcohol with an organic metal hydride, then in a second step the primary alcohol is reacted at room temperature with an alkyl halide of formula R"-(Z')ᵥ -(CH₂)_{z}-Hal, wherein Hal is halogen and z', v and z are as above defined, in the presence of an alkali hydride, obtaining the compounds of formula (I) wherein D'=D1,
iii) if D'=D2, the compounds are prepared according to one of the following processes:
first process, comprising:
- reaction of an ester of the acid of formula (II), with trialkylaluminum and the hydrochloride salt of an amine and subsequent addition to the reaction mixture of R"-(Z')ᵥ-MgBr, wherein Z', v and R" are as defined above, to obtain the compound of formula (I),
second process, comprising:
- reaction of the acid of formula (II), or a reactive derivative thereof, with a metallorganic salt of formula (R"-(Z')ᵥ)⁻ Me⁺, wherein Me⁺ is an alkaline metal cation, obtaining the compound of formula (I),
iiii) if D'=D3 the synthesis comprises the following two steps:
- formation of the compound of formula (I) wherein D'=D2, by using one of the two processes described in iii),
- reduction of the compound obtained in the preceding step and isolation of the final product,
iiiii) if D'=D4, the compounds are prepared by reaction of the acid of formula (II), in the form of a suitable reactive derivative thereof, with a compound of formula:
H₂N-(Z')_{V}-T' (VIIA)
wherein Z', v and T' have the previously defined meanings.

35. Compounds of formula (II'): 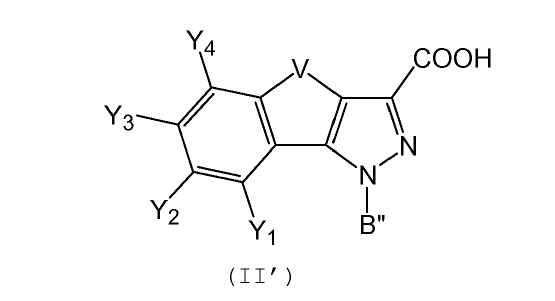 wherein:
V, Y₁, Y₂, Y₃ and Y₄ are as defined above,
B" is hydrogen or a bivalent C₁-C₁₀ aliphatic chain, linear or branched when possible, wherein the chain end not linked to the nitrogen atom is linked to W^{II}, W^{II} selected from hydrogen, halogen, isothiocyanate, CN, OH, OCH₃, NH₂, SO₂NH₂ or -CH=CH₂.

36. Pharmaceutical compositions obtainable from the microemulsions of claims 1-16.

37. Use of the microemulsions of claims 1-16 to prepare pharmaceutical compositions.

38. Use of the pharmaceutical compositions according to claim 36 for the prophylaxis and therapy in mammals and in human beings of the diseases and disorders wherein the receptors of the CB1 and/or CB2 cannabinoids are involved.

39. Use according to claim 38, wherein the diseases and the disorders are the following: diseases involving immune system cells, immune disorders, osteoporosis, renal ischaemia, inflammatory conditions, pain, post-surgery pain, neuropathic pain, eye diseases, pulmonary diseases, asthma, chronic bronchitis, inflammations, arthritis, allergies and allergic reactions, allergic rhinitis, contact dermatitis, allergic conjunctivitis, anxiety, behavioural disorders, delirium conditions, psychotic disorders, schizophrenia, depression, treatment of drug and/or alcohol abuse, alcoholism, tabagism, vomit, nausea, vertigoes, in particular in patients submitted to chemotherapy, neuropathies, hemicrania, stress, psychosomatic origin diseases, epilepsy, Tourette syndrome, Parkinson disease, Huntington disease, Alzheimer disease, senile dementia, cognition disorders and memory loss, pathologies associated with appetite, obesity, bulimia, pathologies of the gastrointestinal tract and of the bladder, cardiovascular diseases, urinary, erectile and fertility disorders, neuroinflammatory pathologies, multiple sclerosis, Guillain-Barré syndrome, viral encephalitis, amyotrophic lateral sclerosis, syndrome associated to demineralization, osteoporosis, in reducing metabolic and/or cardiovascular risk factors, also in patients with metabolic syndrome and/or dyslipidemia and in patients with type 2 diabetes, eye inflammatory conditions, eye autoimmune diseases, uveitis, uveoretinitis and retina neurodegeneration.

40. Compounds according to claims 18-32, or isomers or hydrates or solvates or pharmaceutically acceptable salts thereof, for the manufacture of drugs for the treatment in mammals and in human beings of diseases and disorders in which the receptors of the CB1 and/or CB2 cannabinoids are involved.

41. Use of the compounds according to claims 18-32 as a medicament.

42. Use of the compounds according to claims 18-32 containing radioactive isotopes, or the pharmaceutical formulations thereof, for identifying and labelling the receptors of the CB1 and/or CB2 cannabinoids in mammals or in human beings.

43. Use of the compounds according to claims 18-32 comprising in the molecule an hydroxylic group, to obtain ligands of the cannabinoidergic receptors.

44. Use according to claim 40, wherein the disease and disorders are those of claim 39.
